(19) Europäisches Patentamt
European Patent Office
Office européen des brevets

(11) **EP 4 028 054 B1**

(12) **EUROPEAN PATENT SPECIFICATION**

(45) Date of publication and mention
of the grant of the patent:
**31.12.2025 Bulletin 2026/01**

(21) Application number: **20780466.7**

(22) Date of filing: **10.09.2020**

(51) International Patent Classification (IPC):
***A61K 39/395*** (2006.01)      ***A61P 37/06*** (2006.01)
***C07K 16/28*** (2006.01)      ***A61K 39/00*** (2006.01)

(52) Cooperative Patent Classification (CPC):
**C07K 16/2887; A61K 39/39541; A61P 13/12;**
**A61P 37/06;** A61K 2039/505; A61K 2039/545;
C07K 2317/565; C07K 2317/73

(86) International application number:
**PCT/US2020/050072**

(87) International publication number:
**WO 2021/050645 (18.03.2021 Gazette 2021/11)**

(54) **COMPOSITIONS AND METHODS OF TREATING LUPUS NEPHRITIS**

ZUSAMMENSETZUNGEN UND VERVAHREN ZUR BEHANDLUNG VON LUPUS NEPHRITIS

COMPOSES ET PROCEDES POUR LE TRAITEMENT DE LA NEPHRITE DE LUPUS

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **12.09.2019   US 201962899706 P**
**04.11.2019   US 201962930527 P**
**05.11.2019   US 201962931032 P**
**03.04.2020   US 202063005071 P**

(43) Date of publication of application:
**20.07.2022   Bulletin 2022/29**

(60) Divisional application:
**24155884.0 / 4 438 056**
**24156175.2 / 4 438 057**

(73) Proprietor: **F. Hoffmann-La Roche AG**
**4070 Basel (CH)**

(72) Inventor: **CASCINO, Matthew Dominic**
**San Francisco, California 94080 (US)**

(74) Representative: **Klein, Thomas**
**F. Hoffmann-La Roche AG**
**Patent Department**
**Grenzacherstrasse 124**
**4070 Basel (CH)**

(56) References cited:
**WO-A1-2016/183104      WO-A2-2005/120437**

- **GENENTECH: "Genentech's Gazyva**
**(Obinutuzumab) Delivers Positive Topline**
**Results for Phase II Lupus Nephritis Study",**
**PRESS RELEASES, MONDAY JUN 10, 2019, 10**
**June 2019 (2019-06-10), pages 1 - 7,**
**XP093224986, Retrieved from the Internet**
**<URL:https://www.gene.com/media/**
**press-releases/14796/2019-06-10/**
**genentechs-gazyva-obinutuzumab-delivers->**
- **FURIE RICHARD ET AL: "A Phase II Randomized,**
**Double-Blind, Placebo-Controlled Study to**
**Evaluate the Efficacy and Safety of**
**Obinutuzumab or Placebo in Combination with**
**Mycophenolate Mofetil in Patients with Active**
**Class III or IV Lupus Nephritis", ACR**
**ABSTRACTS: 2019 ACR/ARP ANNUAL**
**MEETING, 10 November 2019 (2019-11-10), pages**
**1 - 4, XP093319985, Retrieved from the Internet**
**<URL:https://acrabstracts.org/abstract/**
**a-phase-ii-randomized-double-blind-placebo-**
**controlled-study-to-evaluate-the-efficacy-and-sa**
**fety-of-obinutuzumab-or-placebo-in-combina**
**tion-with-mycophenolate-mofetil-in-patients-wi**
**th-active-class-iii/>**

EP 4 028 054 B1

**(Cont. next page)**

- PAPPAS A.L. ET AL: "Obinutuzumab (Gazyva)", PHARMACY TIMES, vol. 3, no. 3, 13 May 2014 (2014-05-13), pages 1 - 9, XP093319892
- ROCHE: "Roche's Gazyva (obinutuzumab) delivers positive topline results for phase II lupusnephritis study", 11 June 2019 (2019-06-11), XP002801499, Retrieved from the Internet <URL:https://www.roche.com/investors/updates/inv-update-2019-06-11c.htm> [retrieved on 20201216]
- SCHINDLER T. ET AL.: "NOBILITY, a phase 2 trial to assess the safety and efficacy of obinutuzumab, a novel type 2 anti-CD20 monoclonal antibody (mAB) in patients (Pts) with ISN/RPS class III or IV lupus nephritis (LN)", ANNALS OF THE RHEUMATIC DISEASES, vol. 75, no. S2, AB0423, 9 June 2016 (2016-06-09), pages 1051, XP002801500
- FDA: "Obinutuzumab (Gazyva)", December 2015 (2015-12-01), XP002801501, Retrieved from the Internet <URL:https://www.pbm.va.gov/PBM/clinicalguidance/drugmonographs/Obinutuzumab_GAZYVA_Drug_Monograph.pdf> [retrieved on 20201216]
- GOMEZ MENDEZ L.M. ET AL.: "Peripheral Blood B Cell Depletion after Rituximab and Complete Response in Lupus Nephritis", CLIN. J. AM. SOC. NEPHROL., vol. 13, 13 October 2018 (2018-10-13), pages 1502 - 1509, XP002801502
- CRAVEDI P. ET AL.: "Efficacy and Safety of Rituximab Second-LineTherapy for Membranous Nephropathy:A Prospective, Matched-Cohort Study", AM. J. NEPHROL., vol. 33, 2011, pages 461 - 468, XP002801503
- REDDY VENKAT ET AL: "Optimising B-cell depletion in autoimmune disease: is obinutuzumab the answer?", DRUG DISCOVERY TODAY, ELSEVIER, AMSTERDAM, NL, vol. 21, no. 8, 22 June 2016 (2016-06-22), pages 1330 - 1338, XP029680975, ISSN: 1359-6446, DOI: 10.1016/J.DRUDIS.2016.06.009
- REDDY V. ET AL.: "Obinutuzumab induces superior B-cell cytotoxicity to rituximab in rheumatoid arthritis and systemic lupus erythematosus patient samples", RHEUMATOLOGY, vol. 56, 11 April 2017 (2017-04-11), pages 1227 - 1237, XP002801504
- REDDY V. ET AL.: "Improving B-cell depletion in rheumatoid arthritis and systemic lupus erythematosus: Resistance to rituximab and the potential of obinutuzumab", ANNALS OF THE RHEUMATIC DISEASES, vol. 75, no. S2, OP0159, 9 June 2016 (2016-06-09), pages 116, XP002801505
- FURIE R. ET AL.: "A Phase II Randomized, Double-Blind, Placebo-Controlled Study to Evaluate the Efficacy and Safety of Obinutuzumab or Placebo in Combination with Mycophenolate Mofetil in Patients with Active Class III or IV Lupus Nephritis", 10 November 2019 (2019-11-10), XP002801506, Retrieved from the Internet <URL:https://acrabstracts.org/abstract/a-phase-ii-randomized-double-blind-placebo-controlled-study-to-evaluate-the-efficacy-and-safety-of-obinutuzumab-or-placebo-in-combination-with-mycophenolate-mofetil-in-patients-with-active-class-iii/> [retrieved on 20201216]

Remarks:
The complete document including Reference Table(s) and the Sequence Listing(s) can be downloaded from the EPO website

**Description**

FIELD OF THE INVENTION

Provided herein are type II anti-CD20 antibodies for use in a method for treating lupus nephritis (LN) in an individual that has lupus by administering a type II anti-CD20 antibody.

BACKGROUND

Proliferative lupus nephritis is the most common organ-threatening manifestation of systemic lupus erythematosus. Glomerular injury and tubulointerstitial inflammation result in proteinuria, hematuria, and progressive renal impairment. Goals of treatment include reduction in proteinuria, prevention of renal damage, and minimization of toxicities of immunosuppressive therapies. Hahn et al., Arthritis Care and Research 64:797-808, 2012; Fanouriakis et al., Ann. Rheum. Dis. 78:736-45, 2019. Even with treatment, many patients have a poor outcome such as the development of end-stage renal disease (ESRD), need for hemodialysis or renal transplantation, or death, and the risk of ESRD has not substantially improved during the last twenty years. Hanly, et al., Rheumatology 55(2):252-62, 2016; Tektonidou et al., Arthritis Rheumatol 68(6):1432-1441, 2016). There are currently no therapies approved for the treatment of lupus nephritis in the United States. Current non-approved, standard of care treatments are associated with toxicities and low rates of complete response.

Two anti-CD20 antibodies have been tested in clinical studies for efficacy in treating lupus nephritis. Rituximab, a type I anti-CD20 antibody, depleted peripheral CD19+ B cells in 71 of 72 patients and led to more responders and greater reductions in anti-dsDNA and C3/C4 levels in a clinical study (LUNAR). Dose regimen for the LUNAR study consisted of administration to patients with class III or class IV lupus nephritis (LN), rituximab (1,000 mg) or placebo on days 1, 15, 168, and 182 (week 0, 2, 24, 26). However, rituximab therapy did not improve clinical outcomes after 1 year of treatment.

Ocrelizumab, another type I anti-CD20 antibody, was tested in a clinical study (BELONG). Patients were randomized 1:1:1 to receive placebo, 400 mg ocrelizumab, or 1,000 mg ocrelizumab given as an intravenous infusion on days 1 and 15, followed by a single infusion at week 16 and every 16 weeks thereafter, accompanied by background glucocorticoids plus either mycophenolate mofetil (MMF) or the Euro-Lupus Nephritis Trial (ELNT) regimen (cyclophosphamide followed by azathioprine). The study was terminated, in part, because of an imbalance of serious infectious events (Mysler, E.F. et al. (2013) Arthritis Rheum. 65:2368-2379).

Therefore, there remains a need for testing the efficacy of other options in treating or preventing LN in patients with lupus. Preliminary data concerning the use of obinutuzumab, a type II anti-CD20 antibody, were obtained in the Phase II NOBILITY trial, but these concerned only primary endpoints and did not reflect long-term therapy and outcomes.

SUMMARY

The invention relates to obinutuzumab for use in a method of treating lupus nephritis in a human patient, as per the appended claims. Reference to "methods of treatment" using a given compound, or "uses of the compound" in treatment, are to be construed as "compound for use in a method of treatment".

In a first aspect, the invention relates to obinutuzumab for use in a method of treating lupus nephritis in a human individual having lupus, wherein the method comprises: (a) the treatment of the individual with (i) an effective amount of mycophenolate mofetil, or mycophenolic acid or a salt thereof, and (ii) an effective amount of a glucocorticoid or a corticosteroid; and (b) the administration to the individual of four doses of 1000 mg each of obinutuzumab intravenously (IV) at weeks 0, 2, 24 and 26 of treatment; followed by the administration of an additional dose of 1000 mg obinutuzumab IV every six months thereafter, beginning at week 52 of treatment.

In some embodiments, the obinutuzumab is administered at days 1, 15, 168 and 182 of treatment, and the additional doses are administered every six months starting on day 364 of treatment. In some embodiments, the method further comprises an administration of 1000 mg IV obinutuzumab at week 50, preferably on day 350.

In embodiments of the invention, the first antibody exposure, the second antibody exposure, and the third antibody exposure, are administered intravenously.

In some embodiments, the individual has class III or class IV lupus nephritis. In some embodiments, the individual is at risk for developing class III or class IV lupus nephritis. In some embodiments, the individual has class III (C) or class IV (C) lupus nephritis. In some embodiments, the individual has concomitant class V lupus nephritis.

In some embodiments, the immunosuppressive agent comprises mycophenolate mofetil. In some embodiments, the glucocorticoid or corticosteroid comprises methylprednisolone. In some embodiments, the glucocorticoid or corticosteroid comprises prednisone. In some embodiments, the method further comprises administering to the individual an effective amount of an antihistamine. In some embodiments, the antihistamine comprises diphenhydramine. In some embodiments, the further comprises administering to the individual an effective amount of a non-steroidal anti-inflammatory drug (NSAID). In some embodiments, the NSAID comprises acetaminophen. In some embodiments, the method further comprises administering to the individual an effective amount of an antihypertensive agent. In some embodiments, the antihypertensive agent is an angiotensin-converting enzyme (ACE) inhibitor or an angiotensin-receptor blocker.

In some embodiments, the individual is administered at least 1500 mg/day mycophenolate mofetil starting with 1500 mg on day 1 of treatment, and then increasing the dose by 500 mg/week to a dose of between 2.0 g/day and 2.5 g/day by week 4 of treatment.

In some embodiments, the individual is administered oral prednisone at a dose of 0.5 mg/kg/day on day 2 of the treatment and until week 2, then tapered to a dose of 5 mg/day by week 24 of the treatment. In some embodiments, the individual is administered an intravenous infusion of methylprednisolone at weeks 0, 2, 24 and 26 of the treatment.

In some embodiments, 50 mg diphenhydramine is administered orally between 30 and 60 minutes prior to a dose of obinutuzumab.

In some embodiments, between 650 mg and 1000 mg acetaminophen are administered orally between 30 and 60 minutes prior to a dose of obinutuzumab.

Ideally, the method results in a complete renal response (CRR) in the individual, or at least in a partial renal response (PRR) in the individual. Typically, the method results in a depletion of circulating peripheral B cells in the individual, and in particular of the circulating peripheral CD19+ B cells. The B cells may be naive B cells *(e.g.,* CD19+ CD27- B cells), memory B cells *(e.g.,* CD19+ CD27+ B cells), or plasmablasts *(e.g.,* CD19+ CD27+ CD38++ B cells). In some cases, the B cells are CD19+CD3-CD14-CD33-CD56- cells. In some cases, after administration of the type II anti-CD20 antibody, B cells are depleted to a level such that circulating peripheral B cells are present in peripheral blood from the individual at about 5 cells/$\mu$L or fewer. In some cases, B cells are depleted to a level such that circulating peripheral B cells are present in peripheral blood from the individual at about 1 cells/$\mu$L or fewer. In some cases, B cells are depleted to a level such that circulating peripheral B cells are present in peripheral blood from the individual at about 0.5 cells/$\mu$L or fewer. In some cases, B cells are depleted to a level such that circulating peripheral B cells are present in peripheral blood from the individual the depletion is achieved after the first antibody exposure. In some cases, B cells are depleted to a level that is below the detectable limit using HSFC. In some cases, the HSFC has a lower limit of quantitation (LLOQ) for B cells of about 1.0 cells/$\mu$L or fewer, about 0.8 cells/$\mu$L or fewer, about 0.6 cells/$\mu$L or fewer, about 0.5 cells/$\mu$L or fewer, or 0.441 cells/$\mu$L or fewer. With proper treatment as described, B cell depletion can be sustained for at least 52 weeks after the first dose of the first antibody exposure. In some cases, after administration of the type II anti-CD20 antibody, circulating peripheral B cells in the individual are depleted by at least about 90%, as compared to a corresponding measurement in the same individual before administration of the type II anti-CD20 antibody, or as compared to a corresponding measurement in an individual that has not received treatment with a type II anti-CD20 antibody.

In embodiments of the invention, the first antibody exposure comprises two doses of 1000mg of the type II anti-CD20 antibody on weeks 0 and 2 of treatment; the second antibody exposure comprises two doses of 1000mg of the type II anti-CD20 antibody on weeks 24 and 26 of treatment; the third antibody exposure comprises one dose of 1000 mg of the type II anti-CD20 antibody on week 52 of treatment; the type II anti-CD20 antibody is obinutuzumab; the type II anti-CD20 antibody is administered intravenously. In some embodiments, the first antibody exposure comprises two doses of 1000mg of the type II anti-CD20 antibody on days 1 and 15 of treatment; the second antibody exposure comprises two doses of 1000mg of the type II anti-CD20 antibody on days 168 and 182 of treatment; the third antibody exposure comprises one dose of 1000 mg of the type II anti-CD20 antibody on day 364 of treatment; the type II anti-CD20 antibody is obinutuzumab. In some embodiments, the first antibody exposure comprises two doses of 1000mg of the type II anti-CD20 antibody on days 1 and 15 of treatment; the second antibody exposure comprises two doses of 1000mg of the type II anti-CD20 antibody on days 168 and 182 of treatment; the third antibody exposure comprises two doses of 1000 mg of the type II anti-CD20 antibody on days 350 and 364 of treatment; the type II anti-CD20 antibody is obinutuzumab. In some embodiments, the first antibody exposure comprises two doses of 1000mg of the type II anti-CD20 antibody on weeks 0 and 2 of treatment; the second antibody exposure comprises two doses of 1000mg of the type II anti-CD20 antibody on weeks 24 and 26 of treatment; the third antibody exposure comprises two doses of 1000 mg of the type II anti-CD20 antibody on weeks 50 and 52 of treatment; the type II anti-CD20 antibody is obinutuzumab.

In some embodiments of the invention, the immunosuppressant of choice is mycophenolate mofetil. In some embodiments, mycophenolate mofetil is administered to the individual at a dose of at least 1500mg/day on day 1 of treatment. In some embodiments, mycophenolate mofetil is administered to the individual at a dose of at least 1500mg/day on day 1 of treatment, and then increasing the dose by 500mg/week to a dose of between 2.0g/day and 2.5g/day by week 4 of treatment. In some embodiments, the corticosteroid of choice is oral prednisone. In some embodiments, oral prednisone is administered to the individual at a dose of 0.5mg/kg/day on day 2 of treatment. In some embodiments, oral prednisone is administered to the individual at a dose of 0.5mg/kg/day on day 2 until week 2, then tapered to a dose of 5mg/day by week 24 of treatment. In some embodiments, the corticosteroid of choice is methylprednisolone administered by intravenous (IV) infusion at weeks 0, 2, 24, and 52 of treatment. In some embodiments, the methods comprise administering to the individual methylprednisolone by intravenous (IV) infusion at weeks 0, 2, 24, 26, and 52 of treatment. In some embodiments, the methods further comprise administering to the individual acetaminophen at between 650mg and 1000mg orally between 30 and 60 minutes prior to one or more doses of obinutuzumab. In some embodiments, the methods further comprise administering to the individual acetaminophen at between 650mg and 1000mg orally between 30 and 60 minutes prior to each dose of the type II anti-CD20 antibody. In some embodiments, the methods further comprise administering to the individual diphenhydramine at 50mg orally between 30 and 60 minutes prior to one or more doses of the type II anti-CD20 antibody. In some embodiments, the methods further comprise administering to the individual diphenhydramine at 50mg orally between 30 and 60 minutes prior to each dose of obinutuzumab.

According to the invention, the treatment includes the administration of an effective amount of a glucocorticoid or corticosteroid. In some embodiments, the glucocorticoid or corticosteroid comprises methylprednisolone. In some embodiments, 80mg methylprednisolone are administered intravenously to the individual between 30 and 60 minutes prior to one or more doses of the type II anti-CD20 antibody. In some embodiments, the method further comprises administering to the individual an effective amount of an antihistamine. In some embodiments, the antihistamine comprises diphenhydramine. In some embodiments, 50mg diphenhydramine are administered orally to the individual between 30 and 60 minutes prior to one or more doses of the type II anti-CD20 antibody. In some embodiments, the further comprises administering to the individual an effective amount of a non-steroidal anti-inflammatory drug (NSAID). In some embodiments, the NSAID comprises acetaminophen. In some embodiments, 650-1000mg acetaminophen are administered orally to the individual between 30 and 60 minutes prior to one or more doses of the type II anti-CD20 antibody.

Ideally, the method results in a complete renal response (CRR) in the individual, or at least in a partial renal response (PRR) in the individual. Typically, the method results in a depletion of circulating peripheral B cells in the individual, in particular circulating peripheral CD19+ B cells. In some cases, the B cells are naive B cells (e.g., CD19+ CD27- B cells), memory B cells (e.g., CD19+ CD27+ B cells), or plasmablasts (e.g., CD19+ CD27+ CD38++ B cells). In some cases, the B cells are CD19+CD3-CD14-CD33-CD56- cells. In some cases, after administration of the type II anti-CD20 antibody, B cells are depleted to a level such that circulating peripheral B cells are present in peripheral blood from the individual at about 5 cells/$\mu$L or fewer. In some cases, B cells are depleted to a level such that circulating peripheral B cells are present in peripheral blood from the individual at about 1 cells/$\mu$L or fewer. In some cases, B cells are depleted to a level such that circulating peripheral B cells are present in peripheral blood from the individual at about 0.5 cells/$\mu$L or fewer. In some cases, B cells are depleted to a level such that circulating peripheral B cells are present in peripheral blood from the individual the depletion is achieved after the first antibody exposure. In some cases, B cells are depleted to a level that is below the detectable limit using HSFC. In some cases, the HSFC has a lower limit of quantitation (LLOQ) for B cells of about 1.0 cells/$\mu$L or fewer, about 0.8 cells/$\mu$L or fewer, about 0.6 cells/$\mu$L or fewer, about 0.5 cells/$\mu$L or fewer, or 0.441 cells/$\mu$L or fewer. If the treatment is appropriate, B cell depletion can be sustained for at least 52 weeks after the first dose of the first antibody exposure. In some cases, after administration of the type II anti-CD20 antibody, circulating peripheral B cells in the individual are depleted by at least about 90%, as compared to a corresponding measurement in the same individual before administration of the type II anti-CD20 antibody, or as compared to a corresponding measurement in an individual that has not received treatment with a type II anti-CD20 antibody.

BRIEF DESCRIPTION OF THE FIGURES

FIG. 1A shows renal responses achieved at weeks 52 and 76 in the obinutuzumab and placebo treatment groups. CRR = complete renal response; PRR = partial renal response.

FIG. 1B shows renal response kinetics in the obinutuzumab and placebo treatment groups. CRR = complete renal response; PRR = partial renal response; ORR = overall renal response.

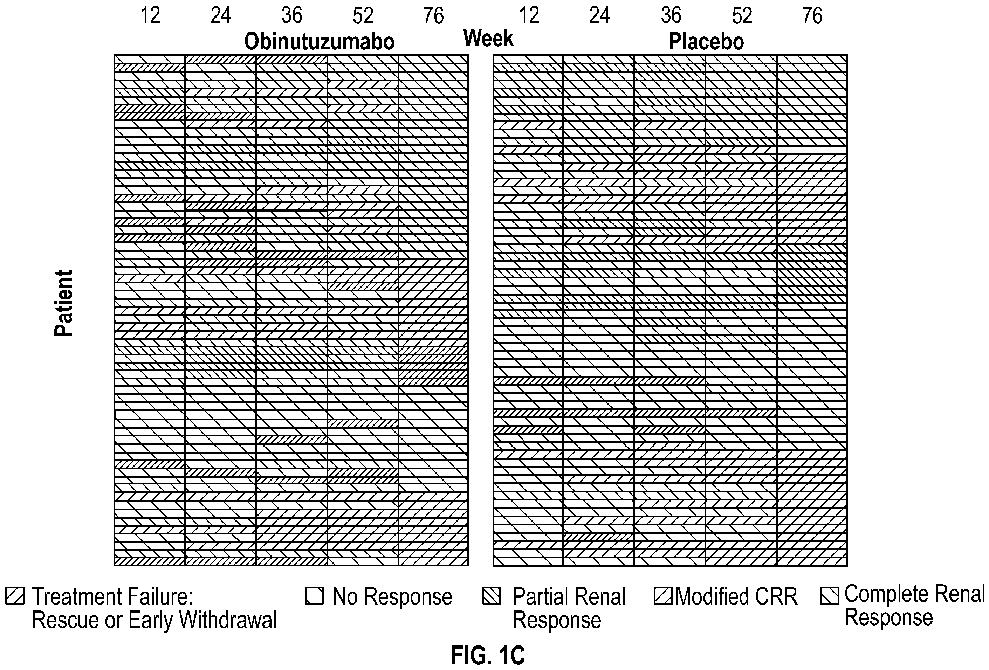

FIG. 1C shows a patient-level renal response heatmap. Rows represent individual patients; columns represent visits. UPCR = urine protein to creatinine ratio; SCr = serum creatinine; ULN = upper limit of normal. Complete renal response required urine protein to creatinine ratio (UPCR) <0.5, serum creatinine less than the upper limit of normal and not increased from baseline by >15%, and urine red blood cell (RBC) <10/hpf without RBC casts. Modified complete renal response required UPCR < 0.5 with serum creatinine less than the upper limit of normal. Partial renal response required UPCR ≥50% reduction from baseline to <1 (to <3 if baseline ≥3), serum creatinine not increased from baseline by >15%, and urine RBC <10/hpf or not increased from baseline by >50%.

FIG. 1D shows mean CD19+ Peripheral B-Cells Over Time Using High-Sensitivity Flow Cytometry. Values <0.441 were imputed as 0.441 for calculation of the mean and SEM. SEM = standard error of the mean.

FIG. 2A shows change from baseline in serologic and laboratory parameters. Mean change from baseline was calculated with the last observation carried forward for missing data. If treatment failure occurred, the last measurement prior to treatment failure was used. SEM = standard error of the mean.

FIG. 2B shows complete response rates (CRR) with permissive serum creatinine criteria over time for the obinutuzumab and placebo cohorts. CRR is defined as UPCR <0.5 and serum creatinine ≤ upper limit of normal.

FIG. 3A shows B-cell depletion kinetics, expressed as the proportions of patients with B-cell depletion using conventional and high-sensitivity flow cytometry.

**FIG. 3B** shows mean B-cell subset measurements over time using high-sensitivity flow cytometry. Values <0.441 were imputed as 0.441 for calculation of the mean and SEM. SEM = standard error of the mean.

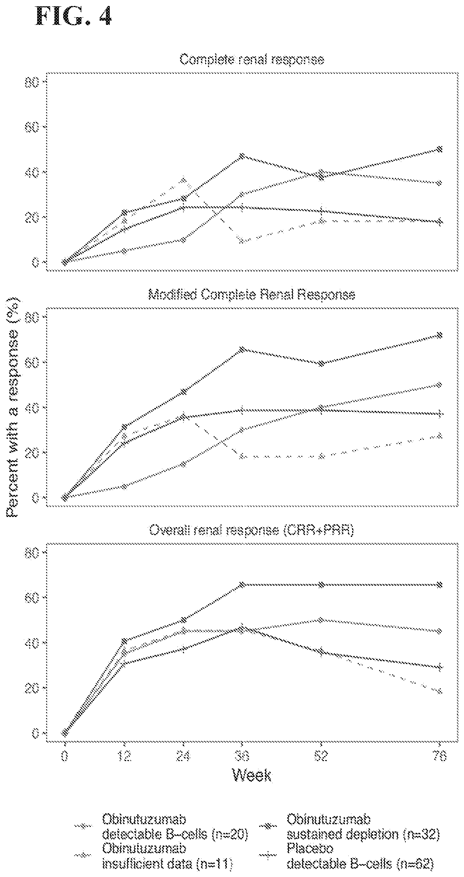

**FIG. 4** shows responses over time by treatment group and peripheral B-cell status. Sustained depletion was present if the peripheral B-cell count was <0.441 at both weeks 24 and 52. In the case of missing data at week 24, sustained depletion was present if the peripheral B-cell count was <0.441 at weeks 12 and 52 in the setting of high obinutuzumab PK at week 24. Detectable B-cells were present if either peripheral B-cell count at week 24 or 52 was >0.441. Obinutuzumab patients with insufficient data to determine status are categorized as "insufficient data." All placebo-treated patients had detectable B-cells at either week 24 or 52. CRR = complete renal response; PRR = partial renal response; UPCR = urine protein to creatinine ratio; SCr = serum creatinine; ULN = upper limit of normal.

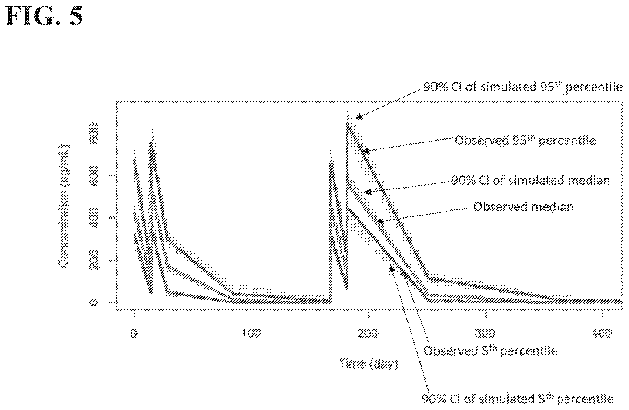

**FIG. 5** shows a visual predictive check (VPC) plot used to validate the pharmacokinetics (PK) model described in Example 2. The solid lines show the median, 5th percentile, and 95th percentile of the observed obinutuzumab concentrations ($\mu$g/mL) at the indicated times (days). The shaded regions show the 90% confidence intervals (90% CI) of simulated median, 5th percentile, and 95th percentile obinutuzumab concentrations ($\mu$g/mL) at the indicated times (days) based on the PK model. The simulated concentrations were computed from 1000 trials with dosing, sampling, and the covariate values of the analysis data set described in Example 2.

FIG. 6 shows predicted obinutuzumab concentration profiles over time based on the PK model described in Example 2 for all patients following the dosing regimen described in Example 1 (1000 mg obinutuzumab on days 0, 14, 168, and 182). Covariate factors and patient's individual random effects were used to simulate concentration profiles. Residual variability was not included. The median, 5th percentile, and 95th percentile simulated obinutuzumab concentrations are plotted as indicated.

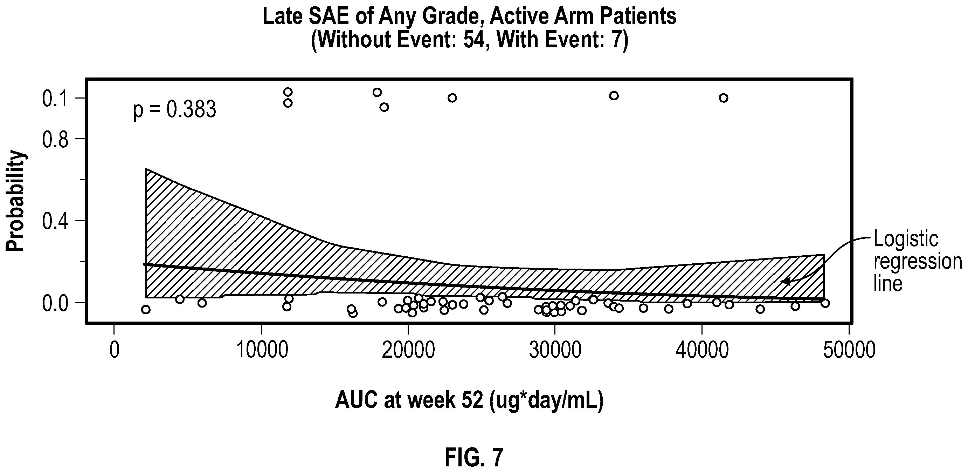

**FIG. 7** is a logistic regression analysis for the probability of occurrences of late SAEs (Serious Adverse Events that occurred after dose 2) of any grade versus cumulative obinutuzumab exposure from treatment start up to week 52 ($AUC_{52}$). Circles illustrate the observed responses (0 = no late SAE event; 1 = late SAE event). The circles are vertically jittered for better visualization. The logistic regression line is indicated. Shaded regions are the 90% confidence intervals (CI) for the regression line. The p value for the relationship between the probability of late SAEs of any grade versus obinutuzumab exposure is provided (p = 0.383). This logistic regression was performed based on late SAE data from Active arm patients only.

FIG. 8 shows the observed B-cell counts versus individual predicted obinutuzumab concentrations from the study described in Example 1 (after 12 weeks following the initial dose of obinutuzumab). Circles with B-cell counts of 0.2 correspond to the observations that were below the B-cell quantification limit (BQL).

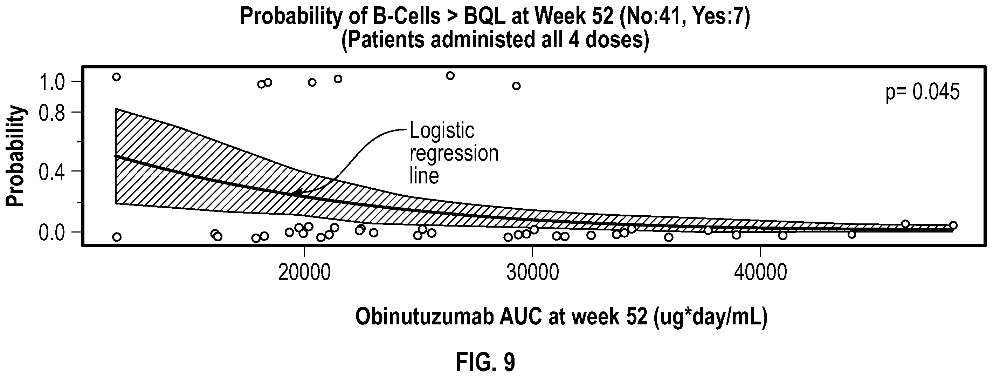

FIG. 9 shows the probability of B-cell counts rebounding above the BQL (B-cells > BQL) at week 52 compared to obinutuzumab exposure ($AUC_{52}$) in patients administered obinutuzumab (1000 mg) on weeks 0, 2, 24, and 26. Circles illustrate the observed B-cell levels (1 = B-cells > BQL; 0 = B-cells < BQL). The logistic regression line is indicated. Shaded regions are the 90% confidence intervals (CI) for the regression line. The p value for the relationship between the probability of B-cell counts rebounding above the BQL (B-cells > BQL) at week 52 versus obinutuzumab exposure is provided (p = 0.045).

**FIG. 10** shows baseline characteristics of patients in the obinutuzumab and control treatment groups. Patients in the obinutuzumab group are further divided into those that would later show sustained B cell depletion in response to obinutuzumab treatment vs. those with detectable B cells in response to obinutuzumab treatment.

FIG. 11 shows serum B-cell activating factor (BAFF) levels (pg/mL) over time (weeks) in patients treated with obinutuzumab and MMF or placebo and MMF.

**FIG. 12** shows percentage of patients showing a response to treatment at week 76 stratified by baseline serum creatinine level.

**FIG.** 13 shows response rates between obinutuzumab and placebo treatment groups at weeks 52 and 76 using alternative response definitions. OBI, obinutuzumab; PBO, placebo; UPCR, urine protein/creatinine ratio; SCr, serum creatinine; ULN, upper limit of normal.

## DETAILED DESCRIPTION

The subject matter for which protection is sought is as defined in the claims.

Although non-clinical data suggested possibility to use both type I anti-CD20 antibodies (rituximab and ocrelizumab) and type II anti-CD20 antibodies (obinutuzumab) to treat lupus, unlike in the case of rituximab and ocrelizumab, obinutuzumab treatment met primary and key secondary efficacy endpoints in a phase II clinical study (NOBILITY). At one year, obinutuzumab treatment resulted in increased complete and partial renal response compared with placebo when added to mycophenolate and corticosteroids for the treatment of proliferative lupus nephritis. Further, obinutuzumab was not associated with increases in rates of serious adverse events or serious infections. Pharmacokinetics (PK) and pharmacodynamics (PD) analyses from the phase II clinical study demonstrated that sustained peripheral B-cell depletion (B-cells below 0.441 cell/$\mu$l) was positively correlated with achievement of complete renal response (CRR) and that obinutuzumab concentrations above 1 $\mu$g/mL are critical for maintaining B-cell depletion. Simulations based on PK modeling of a dosing regimen of 1000 mg obinutuzumab on weeks 0, 2, 24, 26, and 52 showed that an additional single dose of 1000 mg obinutuzumab at 52 weeks is predicted to cause a large percentage of patients to have obinutuzumab concentrations above 1 $\mu$g/mL at week 76. Thus, an additional single dose of obinutuzumab at Week 52 is predicted to maintain obinutuzumab concentration above the critical level of 1 $\mu$g/mL, which is expected to maintain B-cell depletion and translate into better efficacy at Week 76.

Previous obinutuzumab dosing regimens only dosed at weeks 0, 2, 24, and 26 to permit direction comparisons with clinical testing of rituximab. It was also unknown whether obinutuzumab would be effective, and there was no clear rationale for re-dosing without evidence of efficacy. The results presented in Examples 1 and 2 unexpectedly showed that B-cell depletion is predicted to be sustained at week 76 by a dosing regimen of 1000 mg obinutuzumab on weeks 0, 2, 24, 26, and 52, without additional adverse events. Maintenance of B-cell depletion at week 76 is expected to translate into better efficacy (e.g., CRR). Additional doses every 24 weeks thereafter are expected to maintain efficacy. Now that evidence of efficacy and that deeper B cell depletion by obinutuzumab is associated with greater responses, it is believed that continued dosing may maintain B-cell depletion and clinical benefit.

Provided herein are methods for treating lupus nephritis in a human individual, including administering to the individual a first antibody exposure to a type II anti-CD20 antibody, a second antibody exposure to the type II anti-CD20 antibody, and a third second antibody exposure to the type II anti-CD20 antibody. The individual has lupus. The dosing schedule is per the appended claims. The type II anti-CD20 antibody is obinutuzumab.

## I. General Techniques

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3d edition (2001) Cold Spring Harbor Laboratory Press, Cold Spring Harbor, N.Y.; Current Protocols in Molecular Biology (F.M. Ausubel, et al. eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, A Laboratory Manual, and Animal Cell Culture (R.I. Freshney, ed. (1987)); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney), ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Handbook of Experimental Immunology (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

## II. Definitions

The term "lupus nephritis (LN)" refers to a manifestation of lupus (e.g., systemic lupus erythematosus, drug-induced lupus, neonatal lupus, or discoid lupus) in the kidney(s).

The term "antibody" includes monoclonal antibodies (including full length antibodies which have an immunoglobulin Fc region), antibody compositions with polyepitopic specificity, multispecific antibodies (e.g., bispecific antibodies, diabodies, and single-chain molecules, as well as antibody fragments (e.g., Fab, F(ab')$_2$, and Fv). The term "immunoglobulin" (Ig) is used interchangeably with "antibody" herein.

The basic 4-chain antibody unit is a heterotetrameric glycoprotein composed of two identical light (L) chains and two identical heavy (H) chains. An IgM antibody consists of 5 of the basic heterotetramer units along with an additional polypeptide called a J chain, and contains 10 antigen binding sites, while IgA antibodies comprise from 2-5 of the basic 4-chain units which can polymerize to form polyvalent assemblages in combination with the J chain. In the case of IgGs, the 4-chain unit is generally about 150,000 daltons. Each L chain is linked to an H chain by one covalent disulfide bond, while the two H chains are linked to each other by one or more disulfide bonds depending on the H chain isotype. Each H and L chain also has regularly spaced intrachain disulfide bridges. Each H chain has at the N-terminus, a variable domain ($V_H$) followed by three constant domains ($C_H$) for each of the $\alpha$ and $\gamma$ chains and four $C_H$ domains for $\mu$ and $\epsilon$ isotypes. Each L chain has at the N-terminus, a variable domain ($V_L$) followed by a constant domain at its other end. The $V_L$ is aligned with the $V_H$ and the $C_L$ is aligned with the first constant domain of the heavy chain ($C_H1$). Particular amino acid residues are believed to form an interface between the light chain and heavy chain variable domains. The pairing of a $V_H$ and $V_L$ together forms a single antigen-binding site. For the structure and properties of the different classes of antibodies, see *e.g.,* Basic and Clinical Immunology, 8th Edition, Daniel P. Sties, Abba I. Terr and Tristram G. Parsolw (eds), Appleton & Lange, Norwalk, CT, 1994, page 71 and Chapter 6. The L chain from any vertebrate species can be assigned to one of two clearly distinct types, called kappa and lambda, based on the amino acid sequences of their constant domains. Depending on the amino acid sequence of the constant domain of their heavy chains (CH), immunoglobulins can be assigned to different classes or isotypes. There are five classes of immunoglobulins: IgA, IgD, IgE, IgG and IgM, having heavy chains designated $\alpha$, $\delta$, $\epsilon$, $\gamma$ and $\mu$, respectively. The $\gamma$ and $\alpha$ classes are further divided into subclasses on the basis of relatively minor differences in the CH sequence and function, e.g., humans express the following subclasses: IgG1, IgG2A, IgG2B, IgG3, IgG4, IgA1 and IgA2.

The "variable region" or "variable domain" of an antibody refers to the amino-terminal domains of the heavy or light chain of the antibody. The variable domains of the heavy chain and light chain may be referred to as *"VH"* and *"VL"*, respectively. These domains are generally the most variable parts of the antibody (relative to other antibodies of the same class) and contain the antigen binding sites.

The term "variable" refers to the fact that certain segments of the variable domains differ extensively in sequence among antibodies. The V domain mediates antigen binding and defines the specificity of a particular antibody for its particular antigen. However, the variability is not evenly distributed across the entire span of the variable domains. Instead, it is concentrated in three segments called hypervariable regions (HVRs) both in the light-chain and the heavy chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen binding site of antibodies (see Kabat et al., Sequences of Immunological Interest, Fifth Edition, National Institute of Health, Bethesda, MD (1991)). The constant domains are not involved directly in the binding of antibody to an antigen, but exhibit various effector functions, such as participation of the antibody in antibody-dependent cellular toxicity.

The term "monoclonal antibody" as used herein refers to an antibody obtained from a population of substantially homogeneous antibodies, *i.e.,* the individual antibodies comprising the population are identical except for possible naturally occurring mutations and/or post-translation modifications (e.g., isomerizations, amidations) that may be present in minor amounts. Monoclonal antibodies are highly specific, being directed against a single antigenic site. In contrast to polyclonal antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method *(e.g.,* Kohler and Milstein., Nature, 256:495-97 (1975); Hongo et al., Hybridoma, 14 (3): 253-260 (1995), Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, *e.g.,* U.S. Patent No. 4,816,567), phage-display technologies (see, *e.g.,* Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132 (2004), and technologies for producing human or human-like antibodies in animals that have parts or all of the human immunoglobulin loci or genes encoding human immunoglobulin sequences (see, *e.g.,* WO 1998/24893; WO 1996/34096; WO 1996/33735; WO 1991/10741; Jakobovits et al., Proc. Natl. Acad. Sci. USA 90: 2551 (1993); Jakobovits et al., Nature 362: 255-258 (1993); Bruggemann et al., Year in Immunol. 7:33 (1993); U.S. Patent Nos. 5,545,807; 5,545,806; 5,569,825; 5,625,126; 5,633,425; and 5,661,016; Marks et al., Bio/Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996); and Lonberg and Huszar, Intern. Rev. Immunol. 13: 65-93 (1995).

The term "naked antibody" refers to an antibody that is not conjugated to a cytotoxic moiety or radiolabel.

The terms "full-length antibody," "intact antibody" or "whole antibody" are used interchangeably to refer to an antibody in its substantially intact form, as opposed to an antibody fragment. Specifically whole antibodies include those with heavy and light chains including an Fc region. The constant domains may be native sequence constant domains (e.g., human native sequence constant domains) or amino acid sequence variants thereof. In some cases, the intact antibody may have one or more effector functions.

An "antibody fragment" comprises a portion of an intact antibody, preferably the antigen binding and/or the variable region of the intact antibody. Examples of antibody fragments include Fab, Fab', F(ab')$_2$ and Fv fragments; diabodies; linear antibodies (see U.S. Patent 5,641,870, Example 2; Zapata et al., Protein Eng. 8(10): 1057-1062 [1995]); single-chain antibody molecules and multispecific antibodies formed from antibody fragments. Papain digestion of antibodies produced two identical antigen-binding fragments, called "Fab" fragments, and a residual "Fc" fragment, a designation reflecting the ability to crystallize readily. The Fab fragment consists of an entire L chain along with the variable region domain of the H chain (V$_H$), and the first constant domain of one heavy chain (C$_H$1). Each Fab fragment is monovalent with respect to antigen binding, *i.e.,* it has a single antigen-binding site. Pepsin treatment of an antibody yields a single large F(ab')$_2$ fragment which roughly corresponds to two disulfide linked Fab fragments having different antigen-binding activity and is still capable of cross-linking antigen. Fab' fragments differ from Fab fragments by having a few additional residues at the carboxy terminus of the C$_H$1 domain including one or more cysteines from the antibody hinge region. Fab'-SH is the designation herein for Fab' in which the cysteine residue(s) of the constant domains bear a free thiol group. F(ab')$_2$ antibody fragments originally were produced as pairs of Fab' fragments which have hinge cysteines between them. Other chemical couplings of antibody fragments are also known.

The Fc fragment comprises the carboxy-terminal portions of both H chains held together by disulfides. The effector functions of antibodies are determined by sequences in the Fc region, the region which is also recognized by Fc receptors (FcR) found on certain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site.

"Single-chain Fv" also abbreviated as "sFv" or "scFv" are antibody fragments that comprise the V$_H$ and V$_L$ antibody domains connected into a single polypeptide chain. Preferably, the sFv polypeptide further comprises a polypeptide linker between the V$_H$ and V$_L$ domains which enables the sFv to form the desired structure for antigen binding. For a review of the sFv, see Pluckthun in The Pharmacology of Monoclonal Antibodies, vol. 113, Rosenburg and Moore eds., Springer-Verlag, New York, pp. 269-315 (1994).

"Functional fragments" of the antibodies of the invention comprise a portion of an intact antibody, generally including the antigen binding or variable region of the intact antibody or the Fc region of an antibody which retains or has modified FcR binding capability. Examples of antibody fragments include linear antibody, single-chain antibody molecules and multispecific antibodies formed from antibody fragments.

The term "diabodies" refers to small antibody fragments prepared by constructing sFv fragments (see preceding paragraph) with short linkers (about 5-10) residues) between the V$_H$ and V$_L$ domains such that inter-chain but not intra-chain pairing of the V domains is achieved, thereby resulting in a bivalent fragment, *i.e.,* a fragment having two antigen-binding sites. Bispecific diabodies are heterodimers of two "crossover" sFv fragments in which the V$_H$ and V$_L$ domains of the two antibodies are present on different polypeptide chains. Diabodies are described in greater detail in, for example, EP 404,097; WO 93/11161; Hollinger et al., Proc. Natl. Acad. Sci. USA 90: 6444-6448 (1993).

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)). Chimeric antibodies of interest herein include PRIMATIZED® antibodies wherein the antigen-binding region of the antibody is derived from an antibody produced by, e.g., immunizing macaque monkeys with an antigen of interest. As used herein, "humanized antibody" is used a subset of "chimeric antibodies."

"Humanized" forms of non-human (e.g., murine) antibodies are chimeric antibodies that contain minimal sequence derived from non-human immunoglobulin. A humanized antibody is a human immunoglobulin (recipient antibody) in which residues from an HVR (hereinafter defined) of the recipient are replaced by residues from an HVR of a non-human species (donor antibody) such as mouse, rat, rabbit or non-human primate having the desired specificity, affinity, and/or capacity. In some instances, framework ("FR") residues of the human immunoglobulin are replaced by corresponding non-human residues. Furthermore, humanized antibodies may comprise residues that are not found in the recipient antibody or in the donor antibody. These modifications may be made to further refine antibody performance, such as binding affinity. In general, a humanized antibody will comprise substantially all of at least one, and typically two, variable domains, in which all or substantially all of the hypervariable loops correspond to those of a non-human immunoglobulin sequence, and all or substantially all of the FR regions are those of a human immunoglobulin sequence, although the FR regions may include one or more individual FR residue substitutions that improve antibody performance, such as binding affinity, isomerization, immunogenicity, *etc.* The number of these amino acid substitutions in the FR are typically no more than 6 in the H chain, and in the L chain, no more than 3. The humanized antibody optionally will also comprise at least a portion of an immunoglobulin constant region (Fc), typically that of a human immunoglobulin. For further details, see, *e.g.,* Jones et al., Nature 321:522-525 (1986); Riechmann et al., Nature 332:323-329 (1988); and Presta, Curr. Op. Struct. Biol. 2:593-596 (1992). See also, for example, Vaswani and Hamilton, Ann. Allergy, Asthma & Immunol. 1: 105-115 (1998); Harris, Biochem. Soc. Transactions 23:1035-1038 (1995); Hurle and Gross, Curr. Op. Biotech. 5:428-433 (1994); and U.S. Pat. Nos. 6,982,321 and 7,087,409.

A "human antibody" is an antibody that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, *e.g.,* immunized xenomice (see, *e.g.,* U.S. Pat. Nos. 6,075,181 and 6,150,584 regarding XENOMOUSE™ technology). See also, for example, Li et al., Proc. Natl. Acad. Sci. USA, 103:3557-3562 (2006) regarding human antibodies generated via a human B-cell hybridoma technology.

The term "hypervariable region," "HVR," or "HV," when used herein refers to the regions of an antibody variable domain which are hypervariable in sequence and/or form structurally defined loops. Generally, antibodies comprise six HVRs; three in the VH (H1, H2, H3), and three in the VL (L1, L2, L3). In native antibodies, H3 and L3 display the most diversity of the six HVRs, and H3 in particular is believed to play a unique role in conferring fine specificity to antibodies. See, e.g., Xu et al., Immunity 13:37-45 (2000); Johnson and Wu, in Methods in Molecular Biology 248:1-25 (Lo, ed., Human Press, Totowa, NJ, 2003). Indeed, naturally occurring camelid antibodies consisting of a heavy chain only are functional and stable in the absence of light chain. See, *e.g.,* Hamers-Casterman et al., Nature 363:446-448 (1993); Sheriff et al., Nature Struct. Biol. 3:733-736 (1996).

A number of HVR delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, MD. (1991)). Chothia refers instead to the location of the structural loops (Chothia and Lesk, J. Mol. Biol. 196:901-917 (1987)). The AbM HVRs represent a compromise between the Kabat HVRs and Chothia structural loops, and are used by Oxford Molecular's AbM antibody modeling software. The "contact" HVRs are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B (Kabat numbering) |
| H1 | H31-H35 | H26-H35 | H26-H32 | H30-H35 (Chothia numbering) |
| H2 | H50-H65 | H50-H58 | H53-H55 | H47-H58 |
| H3 | H95-H102 | H95-H102 | H96-H101 | H93-H101 |

HVRs may comprise "extended HVRs" as follows: 24-36 or 24-34 (L1), 46-56 or 50-56 (L2) and 89-97 or 89-96 (L3) in the VL and 26-35 (H1), 50-65 or 49-65 (H2) and 93-102, 94-102, or 95-102 (H3) in the VH. The variable domain residues are numbered according to Kabat *et al., supra,* for each of these definitions.

The expression "variable-domain residue-numbering as in Kabat" or "amino-acid-position numbering as in Kabat," and variations thereof, refers to the numbering system used for heavy-chain variable domains or light-chain variable domains of the compilation of antibodies in Kabat *et al., supra.* Using this numbering system, the actual linear amino acid sequence may contain fewer or additional amino acids corresponding to a shortening of, or insertion into, a FR or HVR of the variable domain. For example, a heavy-chain variable domain may include a single amino acid insert (residue 52a according to Kabat) after residue 52 of H2 and inserted residues *(e.g.* residues 82a, 82b, and 82c, *etc.* according to Kabat) after heavy-chain FR residue 82. The Kabat numbering of residues may be determined for a given antibody by alignment at regions of homology of the sequence of the antibody with a "standard" Kabat numbered sequence.

"Framework" or "FR" residues are those variable-domain residues other than the HVR residues as herein defined.

A "human consensus framework" or "acceptor human framework" is a framework that represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, 5th Ed. Public Health Service, National Institutes of Health, Bethesda, MD (1991). Examples include for the VL, the subgroup may be subgroup kappa I, kappa II, kappa III or kappa IV as in Kabat *et al., supra.* Additionally, for the VH, the subgroup may be subgroup I, subgroup II, or subgroup III as in Kabat *et al., supra.* Alternatively, a human consensus framework can be derived from the above in which particular residues, such as when a human framework residue is selected based on its homology to the donor framework by aligning the donor framework sequence with a collection of various human framework sequences. An acceptor human framework "derived from" a human immunoglobulin framework or a human consensus framework may comprise the same amino acid sequence thereof, or it may contain pre-existing amino acid sequence changes. The number of pre-existing amino acid changes may be 10 or less, 9 or less, 8 or less, 7 or less, 6 or less, 5 or less, 4 or less, 3 or less, or 2 or less.

A "VH subgroup III consensus framework" comprises the consensus sequence obtained from the amino acid sequences in variable heavy subgroup III of Kabat *et al., supra.* The VH subgroup III consensus framework amino acid sequence may comprise at least a portion or all of each of the following sequences: EVQLVESGGGLVQPGGSLRLSCAAS (HC-FR1)(SEQ ID NO:35), WVRQAPGKGLEWV (HC-FR2), (SEQ ID NO:36), RFTISADTSKNTAYLQMNSLRAEDTAVYYCAR (HC-FR3, SEQ ID NO:37), WGQGTLVTVSA (HC-FR4), (SEQ ID NO:38).

A "VL kappa I consensus framework" comprises the consensus sequence obtained from the amino acid sequences in variable light kappa subgroup I of Kabat *et al., supra.* The VH subgroup I consensus framework amino acid sequence may comprise at least a portion or all of each of the following sequences: DIQMTQSPSSLSASVGDRVTITC (LC-FR1) (SEQ ID NO:39), WYQQKPGKAPKLLIY (LC-FR2) (SEQ ID NO:40), GVPSRFSGSGSGTDFTLTISSLQPEDFATYYC (LC-FR3)(SEQ ID NO:41), FGQGTKVEIKR (LC-FR4)(SEQ ID NO:42).

An "amino-acid modification" at a specified position, *e.g.* of the Fc region, refers to the substitution or deletion of the specified residue, or the insertion of at least one amino acid residue adjacent the specified residue. Insertion "adjacent" to a specified residue means insertion within one to two residues thereof. The insertion may be N-terminal or C-terminal to the specified residue. The preferred amino acid modification herein is a substitution.

An "affinity-matured" antibody is one with one or more alterations in one or more HVRs thereof that result in an improvement in the affinity of the antibody for antigen, compared to a parent antibody that does not possess those alteration(s). An affinity-matured antibody may have nanomolar or even picomolar affinities for the target antigen. Affinity-matured antibodies are produced by procedures known in the art. For example, Marks et al., Bio/Technology 10:779-783 (1992) describes affinity maturation by VH- and VL-domain shuffling. Random mutagenesis of HVR and/or framework residues is described by, for example: Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

As use herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. The extent of binding of an antibody to an unrelated target may be less than about 10% of the binding of the antibody to the target as measured, *e.g.*, by a radioimmunoassay (RIA). An antibody that specifically binds to a target may have a dissociation constant (Kd) of ≤ 1 μM, ≤ 100 nM, ≤ 10 nM, ≤ 1 nM, or ≤ 0.1 nM. An antibody may specifically bind to an epitope on a protein that is conserved among the protein from different species. Specific binding can include, but does not require exclusive binding.

The term "Fc region" herein is used to define a C-terminal region of an immunoglobulin heavy chain, including native-sequence Fc regions and variant Fc regions. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. Suitable native-sequence Fc regions for use in the antibodies of the invention include human IgG1, IgG2 (IgG2A, IgG2B), IgG3 and IgG4.

"Fc receptor" or "FcR" describes a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of

these receptors, FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see M. Daëron, Annu. Rev. Immunol. 15:203-234 (1997). FcRs are reviewed in Ravetch and Kinet, Annu. Rev. Immunol. 9: 457-92 (1991); Capel et al., Immunomethods 4: 25-34 (1994); and de Haas et al., J. Lab. Clin. Med. 126: 330-41 (1995). Other FcRs, including those to be identified in the future, are encompassed by the term "FcR" herein.

The term "Fc receptor" or "FcR" also includes the neonatal receptor, *FcRn,* which is responsible for the transfer of maternal IgGs to the fetus. Guyer et al., J. Immunol. 117: 587 (1976) and Kim et al., J. Immunol. 24: 249 (1994). Methods of measuring binding to FcRn are known (see, *e.g.,* Ghetie and Ward, Immunol. Today 18: (12): 592-8 (1997); Ghetie et al., Nature Biotechnology 15 (7): 637-40 (1997); Hinton et al., J. Biol. Chem. 279 (8): 6213-6 (2004); WO 2004/92219 (Hinton et al.). Binding to FcRn in vivo and serum half-life of human FcRn high-affinity binding polypeptides can be assayed, e.g., in transgenic mice or transfected human cell lines expressing human FcRn, or in primates to which the polypeptides having a variant Fc region are administered. WO 2004/42072 (Presta) describes antibody variants which improved or diminished binding to FcRs. See also, *e.g.,* Shields et al., J. Biol. Chem. 9(2): 6591-6604 (2001).

The phrase "substantially reduced," or "substantially different," as used herein, denotes a sufficiently high degree of difference between two numeric values (generally one associated with a molecule and the other associated with a reference/comparator molecule) such that one of skill in the art would consider the difference between the two values to be of statistical significance within the context of the biological characteristic measured by said values *(e.g.,* Kd values). The difference between said two values is, for example, greater than about 10%, greater than about 20%, greater than about 30%, greater than about 40%, and/or greater than about 50% as a function of the value for the reference/comparator molecule.

The term "substantially similar" or "substantially the same," as used herein, denotes a sufficiently high degree of similarity between two numeric values (for example, one associated with an antibody of the invention and the other associated with a reference/comparator antibody), such that one of skill in the art would consider the difference between the two values to be of little or no biological and/or statistical significance within the context of the biological characteristic measured by said values *(e.g.,* Kd values). The difference between said two values is, for example, less than about 50%, less than about 40%, less than about 30%, less than about 20%, and/or less than about 10% as a function of the reference/comparator value.

"Carriers" as used herein include pharmaceutically acceptable carriers, excipients, or stabilizers that are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

A "package insert" refers to instructions customarily included in commercial packages of medicaments that contain information about the indications customarily included in commercial packages of medicaments that contain information about the indications, usage, dosage, administration, contraindications, other medicaments to be combined with the packaged product, and/or warnings concerning the use of such medicaments, *etc.*

As used herein, the term "treatment" refers to clinical intervention designed to alter the natural course of the individual or cell being treated during the course of clinical pathology. Desirable effects of treatment include, but are not limited to, decreasing the rate of disease progression, ameliorating or palliating the disease state, remission or improved prognosis, and delaying disease progression. For example, an individual is successfully "treated" if one or more symptoms associated with lupus nephritis are mitigated or eliminated, including, but are not limited to, elevated serum creatinine, proteinuria, red cell casts, reduced renal function, nephrotic syndrome, granular casts, microhematuria, macrohematuria, hypertension, tubular abnormalities, hyperkalemia, rapidly progressive glomerulonephritis (RPGN), and acute renal failure (ARF). Delaying progression of a disease (*e.g.,* lupus nephritis) means to defer, hinder, slow, retard, stabilize, and/or postpone development of the disease. This delay can be of varying lengths of time, depending on the history of the disease and/or individual being treated. As is evident to one skilled in the art, a sufficient or significant delay can, in effect, encompass prevention, in that the individual, e.g., an individual at risk for developing the disease, does not develop the disease. For example, the progression of SLE in an individual before the onset of LN symptoms and/or pathology may be delayed such that the development of LN is postponed or prevented.

As used herein, "complete renal response (CRR)" refers to a response to treatment that includes a normalization of serum creatinine, inactive urinary sediment, and a urinary protein to creatinine ratio of less than 0.5.

As used herein, "partial renal response (PRR)" refers to a response to treatment that is less than a CRR but still includes mitigation of one or more symptoms including without limitation a reduction in serum creatinine, reduced urinary sediment, and a reduction in proteinuria.

An "effective amount" is at least the minimum concentration required to effect a measurable improvement or prevention of a particular disorder. An effective amount herein may vary according to factors such as the disease state, age, sex, and weight of the patient, and the ability of the antibody to elicit a desired response in the individual. An effective amount is also one in which any toxic or detrimental effects of the treatment are outweighed by the therapeutically beneficial effects. For prophylactic use, beneficial or desired results include results such as eliminating or reducing the risk, lessening the severity, or delaying the onset of the disease, including biochemical, histological and/or behavioral symptoms of the disease, its complications and intermediate pathological phenotypes presenting during development of the disease. For therapeutic use, beneficial or desired results include clinical results such as decreasing one or more symptoms resulting from the disease, increasing the quality of life of those suffering from the disease, decreasing the dose of other medications required to treat the disease, enhancing effect of another medication such as via targeting, delaying the progression of the disease, and/or prolonging survival. In the case of lupus nephritis, an effective amount of the drug may have the effect in and/or relieving to some extent one or more of the symptoms associated with the disorder. An effective amount can be administered in one or more administrations. For purposes of this invention, an effective amount of drug, compound, or pharmaceutical composition is an amount sufficient to accomplish prophylactic or therapeutic treatment either directly or indirectly. As is understood in the clinical context, an effective amount of a drug, compound, or pharmaceutical composition may or may not be achieved in conjunction with another drug, compound, or pharmaceutical composition. Thus, an "effective amount" may be considered in the context of administering one or more therapeutic agents, and a single agent may be considered to be given in an effective amount if, in conjunction with one or more other agents, a desirable result may be or is achieved.

"CD20" as used herein refers to the human B-lymphocyte antigen CD20 (also known as CD20, B-lymphocyte surface antigen B1, Leu-16, Bp35, BM5, and LF5; the sequence is characterized by the SwissProt database entry P11836) is a hydrophobic transmembrane protein with a molecular weight of approximately 35 kD located on pre-B and mature B lymphocytes. (Valentine, M.A., et al., J. Biol. Chem. 264(19) (1989 11282-11287; Tedder, T.F., et al, Proc. Natl. Acad. Sci. U.S.A. 85 (1988) 208-12; Stamenkovic, I., et al., J. Exp. Med. 167 (1988) 1975-80; Einfeld, D.A., et al., EMBO J. 7 (1988) 711-7; Tedder, T.F., et al., J. Immunol. 142 (1989) 2560-8). The corresponding human gene is Membrane-spanning 4-domains, subfamily A, member 1, also known as MS4A1. This gene encodes a member of the membrane-spanning 4A gene family. Members of this nascent protein family are characterized by common structural features and similar intron/exon splice boundaries and display unique expression patterns among hematopoietic cells and nonlymphoid tissues. This gene encodes the B-lymphocyte surface molecule which plays a role in the development and differentiation of B-cells into plasma cells. This family member is localized to 11q12, among a cluster of family members. Alternative splicing of this gene results in two transcript variants which encode the same protein.

The terms "CD20" and "CD20 antigen" are used interchangeably herein, and include any variants, isoforms and species homologs of human CD20 which are naturally expressed by cells or are expressed on cells transfected with the CD20 gene. Binding of an antibody of the invention to the CD20 antigen mediate the killing of cells expressing CD20 (e.g., a tumor cell) by inactivating CD20. The killing of the cells expressing CD20 may occur by one or more of the following mechanisms: Cell death/apoptosis induction, ADCC and CDC.

Synonyms of CD20, as recognized in the art, include B-lymphocyte antigen CD20, B-lymphocyte surface antigen B1, Leu-16, Bp35, BM5, and LF5.

The term "anti-CD20 antibody" as referred to herein is an antibody that binds specifically to CD20 antigen. Depending on binding properties and biological activities of anti-CD20 antibodies to the CD20 antigen, two types of anti-CD20 antibodies (type I and type II anti-CD20 antibodies) can be distinguished according to Cragg, M.S., et al., Blood 103 (2004) 2738-2743; and Cragg, M.S., et al., Blood 101 (2003) 1045-1052, see Table 1 below.

**Table 1.** Properties of type I and type II anti-CD20 antibodies

| Type I anti-CD20 antibodies | type II anti-CD20 antibodies |
| --- | --- |
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity (if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

Examples of type II anti-CD20 antibodies include e.g. humanized B-Ly1 antibody IgG1 (a chimeric humanized IgG1 antibody as disclosed in WO 2005/044859), 11B8 IgG1 (as disclosed in WO 2004/035607), and AT80 IgG1. Typically type II anti-CD20 antibodies of the IgG1 isotype show characteristic CDC properties. Type II anti-CD20 antibodies have a decreased CDC (if IgG1 isotype) compared to type I antibodies of the IgG1 isotype.

Examples of type I anti-CD20 antibodies include e.g. rituximab, HI47 IgG3 (ECACC, hybridoma), 2C6 IgG1 (as disclosed in WO 2005/103081), 2F2 IgG1 (as disclosed and WO 2004/035607 and WO 2005/103081) and 2H7 IgG1 (as disclosed in WO 2004/056312).

The afucosylated anti-CD20 antibodies as referred to herein are preferably type II anti-CD20 antibodies, more preferably afucosylated humanized B-Ly1 antibodies as described in WO 2005/044859 and WO 2007/031875.

The "rituximab" antibody (reference antibody; example of a type I anti-CD20 antibody) is a genetically engineered chimeric human gamma 1 murine constant domain containing monoclonal antibody directed against the human CD20 antigen. However this antibody is not glycoengineered and not afocusylates and thus has an amount of fucose of at least 85 %. This chimeric antibody contains human gamma 1 constant domains and is identified by the name "C2B8" in US 5,736,137 (Andersen, et. al.) issued on April 17, 1998, assigned to IDEC Pharmaceuticals Corporation. Rituximab is approved for the treatment of patients with relapsed or refracting low-grade or follicular, CD20 positive, B cell non-Hodgkin's lymphoma. In vitro mechanism of action studies have shown that rituximab exhibits human complement-dependent cytotoxicity (CDC) (Reff, M.E., et. al, Blood 83(2) (1994) 435-445). Additionally, it exhibits activity in assays that measure antibody-dependent cellular cytotoxicity (ADCC).

The term "GA101 antibody" as used herein refers to any one of the following antibodies that bind human CD20: (1) an antibody comprising an HVR-H1 comprising the amino acid sequence of SEQ ID NO: 1, an HVR-H2 comprising the amino acid sequence of SEQ ID NO:2, an HVR-H3 comprising the amino acid sequence of SEQ ID NO:3, an HVR-L1 comprising the amino acid sequence of SEQ ID NO:4, an HVR-L2 comprising the amino acid sequence of SEQ ID NO:5, and an HVR-L3 comprising the amino acid sequence of SEQ ID NO:6; (2) an antibody comprising a VH domain comprising the amino acid sequence of SEQ ID NO:7 and a VL domain comprising the amino acid sequence of SEQ ID NO:8, (3) an antibody comprising an amino acid sequence of SEQ ID NO:9 and an amino acid sequence of SEQ ID NO: 10; (4) an antibody known as obinutuzumab, or (5) an antibody that comprises an amino acid sequence that has at least 95%, 96%, 97%, 98% or 99% sequence identity with amino acid sequence of SEQ ID NO:9 and that comprises an amino acid sequence that has at least 95%, 96%, 97%, 98% or 99% sequence identity with an amino acid sequence of SEQ ID NO:10. The GA101 antibody may be an IgG1 isotype antibody. The anti-CD20 antibody may also be a humanized B-Ly1 antibody. In the invention, obinutuzumab is used.

The term "humanized B-Ly1 antibody" refers to humanized B-Ly1 antibody as disclosed in WO 2005/044859 and WO 2007/031875, which were obtained from the murine monoclonal anti-CD20 antibody B-Ly1 (variable region of the murine heavy chain (VH): SEQ ID NO: 11; variable region of the murine light chain (VL): SEQ ID NO: 12- see Poppema, S. and Visser, L., Biotest Bulletin 3 (1987) 131-139) by chimerization with a human constant domain from IgG1 and following humanization (see WO 2005/044859 and WO 2007/031875). These "humanized B-Ly1 antibodies" are disclosed in detail in WO 2005/ 044859 and WO 2007/031875.

Variable region of the murine monoclonal anti-CD20 antibody B-Ly1 heavy chain (VH) (SEQ ID NO: 11)

```
Gly Pro Glu Leu Val Lys Pro Gly Ala Ser Val Lys Ile Ser Cys
Lys
 1               5               10              15
Ala Ser Gly Tyr Ala Phe Ser Tyr Ser Trp Met Asn Trp Val Lys
Leu
            20              25              30
Arg Pro Gly Gln Gly Leu Glu Trp Ile Gly Arg Ile Phe Pro Gly
Asp
            35              40              45
Gly Asp Thr Asp Tyr Asn Gly Lys Phe Lys Gly Lys Ala Thr Leu
Thr
        50              55              60
Ala Asp Lys Ser Ser Asn Thr Ala Tyr Met Gln Leu Thr Ser Leu
Thr
65              70              75              80
Ser Val Asp Ser Ala Val Tyr Leu Cys Ala Arg Asn Val Phe Asp
Gly
                85              90              95
Tyr Trp Leu Val Tyr Trp Gly Gln Gly Thr Leu Val Thr Val Ser
Ala
            100             105             110
```

Variable region of the murine monoclonal anti-CD20 antibody B-Ly1 light chain (VL) (SEQ ID NO: 12)

```
Asn Pro Val Thr Leu Gly Thr Ser Ala Ser Ile Ser Cys Arg Ser
Ser
 1               5               10              15
Lys Ser Leu Leu His Ser Asn Gly Ile Thr Tyr Leu Tyr Trp Tyr
Leu
            20              25              30
Gln Lys Pro Gly Gln Ser Pro Gln Leu Leu Ile Tyr Gln Met Ser
Asn
            35              40              45
Leu Val Ser Gly Val Pro Asp Arg Phe Ser Ser Ser Gly Ser Gly
Thr
        50              55              60
Asp Phe Thr Leu Arg Ile Ser Arg Val Glu Ala Glu Asp Val Gly
Val
65              70              75              80

Tyr Tyr Cys Ala Gln Asn Leu Glu Leu Pro Tyr Thr Phe Gly Gly
Gly
                85              90              95
Thr Lys Leu Glu Ile Lys Arg
            100
```

The "humanized B-Ly1 antibody" can have a variable region of the heavy chain (VH) selected from group of SEQ ID NO:7, 8, and 13 to 33 (corresponding to, *inter alia,* B-HH2 to B-HH9 and B-HL8 to B-HL17 of WO 2005/044859 and WO 2007/031875). Such variable domain may be selected from the group consisting of SEQ ID NOS:14, 15, 7, 19, 25, 27, and 29 (corresponding to B-HH2, BHH-3, B-HH6, B-HH8, B-HL8, B-HL11 and B-HL13 of WO 2005/044859 and WO 2007/031875). The "humanized B-Ly1 antibody" may have a variable region of the light chain (VL) of SEQ ID NO:8 (corresponding to B-KV1 of WO 2005/044859 and WO 2007/031875). The "humanized B-Ly1 antibody" may have a variable region of the heavy chain (VH) of SEQ ID NO:7 (corresponding to B-HH6 of WO 2005/044859 and WO 2007/031875) and a variable region of the light chain (VL) of SEQ ID NO:8 (corresponding to B-KV1 of WO 2005/044859 and WO 2007/031875). Furthermore, the humanized B-Ly1 antibody may be an IgG1 antibody. Such afocusylated humanized B-Ly1 antibodies may be glycoengineered (GE) in the Fc region according to the procedures described in WO 2005/044859, WO 2004/065540, WO 2007/031875, Umana, P. et al., Nature Biotechnol. 17 (1999) 176-180 and WO 99/154342. The afucosylated glyco-engineered humanized B-Ly1 is B-HH6-B-KV1 GE. According to the invention, as per the appended claims, the anti-CD20 antibody is obinutuzumab (recommended INN, WHO Drug Information, Vol. 26, No. 4, 2012, p. 453). As used herein, obinutuzumab is synonymous for GA101 or RO5072759. This replaces all previous versions (e.g. Vol. 25, No. 1, 2011, p.75-76), and is formerly known as afutuzumab (recommended INN, WHO Drug Information, Vol. 23, No. 2, 2009, p. 176;Vol. 22, No. 2, 2008, p. 124). As used herein, references to obinutuzumab refer to GAZYVA® as well as biosimilar antibodies thereof. Obinutuzumab is a humanized B-Ly1 antibody and comprises a heavy chain comprising the amino acid sequence of SEQ ID NO:9 and a light chain comprising the amino acid sequence of SEQ ID NO:10 (i.e. obinutuzumab).

Heavy chain (SEQ ID NO:9)

```
QVQLVQSGAE  VKKPGSSVKV  SCKASGYAFS  YSWINWVRQA  PGQGLEWMGR  50
IFPGDGDTDY  NGKFKGRVTI  TADKSTSTAY  MELSSLRSED  TAVYYCARNV  100
FDGYWLVYWG  QGTLVTVSSA  STKGPSVFPL  APSSKSTSGG  TAALGCLVKD  150
YFPEPVTVSW  NSGALTSGVH  TFPAVLQSSG  LYSLSSVVTV  PSSSLGTQTY  200
ICNVNHKPSN  TKVDKKVEPK  SCDKTHTCPP  CPAPELLGGP  SVFLFPPKPK  250

DTLMISRTPE  VTCVVVDVSH  EDPEVKFNWY  VDGVEVHNAK  TKPREEQYNS  300
TYRVVSVLTV  LHQDWLNGKE  YKCKVSNKAL  PAPIEKTISK  AKGQPREPQV  350
YTLPPSRDEL  TKNQVSLTCL  VKGFYPSDIA  VEWESNGQPE  NNYKTTPPVL  400
DSDGSFFLYS  KLTVDKSRWQ  QGNVFSCSVM  HEALHNHYTQ  KSLSLSPG    449
```

Light chain (SEQ ID NO:10)

```
DIVMTQTPLS  LPVTPGEPAS  ISCRSSKSLL  HSNGITYLYW  YLQKPGQSPQ  50
LLIYQMSNLV  SGVPDRFSGS  GSGTDFTLKI  SRVEAEDVGV  YYCAQNLELP  100
YTFGGGTKVE  IKRTVAAPSV  FIFPPSDEQL  KSGTASVVCL  LNNFYPREAK  150
VQWKVDNALQ  SGNSQESVTE  QDSKDSTYSL  SSTLTLSKAD  YEKHKVYACE  200
VTHQGLSSPV  TKSFNRGEC                                      219
```

Obinutuzumab is an afucosylated glyco-engineered humanized B-Ly1. Generally speaking, glycoengineered humanized B-Ly1 antibodies have an altered pattern of glycosylation in the Fc region, preferably having a reduced level of fucose residues. Preferably the amount of fucose is 60 % or less of the total amount of oligosaccharides at Asn297 (for example, the amount of fucose may be between 40 % and 60 %, the amount of fucose may be 50 % or less, and the amount of fucose may be 30 % or less). Furthermore the oligosaccharides of the Fc region are preferably bisected. These glycoengineered humanized B-Ly1 antibodies have an increased ADCC.

The "ratio of the binding capacities to CD20 on Raji cells (ATCC-No. CCL-86) of an anti-CD20 antibodies compared to rituximab" is determined by direct immunofluorescence measurement (the mean fluorescence intensities (MFI) is measured) using said anti-CD20 antibody conjugated with Cy5 and rituximab conjugated with Cy5 in a FACSArray (Becton Dickinson) with Raji cells (ATCC-No. CCL-86), as described in Example No. 2, and calculated as follows:

$$\text{Ratio of the binding capacities to CD20 on Raji cells (ATCC-No. CCL-86)} =$$

$$\frac{\text{MFI(Cy5-anti-CD20 antibody)}}{\text{MFI(Cy5-rituximab)}} \times \frac{\text{Cy5-labeling ratio (Cy5-rituximab)}}{\text{Cy5-labeling ratio (Cy5-anti-CD20 antibody)}}$$

MFI is the mean fluorescent intensity. The "Cy5-labeling ratio" as used herein means the number of Cy5-label molecules per molecule antibody.

Typically said type II anti-CD20 antibody has a ratio of the binding capacities to CD20 on Raji cells (ATCC-No. CCL-86) of said second anti-CD20 antibody compared to rituximab of 0.3 to 0.6, for instance 0.35 to 0.55, or 0.4 to 0.5.

Said type II anti-CD20 antibody, e.g., a GA101 antibody, may have increased antibody dependent cellular cytotoxicity (ADCC).

By "antibody having increased antibody dependent cellular cytotoxicity (ADCC)", it is meant an antibody, as that term is defined herein, having increased ADCC as determined by any suitable method known to those of ordinary skill in the art. One accepted in vitro ADCC assay is as follows:

1) the assay uses target cells that are known to express the target antigen recognized by the antigen-binding region of the antibody;

2) the assay uses human peripheral blood mononuclear cells (PBMCs), isolated from blood of a randomly chosen healthy donor, as effector cells;

3) the assay is carried out according to following protocol:

i) the PBMCs are isolated using standard density centrifugation procedures and are suspended at $5 \times 10^6$ cells/ml in RPMI cell culture medium;

ii) the target cells are grown by standard tissue culture methods, harvested from the exponential growth phase with a viability higher than 90%, washed in RPMI cell culture medium, labeled with 100 micro-Curies of $^{51}$Cr, washed twice with cell culture medium, and resuspended in cell culture medium at a density of $10^5$ cells/ml;

iii) 100 microliters of the final target cell suspension above are transferred to each well of a 96-well microtiter plate;

iv) the antibody is serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above;

v) for the maximum release (MR) controls, 3 additional wells in the plate containing the labeled target cells, receive 50 microliters of a 2% (VN) aqueous solution of non-ionic detergent (Nonidet, Sigma, St. Louis), instead of the antibody solution (point iv above);

vi) for the spontaneous release (SR) controls, 3 additional wells in the plate containing the labeled target cells, receive 50 microliters of RPMI cell culture medium instead of the antibody solution (point iv above);

vii) the 96-well microtiter plate is then centrifuged at 50 x g for 1 minute and incubated for 1 hour at 4°C;

viii) 50 microliters of the PBMC suspension (point i above) are added to each well to yield an effector:target cell ratio of 25:1 and the plates are placed in an incubator under 5% CO2 atmosphere at 37°C for 4 hours;

ix) the cell-free supernatant from each well is harvested and the experimentally released radioactivity (ER) is quantified using a gamma counter;

x) the percentage of specific lysis is calculated for each antibody concentration according to the formula (ER-MR)/(MR-SR) x 100, where ER is the average radioactivity quantified (see point ix above) for that antibody concentration, MR is the average radioactivity quantified (see point ix above) for the MR controls (see point V above), and SR is the average radioactivity quantified (see point ix above) for the SR controls (see point vi above);

4) "increased ADCC" is defined as either an increase in the maximum percentage of specific lysis observed within the antibody concentration range tested above, and/or a reduction in the concentration of antibody required to achieve one half of the maximum percentage of specific lysis observed within the antibody concentration range tested above. The increase may be relative to the ADCC, measured with the above assay, mediated by the same antibody, produced by the same type of host cells, using the same standard production, purification, formulation and storage methods, which are known to those skilled in the art, except that the comparator antibody (lacking increased ADCC) has not been produced by host cells engineered to overexpress GnTIII and/or engineered to have reduced expression from the fucosyltransferase 8 (FUT8) gene (e.g., including, engineered for FUT8 knock out).

Said "increased ADCC" can be obtained by, for example, mutating and/or glycoengineering of said antibodies. The antibody may be glycoengineered to have a biantennary oligosaccharide attached to the Fc region of the antibody that is bisected by GlcNAc, e.g., in WO 2003/011878 (Jean-Mairet et al.); US Patent No. 6,602,684 (Umana et al.); US 2005/0123546 (Umana et al.), Umana, P., et al., Nature Biotechnol. 17 (1999) 176-180). The antibody may be glycoengineered to lack fucose on the carbohydrate attached to the Fc region by expressing the antibody in a host cell that is deficient in protein fucosylation (e.g., Lec13 CHO cells or cells having an alpha-1,6-fucosyltransferase gene (FUT8) deleted or the FUT gene expression knocked down (see, e.g., Yamane-Ohnuki et al. Biotech. Bioeng. 87: 614 (2004); Kanda, Y. et al., Biotechnol. Bioeng., 94(4):680-688 (2006); and WO2003/085107). In yet another instance, the antibody sequence may have been engineered in its Fc region to enhance ADCC (e.g., such engineered antibody variant comprises an Fc region with one or more amino acid substitutions at positions 298, 333, and/or 334 of the Fc region (EU numbering of residues)).

The term "complement-dependent cytotoxicity (CDC)" refers to lysis of human tumor target cells by the antibody according to the invention in the presence of complement. CDC can be measured by the treatment of a preparation of CD20 expressing cells with an anti-CD20 antibody according to the invention in the presence of complement. CDC is found if the antibody induces at a concentration of 100 nM the lysis (cell death) of 20% or more of the tumor cells after 4 hours. For example, the assay may be performed with $^{51}$Cr or Eu labeled tumor cells and measurement of released $^{51}$Cr or Eu. Controls include the incubation of the tumor target cells with complement but without the antibody.

The term "expression of the CD20" antigen is intended to indicate a significant level of expression of the CD20 antigen in a cell, e.g., a T- or B- Cell. Patients to be treated according to the methods of this invention can express significant levels of CD20 on a B-cell. CD20 expression on a B-cell can be determined by standard assays known in the art. e.g., CD20 antigen expression is measured using immunohistochemical (IHC) detection, FACS or via PCR-based detection of the corresponding mRNA.

As used in this specification and the appended claims, the singular forms "a", "an" and "the" include plural referents unless the content clearly dictates otherwise. Thus, for example, reference to "a molecule" optionally includes a combination of two or more such molecules, and the like.

The term "about" as used herein refers to the usual error range for the respective value readily known to the skilled person in this technical field. Reference to "about" a value or parameter herein includes (and describes) embodiments that are directed to that value or parameter per se.

### III. Methods

In one aspect, obinutuzumab is provided herein for use in methods for treating lupus nephritis in an individual that has lupus, wherein the method is defined as per the appended claims. Reference to "methods of treatment" using a given compound, or "uses of the compound" in treatment, are to be construed as "compound for use in a method of treatment". In some embodiments, the lupus nephritis is class III or class IV lupus nephritis. In some embodiments, the individual has class III (C) or class IV (C) lupus nephritis. In some embodiments, the individual has concomitant class V lupus nephritis.

### Anti-CD20 antibodies

According to the claims, the type II anti-CD20 antibody obinutuzumab is for use in the treatment of lupus nephritis (LN). While the scope of the invention is, accordingly, limited to obitnutuzumab, the analogous use of other anti-CD20 antibodies, and in particular type II anti-CD20 antibodies, could be considered. Also, anti-CD20 antibodies as described, and in particular type II anti-CD20 antibodies, could be considered for use in treating or preventing progression of membranous nephropathy (pMN).

Examples of type II anti-CD20 antibodies include e.g. humanized B-Ly1 antibody IgG1 (a chimeric humanized IgG1 antibody as disclosed in WO 2005/044859), 11B8 IgG1 (as disclosed in WO 2004/035607), and AT80 IgG1. Typically type II anti-CD20 antibodies of the IgG1 isotype show characteristic CDC properties. Type II anti-CD20 antibodies have a decreased CDC (if IgG1 isotype) compared to type I antibodies of the IgG1 isotype.

The anti-CD20 antibody may be a GA101 antibody described herein, comprising an HVR-H1 comprising the amino acid sequence of GYAFSY (SEQ ID NO: 1), an HVR-H2 comprising the amino acid sequence of FPGDGDTD (SEQ ID NO:2), an HVR-H3 comprising the amino acid sequence of NVFDGYWLVY (SEQ ID NO:3), an HVR-L1 comprising the amino acid sequence of RSSKSLLHSNGITYLY (SEQ ID NO:4), an HVR-L2 comprising the amino acid sequence of QMSNLVS (SEQ ID NO:5), and an HVR-L3 comprising the amino acid sequence of AQNLELPYT (SEQ ID NO:6).

The anti-CD20 antibody may comprise a heavy chain variable region (VH) comprising the amino acid sequence of SEQ ID NO:7, and a light chain variable region (VL) comprising the amino acid sequence of SEQ ID NO:8.

QVQLVQSGAEVKKPGSSVKVSCKAS**GYAFSY**SWINWVRQAPGQGLEWMGRI**FPGDGDTD**YNGKFKGRVTITADKSTSTAYMELSSLRSEDTAVYYCAR**NVFDGYWLVY**WGQGTLVTVSS (SEQ ID NO:7)

DIVMTQTPLSLPVTPGEPASISC**RSSKSLLHSNGITYLY**WYLQKPGQSPQLLIY**QMSNLVS**GVPDRFSGSGSGTDFTLKISRVEAEDVGVYYC**AQNLELPYT**FGGGTKVEIKRTV (SEQ ID NO:8).

The anti-CD20 antibody may comprise a heavy chain comprising the amino acid sequence of SEQ ID NO:9, and a light chain comprising the amino acid sequence of SEQ ID NO:10.

QVQLVQSGAEVKKPGSSVKVSCKAS*GYAFSY*SWINWVRQAPGQGLEWMGRI*FPGDGDTD*YNG KFKGRVTITADKSTSTAYMELSSLRSEDTAVYYCAR*NVFDGYWLVY*WGQGTLVTVSSASTKG PSVFPLAPSSKSTSGGTAALGCLVKDYFPEPVTVSWNSGALTSGVHTFPAVLQSSGLYSLSS VVTVPSSSLGTQTYICNVNHKPSNTKVDKKVEPKSCDKTHTCPPCPAPELLGGPSVFLFPPK PKDTLMISRTPEVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSRDELTKNQVSLTCLVK GFYPSDIAVEWESNGQPENNYKTTPPVLDSDGSFFLYSKLTVDKSRWQQGNVFSCSVMHEAL HNHYTQKSLSLSPG (SEQ ID NO:9)

DIVMTQTPLSLPVTPGEPASISC*RSSKSLLHSNGITYLY*WYLQKPGQSPQLLIY*QMSNLVS*G VPDRFSGSGSGTDFTLKISRVEAEDVGVYYC*AQNLELPYT*FGGGTKVEIKRTVAAPSVFIFP PSDEQLKSGTASVVCLLNNFYPREAKVQWKVDNALQSGNSQESVTEQDSKDSTYSLSSTLTL SKADYEKHKVYACEVTHQGLSSPVTKSFNRGEC (SEQ ID NO:10)

The anti-CD20 antibody may be a humanized B-Ly1 antibody. In this context, some useful polypeptides are provided in Table 2:

Table 2. Polypeptide sequences.

| CONSTRUCT | POLYPEPTIDE SEQUENCE | SEQ ID NO |
|---|---|---|
| B-HH1 | QVQLVQSGAEVKKPGSSVKVSCKASGYTFSYSWM SWVRQAPGQGLEWMGRIFPGDGDTDYAQKFQGRV TITADKSTSTAYMELSSLRSEDTAVYYCARNVFDG YWLVYWGQGTLVTVSS | 13 |
| B-HH2 | QVQLVQSGAEVKKPGSSVKVSCKASGYAFSYSWM NWVRQAPGQGLEWMGRIFPGDGDTDYNGKFKGR VTITADKSTSTAYMELSSLRSEDTAVYYCARNVFD GYWLVYWGQGTLVTVSS | 14 |
| B-HH3 | QVQLVQSGAEVKKPGSSVKVSCKASGYAFSYSWM NWVRQAPGQGLEWMGRIFPGDGDTDYNGKFKGR VTITADKSTSTAYMELSSLRSEDTAVYLCARNVFDG YWLVYWGQGTLVTVSS | 15 |
| B-HH4 | QVQLVQSGAEVKKPGASVKVSCKVSGYAFSYSWM NWVRQAPGQGLEWMGRIFPGDGDTDYNGKFKGR VTITADKSTSTAYMELSSLRSEDTAVYYCARNVFD GYWLVYWGQGTLVTVSS | 16 |
| B-HH5 | QVQLVQSGAEVKKPGSSVKVSCKASGYAFSYSWM SWVRQAPGQGLEWMGRIFPGDGDTDYNGKFKGRV TITADKSTSTAYMELSSLRSEDTAVYYCARNVFDG YWLVYWGQGTLVTVSS | 17 |

(continued)

| CONSTRUCT | POLYPEPTIDE SEQUENCE | SEQ ID NO |
|---|---|---|
| B-HH6 | QVQLVQSGAEVKKPGSSVKVSCKASGYAFSYSWIN WVRQAPGQGLEWMGRIFPGDGDTDYNGKFKGRVT ITADKSTSTAYMELSSLRSEDTAVYYCARNVFDGY WLVYWGQGTLVTVSS | 7 |
| B-HH7 | QVQLVQSGAEVKKPGSSVKVSCKASGYAFSYSWIS WVRQAPGQGLEWMGRIFPGDGDTDYNGKFKGRVT ITADKSTSTAYMELSSLRSEDTAVYYCARNVFDGY WLVYWGQGTLVTVSS | 18 |
| B-HH8 | QVQLVQSGAEVKKPGASVKVSCKASGYTFTYSWM NWVRQAPGQGLEWMGRIFPGDGDTDYNGKFKGR VTITADKSTSTAYMELSSLRSEDTAVYYCARNVFD GYWLVYWGQGTLVTVSS | 19 |
| B-HH9 | QVQLVQSGAEVKKPGASVKVSCKASGYTFSYSWM NWVRQAPGQGLEWMGRIFPGDGDTDYNGKFKGR VTITADKSTSTAYMELSSLRSEDTAVYYCARNVFD GYWLVYWGQGTLVTVSS | 20 |
| B-HL1 | QVQLVQSGAEVKKPGASVKVSCKASGYTFTYSWM HWVRQAPGQGLEWMGRIFPGDGDTDYAQKFQGR VTMTRDTSTSTVYMELSSLRSEDTAVYYCARNVFD GYWLVYWGQGTLVTVSS | 21 |
| B-HL2 | EVQLVQSGAEVKKPGATVKISCKVSGYTFTYSWMH WVQQAPGKGLEWMGRIFPGDGDTDYAEKFQGRVT ITADTSTDTAYMELSSLRSEDTAVYYCATNVFDGY WLVYWGQGTLVTVSS | 22 |
| B-HL3 | EVQLVQSGAEVKKPGATVKISCKVSGYTFTYSWMN WVQQAPGKGLEWMGRIFPGDGDTDYNGKFKGRVT ITADTSTDTAYMELSSLRSEDTAVYYCATNVFDGY WLVYWGQGTLVTVSS | 23 |
| B-HL4 | QMQLVQSGAEVKKTGSSVKVSCKASGYTFTYSWM SWVRQAPGQGLEWMGRIFPGDGDTDYAQKFQGRV TITADKSTSTAYMELSSLRSEDTAVYYCARNVFDG YWLVYWGQGTLVTVSS | 24 |
| B-HL8 | EVQLVESGGGLVKPGGSLRLSCAASGFTFSYSWMN WVRQAPGKGLEWVGRIFPGDGDTDYNGKFKGRVT ITADKSTSTAYMELSSLRSEDTAVYYCARNVFDGY WLVYWGQGTLVTVSS | 25 |
| B-HL10 | EVQLVESGGGLVKPGGSLRLSCAASGFAFSYSWMN WVRQAPGKGLEWVGRIFPGDGDTDYNGKFKGRVT ITADKSTSTAYMELSSLRSEDTAVYYCARNVFDGY WLVYWGQGTLVTVSS | 26 |

(continued)

| CONSTRUCT | POLYPEPTIDE SEQUENCE | SEQ ID NO |
|---|---|---|
| B-HL11 | QVQLVESGGGLVKPGGSLRLSCAASGFTFSYSWMN WVRQAPGKGLEWVGRIFPGDGDTDYNGKFKGRVT ITADKSTSTAYMELSSLRSEDTAVYYCARNVFDGY WLVYWGQGTLVTVSS | 27 |
| B-HL12 | EVQLVESGAGLVKPGGSLRLSCAASGFTFSYSWMN WVRQAPGKGLEWMGRIFPGDGDTDYNGKFKGRVT ITADKSTSTAYMELSSLRSEDTAVYYCARNVFDGY WLVYWGQGTLVTVSS | 28 |
| B-HL13 | EVQLVESGGGVVKPGGSLRLSCAASGFTFSYSWMN WVRQAPGKGLEWMGRIFPGDGDTDYNGKFKGRVT ITADKSTSTAYMELSSLRSEDTAVYYCARNVFDGY WLVYWGQGTLVTVSS | 29 |
| B-HL14 | EVQLVESGGGLKKPGGSLRLSCAASGFTFSYSWMN WVRQAPGKGLEWMGRIFPGDGDTDYNGKFKGRVT ITADKSTSTAYMELSSLRSEDTAVYYCARNVFDGY WLVYWGQGTLVTVSS | 30 |
| B-HL15 | EVQLVESGGGLVKPGSSLRLSCAASGFTFSYSWMN WVRQAPGKGLEWMGRIFPGDGDTDYNGKFKGRVT ITADKSTSTAYMELSSLRSEDTAVYYCARNVFDGY WLVYWGQGTLVTVSS | 31 |
| B-HL16 | EVQLVESGGGLVKPGGSLRVSCAASGFTFSYSWMN WVRQAPGKGLEWMGRIFPGDGDTDYNGKFKGRVT ITADKSTSTAYMELSSLRSEDTAVYYCARNVFDGY WLVYWGQGTLVTVSS | 32 |
| B-HL17 | EVQLVESGGGLVKPGGSLRLSCAASGFTFSYSWMN WVRQAPGKGLEWMGRIFPGDGDTDYNGKFKGRVT ITADKSTSTAYMELSSLRSEDTAVYYCARNVFDGY WLVYWGQGTLVTVSS | 33 |
| VH Signal Sequence | MDWTWRILFLVAAATGAHS | 34 |
| B-KV1 | DIVMTQTPLSLPVTPGEPASISCRSSKSLLHSNGITYL YWYLQKPGQSPQLLIYQMSNLVSGVPDRFSGSGSG TDFTLKISRVEAEDVGVYYCAQNLELPYTFGGGTK VEIKRTV | 8 |
| VL Signal Sequence | MDMRVPAQLLGLLLLWFPGARC | 43 |

The anti-CD20 antibody may be an afucosylated glyco-engineered antibody. Such glycoengineered antibodies have an altered pattern of glycosylation in the Fc region, preferably having a reduced level of fucose residues. Preferably the amount of fucose is 60 % or less of the total amount of oligosaccharides at Asn297 (for example, the amount of fucose may be between 40 % and 60 %, the amount of fucose may be 50 % or less, or the amount of fucose may be 30 % or less). Furthermore the oligosaccharides of the Fc region are preferably bisected. The type II anti-CD20 antibody may comprise an Fc region comprising a biantennary oligosaccharide that is bisected by N-acetyl glucosamine (GlcNAc). These glycoengineered humanized anti-CD20 (e.g., B-Ly1) antibodies have an increased ADCC.

The oligosaccharide component can significantly affect properties relevant to the efficacy of a therapeutic glycoprotein, including physical stability, resistance to protease attack, interactions with the immune system, pharmacokinetics, and specific biological activity. Such properties may depend not only on the presence or absence, but also on the specific structures, of oligosaccharides. Some generalizations between oligosaccharide structure and glycoprotein function can be made. For example, certain oligosaccharide structures mediate rapid clearance of the glycoprotein from the bloodstream through interactions with specific carbohydrate binding proteins, while others can be bound by antibodies and trigger undesired immune reactions. (Jenkins, N., et al., Nature Biotechnol. 14 (1996) 975-81).

Mammalian cells are the preferred hosts for production of therapeutic glycoproteins, due to their capability to glycosylate proteins in the most compatible form for human application. (Cumming, D.A., et al., Glycobiology 1 (1991) 115-30; Jenkins, N., et al., Nature Biotechnol. 14 (1996) 975-81). Bacteria very rarely glycosylate proteins, and like other types of common hosts, such as yeasts, filamentous fungi, insect and plant cells, yield glycosylation patterns associated with rapid clearance from the blood stream, undesirable immune interactions, and in some specific cases, reduced biological activity. Among mammalian cells, Chinese hamster ovary (CHO) cells have been most commonly used during the last two decades. In addition to giving suitable glycosylation patterns, these cells allow consistent generation of genetically stable, highly productive clonal cell lines. They can be cultured to high densities in simple bioreactors using serum free media, and permit the development of safe and reproducible bioprocesses. Other commonly used animal cells include baby hamster kidney (BHK) cells, NSO- and SP2/0-mouse myeloma cells. More recently, production from transgenic animals has also been tested. (Jenkins, N., et al., Nature Biotechnol. 14 (1996) 975-981).

Antibodies may contain carbohydrate structures at conserved positions in the heavy chain constant regions, with each isotype possessing a distinct array of N-linked carbohydrate structures, which variably affect protein assembly, secretion or functional activity. (Wright, A., and Morrison, S.L., Trends Biotech. 15 (1997) 26-32). The structure of the attached N-linked carbohydrate varies considerably, depending on the degree of processing, and can include high-mannose, multiply-branched as well as biantennary complex oligosaccharides. (Wright, A., and Morrison, S.L., Trends Biotech. 15 (1997) 26-32). Typically, there is heterogeneous processing of the core oligosaccharide structures attached at a particular glycosylation site such that even monoclonal antibodies exist as multiple glycoforms. Likewise, it has been shown that major differences in antibody glycosylation occur between cell lines, and even minor differences are seen for a given cell line grown under different culture conditions. (Lifely, M.R., et al., Glycobiology 5(8) (1995) 813-22).

One way to obtain large increases in potency, while maintaining a simple production process and potentially avoiding significant, undesirable side effects, is to enhance the natural, cell-mediated effector functions of monoclonal antibodies by engineering their oligosaccharide component as described in Umana, P., et al., Nature Biotechnol. 17 (1999) 176-180 and US 6,602,684. IgG1 type antibodies, the most commonly used antibodies in cancer immunotherapy, are glycoproteins that have a conserved N-linked glycosylation site at Asn297 in each CH2 domain. The two complex biantennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC) (Lifely, M.R., et al., Glycobiology 5 (1995) 813-822; Jefferis, R., et al., Immunol. Rev. 163 (1998) 59-76; Wright, A., and Morrison, S.L., Trends Biotechnol. 15 (1997) 26-32).

It was previously shown that overexpression in Chinese hamster ovary (CHO) cells of $\beta$(1,4)-N-acetylglucosaminyltransferase I11 ("GnTII17y"), a glycosyltransferase catalyzing the formation of bisected oligosaccharides, significantly increases the in vitro ADCC activity of an antineuroblastoma chimeric monoclonal antibody (chCE7) produced by the engineered CHO cells. (See Umana, P., et al., Nature Biotechnol. 17 (1999) 176-180; and WO 99/154342). The antibody chCE7 belongs to a large class of unconjugated monoclonal antibodies which have high tumor affinity and specificity, but have too little potency to be clinically useful when produced in standard industrial cell lines lacking the GnTIII enzyme (Umana, P., et al., Nature Biotechnol. 17 (1999) 176-180). That study was the first to show that large increases of ADCC activity could be obtained by engineering the antibody producing cells to express GnTIII, which also led to an increase in the proportion of constant region (Fc)-associated, bisected oligosaccharides, including bisected, non-fucosylated oligosaccharides, above the levels found in naturally-occurring antibodies.

The anti-CD20 antibody may comprise a human Fc region (e.g., a human IgG1 Fc region). The Fc region may comprise an N-linked oligosaccharide that has been modified. The N-linked oligosaccharides of the Fc region may have reduced fucose residues as compared to an antibody with non-modified N-linked oligosaccharides. The bisected oligosaccharide may be a bisected complex oligosaccharide. The N-linked oligosaccharides may have been modified to have increased bisected, nonfucosylated oligosaccharides. The bisected, nonfucosylated oligosaccharides may be the hybrid type. The bisected, nonfucosylated oligosaccharides may be the complex type. For more detailed description, see, e.g., WO 2003/011878 (Jean-Mairet et al.); US Patent No. 6,602,684 (Umana et al.); US 2005/0123546 (Umana et al.); and U.S. Patent No. 8,883,980 (Umana et al.).

According to the invention as per the appended claims, the type II anti-CD20 antibody is obinutuzumab.

*Antibody Preparation*

A generic type II anti-CD20 antibody as described above may incorporate any of the features, singly or in combination, as described in Sections 1-2 below:

**1. Chimeric and Humanized Antibodies**

A generic type II anti-CD20 antibody described above may be a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

A chimeric antibody may be a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. Some FR residues in a humanized antibody may be substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall' Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

**2. Antibody Variants**

**a) Glycosylation variants**

A generic type II anti-CD20 antibody described above may be altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. Modifications of the oligosaccharide in an antibody of the invention may be made in order to create antibody variants with certain improved properties.

Antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

## b) Fc region variants

One or more amino acid modifications may be introduced into the Fc region of an antibody provided herein, thereby generating an Fc region variant. The Fc region variant may comprise a human Fc region sequence *(e.g.,* a human IgG1, IgG2, IgG3 or IgG4 Fc region) comprising an amino acid modification (e.g. a substitution) at one or more amino acid positions.

An antibody variant may possess some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express Fc(RIII only, whereas monocytes express Fc(RI, Fc(RII and Fc(RIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

The Fc variants described herein may further comprise one or more amino acid modifications for attenuating effector function (such as CDC and/or ADCC). The modification to attenuate effector function may be a modification that does not alter the glycosylation pattern of the Fc region. The modification to attenuate effector function may reduce or eliminate binding to human effector cells, binding to one or more Fc receptors, and/or binding to cells expressing an Fc receptor. For example, the Fc variants described herein may comprise the following modifications: L234A, L235A and P329G in the Fc region of human IgG1, that result in attenuated effector function. Substitutions L234A, L235A, and P329G (the L234A/L235A/P329G triple variant is referred to as LALAPG) have previously been shown to reduce binding to Fc receptors and complement (see e.g., US Publication No. 2012/0251531).

Fc variants having reduced effector function may refer to Fc variants that reduce effector function (e.g., CDC,

# EP 4 028 054 B1

ADCC, and/or binding to FcR, etc. activities) by at least 10%, 20%, 30%, 40%, 50%, 60%, 70%, 80%, 90%, 95%, 97%, 98%, 99% or more as compared to the effector function achieved by a wild-type Fc region (e.g., an Fc region not having a mutation to reduce effector function, although it may have other mutations). Fc variants having reduced effector function may refer to Fc variants that eliminate all detectable effector function as compared to a wild-type Fc region. Assays for measuring effector function are known in the art and described below.

*In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity). The primary cells for mediating ADCC, NK cells, express FcγRIII only, whereas monocytes express FcγRI, FcγRII and FcγRIII. FcR expression on hematopoietic cells is summarized in Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

An antibody variant may comprise an Fc region with one or more amino acid substitutions which improve ADCC, e.g., substitutions at positions 298, 333, and/or 334 of the Fc region (EU numbering of residues).

Alterations can be made in the Fc region that result in altered *(i.e.,* either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

## A. Recombinant Methods and Compositions

Antibodies may be produced using recombinant methods and compositions, e.g., as described in U.S. Patent No. 4,816,567. Isolated nucleic acids encoding an anti-CD20 antibody described herein are provided. Such nucleic acid may encode an amino acid sequence comprising the VL and/or an amino acid sequence comprising the VH of the antibody (e.g., the light and/or heavy chains of the antibody). One or more vectors (e.g., expression vectors) comprising such nucleic acid are provided. A host cell comprising such nucleic acid is provided. Such a host cell may comprise (e.g., may have been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. The host cell is preferably eukaryotic, e.g. a Chinese Hamster Ovary (CHO) cell or lymphoid cell (e.g., Y0, NS0, Sp20 cell). A method of making an anti-CD20 antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-CD20 antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli.*) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

### B. Assays

Anti-CD20 antibodies referred to herein may be identified, screened for, or characterized for their physical/-chemical properties and/or biological activities by various assays known in the art.

### 1. Binding assays and other assays

An antibody may be tested for its antigen binding activity, e.g., by known methods such as ELISA, Western blot, etc. CD20 binding may be determined using methods known in the art and exemplary methods are disclosed herein. Binding is measured using radioimmunoassay. An exemplary radioimmunoassay is provided below. CD20 antibody is iodinated, and competition reaction mixtures are prepared containing a fixed concentration of iodinated antibody and decreasing concentrations of serially diluted, unlabeled CD20 antibody. Cells expressing CD20 (e.g., BT474 cells stably transfected with human CD20) are added to the reaction mixture. Following an incubation, cells are washed to separate the free iodinated CD20 antibody from the CD20 antibody bound to the cells. Level of bound iodinated CD20 antibody is determined, e.g., by counting radioactivity associated with cells, and binding affinity determined using standard methods. Ability of CD20 antibody to bind to surface-expressed CD20 (e.g., on B cell subsets) can be assessed using flow cytometry. Peripheral white blood cells are obtained (e.g., from human, cynomolgus monkey, rat or mouse) and cells are blocked with serum. Labeled CD20 antibody is added in serial dilutions, and T cells are also stained to identify T cell subsets (using methods known in the art). Following incubation of the samples and washing, the cells are sorted using flow cytometer, and data analyzed using methods well known in the art. CD20 binding may be analyzed using surface plasmon resonance. An exemplary surface plasmon resonance method is exemplified in the Examples.

In another aspect, competition assays may be used to identify an antibody that competes with any of the anti-CD20 antibodies disclosed herein for binding to CD20. Such a competing antibody may bind to the same epitope (e.g., a linear or a conformational epitope) that is bound by any of the anti-CD20 antibodies disclosed herein. Detailed exemplary methods for mapping an epitope to which an antibody binds are provided in Morris (1996) "Epitope Mapping Protocols," in Methods in Molecular Biology vol. 66 (Humana Press, Totowa, NJ).

To illustrate a competition assay, immobilized CD20 is incubated in a solution comprising a first labeled antibody that binds to CD20 (e.g., rituximab, a GA101 antibody, etc.) and a second unlabeled antibody that is being tested for its ability to compete with the first antibody for binding to CD20. The second antibody may be present in a hybridoma supernatant. As a control, immobilized CD20 is incubated in a solution comprising the first labeled antibody but not the second unlabeled antibody. After incubation under conditions permissive for binding of the first antibody to CD20, excess unbound antibody is removed, and the amount of label associated with immobilized CD20 is measured. If the amount of label associated with immobilized CD20 is substantially reduced in the test sample relative to the control sample, then that indicates that the second antibody is competing with the first antibody for binding to CD20. See Harlow and Lane (1988) Antibodies: A Laboratory Manual ch.14 (Cold Spring Harbor Laboratory, Cold Spring Harbor, NY).

2. Activity assays

Anti-CD20 antibodies referred to herein *(e.g.,* a type II antibody) may be identified and/or characterized by one or more activity assays known in the art. For example, a complement-dependent cytotoxicity (CDC) and/or antibody-dependent cellular cytotoxicity (ADCC) may be used, as described herein.

It is understood that any of the above assays may be carried out using an immunoconjugate of the invention in place of or in addition to an anti-CD20 antibody.

It is understood that any of the above assays may be carried out using anti-CD20 antibody and an additional therapeutic agent.

*Treating lupus nephritis*

Provided herein are methods for treating lupus nephritis (LN) in an individual that has lupus, in accordance with the appended claims.

LN is known in the art as a manifestation of lupus (e.g., systemic lupus erythematosus, drug-induced lupus, neonatal lupus, or discoid lupus) in the kidney(s). The most common type of lupus that manifests in the kidneys is systemic lupus erythematosus (SLE). It is thought that 25-50% of SLE patients have abnormalities in the urine and/or renal function early in the course of their disease, with up to 60% of adults and 80% of children eventually developing LN (for more details, see Cameron, J.S. (1999) J. Am. Soc. Nephrol. 10:413-424). LN is thought to account for at least 50% of the morbidity and mortality associated with SLE.

In addition, renal manifestations have also been noted in other types of lupus, such as discoid (Roujeau, J.C. et al. (1984) Acta Derm. Venereol. 64:160-163) and drug-induced lupus (Smith, P.R. et al. (1999) Rheumatology (Oxford) 38:1017-1018). In some embodiments, the individual has SLE, discoid lupus, or drug-induced lupus.

Diagnosis of SLE may be according to current American College of Rheumatology (ACR) criteria. Active disease may be defined by one British Isles Lupus Activity Group's (BILAG) "A" criteria or two BILAG "B" criteria; SLE Disease Activity Index (SLEDAI); or systemic lupus erythematosus (SLE) responder index (SRI) as noted in the Examples below and descrived in Furie et al., Arthritis Rheum. 61(9):1143-51 (2009). Some signs, symptoms, or other indicators used to diagnose SLE adapted from: Tan et al. "The Revised Criteria for the Classification of SLE" Arth Rheum 25 (1982) may be malar rash such as rash over the cheeks, discoid rash, or red raised patches, photosensitivity such as reaction to sunlight, resulting in the development of or increase in skin rash, oral ulcers such as ulcers in the nose or mouth, usually painless, arthritis, such as non-erosive arthritis involving two or more peripheral joints (arthritis in which the bones around the joints do not become destroyed), serositis, pleuritis or pericarditis, renal disorder such as excessive protein in the urine (greater than 0.5 gm/day or 3+ on test sticks) and/or cellular casts (abnormal elements derived from the urine and/or white cells and/or kidney tubule cells), neurologic signs, symptoms, or other indicators, seizures (convulsions), and/or psychosis in the absence of drugs or metabolic disturbances that are known to cause such effects, and hematologic signs, symptoms, or other indicators such as hemolytic anemia or leukopenia (white blood count below 4,000 cells per cubic millimeter) or lymphopenia (less than 1,500 lymphocytes per cubic millimeter) or thrombocytopenia (less than 100,000 platelets per cubic millimeter). The leukopenia and lymphopenia must be detected on two or more occasions. The thrombocytopenia must be detected in the absence of drugs known to induce it. The invention is not limited to these signs, symptoms, or other indicators of lupus.

The presence of autoantibodies may be tested as an indication for lupus. Autoantibodies may include without limitation anti-dsDNA antibodies, anti-complement antibodies, and antinuclear antibodies (e.g., an ENA panel). ENA refers to Extractable Nuclear Antigens, i.e., a group of nuclear antigens including, e.g., RNP, Ro/SS-A, La/ SS-B, Sm, SCL-70, Jo-1, as described in McNeilage et al., J., Clin. Lab. Immunol. 15:1-17 (1984); Whittingham, Ann. Acad. Med. 17(2):195-200 (1988); Wallace and Hahn, DUBOIS' LUPUS ERYTHEMATOSUS, 7TH ED. LIPPINCOTT (2007); Tang et al., Medicine 89(1): 62-67 (2010). Antibodies to ENA have been correlated to lupus. McNeilage et al., 1984; Whittingham 1988; Asherson et al., Medicine 68(6): 366-374 (1989); and Tang et al., 2010. Reduced complement activity may also be associated with lupus, *e.g.,* as measured by C3 levels, C4 levels, and/or a CH50 assay.

As described above in reference to SLE, it is known in the art that LN often manifests progressively in patients with lupus (e.g., systemic lupus erythematosus, drug-induced lupus, neonatal lupus, or discoid lupus). That is to say, a patient may be diagnosed with lupus without a clinical or pathological manifestation of one or more LN symptoms. Nonetheless, the patient may still be considered to be at risk for developing LN due to the high frequency of lupus patients that eventually develop LN. Therefore, in some embodiments, the methods of the present disclosure may find use in delaying progression of LN, or preventing LN, in a patient with lupus. In some embodiments, the methods of the present disclosure may find use in postponing or preventing the onset of LN in a patient with lupus *(e.g.,* a form of lupus that lacks a manifestation in the kidney(s)).

LN pathology may be classified according to the International Society of Nephrology/Renal Pathology Society (ISN/RPS) 2003 classification system, as shown in the table below (see Markowitz GS, D'Agati VD (2007) Kidney Int 71:491-495 and Weening, JJ (2004) Kidney Int 65:521-530 for further descriptions and definitions of terms).

**Table 3.** ISN/RPS 2003 Classification of Lupus Nephritis.

| | |
|---|---|
| Class I | Minimal mesangial LN (Normal glomeruli by light microscopy, but mesangial immune deposits by immunofluorescence) |
| Class II | Mesangial proliferative LN (Purely mesangial hypercellularity of any degree or mesangial matrix expansion by light microscopy, with mesangial immune deposits. A few isolated subepithelial or subendothelial deposits may be visible by immunofluorescence or electron microscopy, but not by light microscopy) |
| Class III | Focal LN (Active or inactive focal, segmental or global endo- or extracapillary glomerulonephritis involving < 50% of all glomeruli, typically with focal subendothelial immune deposits, with or without mesangial alterations) <br> III (A): active lesions (focal proliferative LN) <br> III (A/C): active and chronic lesions (focal proliferative and sclerosing LN) <br> III (C): chronic inactive lesions with glomerular scars (focal sclerosing LN) |
| Class IV | Diffuse LN (Active or inactive diffuse, segmental or global endo- or extracapillary glomerulonephritis involving $\geq$ 50% of all glomeruli, typically with diffuse |
| | subendothelial immune deposits, with or without mesangial alterations. This class is divided into diffuse segmental (IV-S) LN when $\geq$ 50% of the involved glomeruli have segmental lesions, and diffuse global (IV-G) LN when $\geq$ 50% of the involved glomeruli have global lesions. Segmental is defined as a glomerular lesion that involves less than half of the glomerular tuft. This class includes cases with diffuse wire loop deposits but with little or no glomerular proliferation.) <br> IV-S (A): active lesions (diffuse segmental proliferative LN) <br> IV-G (A): active lesions (diffuse global proliferative LN) <br> IV-S (A/C): active and chronic lesions (diffuse segmental proliferative and sclerosing LN) <br> IV-G (A/C): active and chronic lesions (diffuse global proliferative and sclerosing LN) <br> IV-S (C): chronic inactive lesions with scars (diffuse segmental sclerosing LN) <br> IV-G (C): chronic inactive lesions with scars (diffuse global sclerosing LN) |
| Class V | Membranous LN (Global or segmental subepithelial immune deposits or their morphologic sequelae by light microscopy and by immunofluorescence or electron microscopy, with or without mesangial alterations.) |
| Class VI | Advanced sclerotic LN ($\geq$ 90% of glomeruli globally sclerosed without residual activity) |
| LN = lupus nephritis; A = active; C = chronic; G = global; S = segmental. <br> Note: Class V may occur in combination with Class III or IV, in which case both will be diagnosed. <br> Class V LN may show advanced sclerosis. | |

In some embodiments, the patient has class III or class IV LN. In some embodiments, the patient has class III LN. For example, in some embodiments, the patient has class III(A) or class III(A/C) LN. In some embodiments, the patient has class IV LN. For example, in some embodiments, the patient has class IV-S(A), IV-G(A), IV-S(A/C), or IV-G(A/C) LN. As shown in Table 3 above, class V LN may also occur concomitantly with class III or class IV LN. In some embodiments, the methods of the present disclosure are used to treat a patient with class III or class IV LN and concomitant class V LN.

As discussed above, a high frequency of patients with lupus *(e.g.,* SLE) eventually develop LN. In some embodiments, the patient is at risk for developing LN. In some embodiments, the patient is at risk for developing class III or class IV LN. In some embodiments, the patient is at risk for developing class III or class IV LN with concomitant class V LN.

In some embodiments, the patient does not have class III(C) LN *(e.g.,* as described in Table 3 above). In some embodiments, the patient does not have class IV(C) LN, such as class IV-S(C) or IV-G(C) LN *(e.g.,* as described in Table 3 above).

In some embodiments, the patient has a urine to protein creatinine ratio (UPCR) of >1 prior to treatment, *e.g.*, on a 24-hour urine collection. In some embodiments, the patient has received at least one dose of pulse methylprednisolone (*e.g.*, 500-1000mg IV) prior to treatment. In some embodiments, the patient has received an ACE inhibitor or angiotensin-receptor blocker (ARB) at a stable dose of ≥10 days prior to treatment.

In some embodiments, the patient does not have severe renal impairment or need for dialysis or renal transplantation, *e.g.*, prior to treatment as described herein. In some embodiments, the patient does not have sclerosis in >50% of glomeruli on renal biopsy, *e.g.*, prior to treatment as described herein. In some embodiments, the patient does not have active central nervous system SLE, *e.g.*, prior to treatment as described herein. In some embodiments, the patient does not have history of progressive multifocal leukoencephalopathy (PML), *e.g.*, prior to treatment as described herein. In some embodiments, the patient does not have positive hepatitis C serology, hemoglobin < 7g/dL (unless caused by autoimmune hemolytic anemia resulting from SLE), platelet count <20,000/uL, or positive serum human chorionic gonadotropin, *e.g.*, prior to treatment as described herein. In some embodiments, the patient does not have known HIV infection, *e.g.*, prior to treatment as described herein. In some embodiments, the patient has not been treated with one or more of: cyclophosphamide, calcineurin inhibitor, JAK inhibitor, BTK inhibitor, TYK2 inhibitor, or IV antibiotic prior to treatment as described herein (*e.g.*, 3 months prior to treatment as described herein).

Several lab tests known in the art may be used to diagnose and/or monitor the presence, progression, and/or response to treatment in lupus nephritis. In some embodiments, serum creatinine may be measured. In some embodiments, the normal range for serum creatinine may be from about 0.6 to about 1.3 mg/dL, with some variation seen by age, between men and women, and from lab to lab. In some embodiments, the presence of urinary sediment and/or casts may be measured, *e.g.*, by microscopic examination of urine. For example, the number of red blood cells in a urine sample may be assayed by microscopic examination. In some embodiments, a normal value for urinary sediment may be about 4 red blood cells (RBC) or less per high power field (HPF). Urinary casts may include without limitation red blood cell casts, white blood cell casts, renal tubular epithelial cell casts, waxy casts, hyaline casts, granular casts, and fatty casts. In some embodiments, a urinary protein to creatinine ratio (UPCR) may be measured. The presence of protein in the urine (proteinuria) may also be assayed by tests including without limitation a urine albumin to creatinine ratio (UACR) and dipstick urinalysis. Other tests and/or measures that may be useful for examining renal function include without limitation a renal panel, creatinine clearance, sodium, potassium, chloride, bicarbonate, phosphorus, calcium, albumin, blood urea nitrogen (BUN), creatinine, glucose, estimated glomerular filtration rate (eGFR), BUN/creatinine ratio, and anion gap, and may include a measurement of the above parameters in the blood and/or urine, where appropriate. For more detailed description, see, *e.g.*, the American College of Rheumatology Guidelines for Screening, Case Definition, Treatment and Management of Lupus Nephritis (Hahn, B. et al. (2012) Arthritis Care Res. 64:797-808).

In some embodiments, the individual has been, or concurrently is being, treated with a renin-angiotensin system blockade, *e.g.*, an angiotensin-converting enzyme (ACE) inhibitor and/or angiotensin-receptor blocker (ARB). In some embodiments, the individual has a urine protein to creatinine ratio (UPCR) of greater than or equal to 5g (*e.g.*, from a 24-hour urine collection) despite treatment with a renin-angiotensin system blockade, *e.g.*, an angiotensin-converting enzyme (ACE) inhibitor and/or angiotensin-receptor blocker (ARB), for greater than or equal to 3 months prior to treatment with the type II anti-CD20 antibody, or a UPCR of greater than or equal to 4g (*e.g.*, from a 24-hour urine collection) despite treatment with a renin-angiotensin system blockade, *e.g.*, an angiotensin-converting enzyme (ACE) inhibitor and/or angiotensin-receptor blocker (ARB), for greater than or equal to 6 months prior to treatment with the type II anti-CD20 antibody. In some embodiments, the individual has an estimated glomerular filtration rate (eGFR) ≥40 mL/min/1.73 m$^2$ or endogenous creatinine clearance ≥40 mL/min (*e.g.*, based on a 24-hour urine collection). In some embodiments, eGFR is calculated using the CKD-EPI equation.

In some embodiments, the individual does not have secondary MN. In some embodiments, the individual is not hypertensive or does not have uncontrolled blood pressure, *e.g.*, for at least 3 months prior to treatment with the type II anti-CD20 antibody. In some embodiments, the individual has systolic blood pressure ≤ 140 mmHg and diastolic blood pressure ≤ 90 mmHg, *e.g.*, prior to treatment with the type II anti-CD20 antibody. In some embodiments, the individual has not been treated with a calcineurin inhibitor drug (CNI) (*e.g.*, cyclosporin A or an mTOR inhibitor) or alkylating agent for at least 6 months prior to treatment with the type II anti-CD20 antibody. In some embodiments, the individual has not been treated with rituximab for at least 6 months prior to treatment with the type II anti-CD20 antibody. In some embodiments, the individual has not been treated with renal replacement therapy (e.g., renal transplantation, chronic dialysis) prior to treatment with the type II anti-CD20 antibody. In some embodiments, the individual does not have type 1 or type 2 diabetes mellitus. In some embodiments, the individual does not have an active infection, history of serious recurrent or chronic infection, HIV infection, or TB infection. In some embodiments, the individual does not have a history of cancer or PML. In some embodiments, the individual is not positive for HBV, HCV, or serum human chorionic gonadotropin. In some embodiments, the individual does not have any or all of the following (*e.g.*, prior to treatment with the type II anti-CD20 antibody): AST or ALT >2.5 x the upper limit of normal (ULN), Amylase or lipase > 2 x ULN, Neutrophils <1.5x10$^3$/μL, CD19+ B cells <5/μL, Hemoglobin < 9g/dL, or Platelet count < 75,000/μL.

*Regimen*

The methods of the present disclosure include administering to the individual a first antibody exposure to a type II anti-CD20 antibody of the present disclosure, a second antibody exposure to the type II anti-CD20 antibody of the present disclosure, and a third antibody exposure to the type II anti-CD20 antibody of the present disclosure. The dosing regimen is as per the appended claims, being tailored to the use of obinutuzumab. As mentioned earlier, albeit not claimed, other anti-CD20 antibodies (in particular type II anti-CD20 antibodies) can be considered for analogous use, in which case the regimen may have to be chosen according to the following criteria.

The dosing regimens described herein use a consistent system for tracking time between doses whereby the first dose is administered to the patient on Day 1 or week 0. As described herein, an antibody exposure of the present disclosure may include one or two doses. In cases where the antibody exposures contain one dose, references to a second antibody exposure not provided until a period of time has elapsed after a first antibody exposure (as described herein) refer to the amount of time elapsed between the dose of the first antibody exposure (*e.g.*, Day 1 or week 0) and the dose of the second antibody exposure. If the first antibody exposure includes two doses, the first dose of the first antibody exposure is provided on Day 1 or week 0. In cases where the antibody exposures contain two doses, references to a second antibody exposure not provided until a period of time has elapsed after a first antibody exposure (as described herein) refer to the amount of time elapsed between the first of the two doses of the first antibody exposure (*e.g.*, Day 1 or week 0) and the first dose of the two doses of the second antibody exposure. For example, if a method of the present disclosure includes a first antibody exposure with two doses and a second antibody exposure with two doses, and the second antibody exposure is not provided until about 22 weeks after the first antibody exposure, then the interval between the first dose of the first antibody exposure and the first dose of the second antibody exposure is about 22 weeks.

The first antibody exposure can include two doses. The first dose of the first antibody exposure contains 1000mg of the type II anti-CD20 antibody. The second dose of the first antibody exposure contains 1000mg of the type II anti-CD20 antibody. The second dose of the first antibody exposure is not provided until about 2 weeks after the first dose of the first antibody exposure.

The second antibody exposure can include two doses. The first dose of the second antibody exposure contains 1000mg of the type II anti-CD20 antibody. The second dose of the second antibody exposure contains 1000mg of the type II anti-CD20 antibody. The second dose of the second antibody exposure may not be provided until about 2 weeks after the first dose of the second antibody exposure.

The third antibody exposure of the present disclosure can include one or two doses of the type II anti-CD20 antibody.

The therapeutic methods described may further include (and in the case of the claimed invention do include) the administration of an effective amount of an immunosuppressive agent in conjunction with the type II anti-CD20 antibody. Several classes of immunosuppressive agents are known in the art, including without limitation cytostatics (*e.g.,* cytotoxic agents such as antibiotics, alkylating agents (*e.g.,* cyclophosphamide, also known as cytophosphane), inosine monophosphate dehydrogenase inhibitors, antimetabolites such as protein synthesis inhibitors, folic acid analogs, purine analogs, pyrimidine analogs, and the like), immunosuppressive antibodies, glucocorticoids, drugs targeting immunophilins (*e.g.*, tacrolimus, sirolimus, rapamycin and analogs thereof, ciclosporin, and the like), mTOR active site inhibitors, mycophenolic acid and derivatives or salts thereof, TNF binding proteins, interferons, opiods, and other small molecules (*e.g.*, fingolimod). Preferably, the immunosuppressive agent includes mycophenolic acid, a derivative of mycophenolic acid, or a salt of mycophenolic acid. More preferably, the immunosuppressive agent includes mycophenolate mofetil. In some cases, the immunosuppressive agent includes CellCept® (Roche) or Myfortic® (Novartis). Effective amounts of the immunosuppressive agents are known in the art and readily ascertainable by standard assays. For example, mycophenolate mofetil may be administered at 2.0-2.5g/day. In some embodiments, mycophenolate mofetil may be administered starting at 1000mg/day in divided doses (2 times/day) and titrating up to 2.0-2.5g/day in divided doses (2 times/day) by week 4.

The immunosuppressive agent may be administered before, during, or after administration of the type II anti-CD20 antibody, *e.g.*, as a treatment for lupus. In some cases, an immunosuppressive agent may be administered throughout the period of treatment with a type II anti-CD20 antibody of the present disclosure. In some cases, mycophenolate mofetil may be administered as described above throughout the period of treatment with the type II anti-CD20 antibody.

The therapeutic methods may further include (and in the case of the claimed invention do include) the administration of an effective amount of a glucocorticoid or corticosteroid in conjunction with the type II anti-CD20 antibody. A variety of naturally occurring and synthetic glucocorticoids/corticosteroids are known in the art, including without limitation beclometasone, triamcinolone, dexamethasone, betamethasone, prednisone, methylprednisolone, prednisolone, cortisone, and cortisol. For example, the glucocorticoids/corticosteroid may include methylprednisolone or may include prednisone. Effective amounts of the glucocorticoids/corticosteroids are known in the art and readily ascertainable by standard assays. For example, methylprednisolone may be administered at 750-1000mg doses once daily by IV. As another example, prednisone may be administered orally at 0.5mg/kg and optionally tapered to 7.5mg/day.

A glucocorticoid may be administered before, during, or after administration of the type II anti-CD20 antibody. In some cases, a glucocorticoid may be administered prior to administration of a type II anti-CD20 antibody, *e.g.*, 30-60 minutes before the type II anti-CD20 antibody. For example, 80mg methylprednisolone may be administered by IV 30-60 minutes before administration of obinutuzumab. In some cases, prednisone (*e.g.*, orally administered) and/or methyl prednisolone (*e.g.*, IV administered) may be administered with treatment, followed by a maintenance treatment (*e.g.*, mycophenolate mofetil or cyclophosphamide).

The therapeutic methods described (including some embodiments of the invention) may further include administering an effective amount of an antihistamine in conjunction with the type II anti-CD20 antibody. Antihistamines known in the art and currently in clinical use include histamine Hi-receptor and histamine H$_2$-receptor antagonists or inverse agonists. In some embodiments, the antihistamine includes diphenhydramine. Effective amounts of the antihistamines are known in the art and readily ascertainable by standard assays. For example, diphenhydramine may be administered in 50mg oral doses.

An antihistamine may be administered before, during, or after administration of the type II anti-CD20 antibody, *e.g.*, as a prophylactic treatment. For example, an antihistamine may be administered prior to administration of obinutuzumab, *e.g.*, 30-60 minutes before obinutuzumab. For instance, 50mg diphenhydramine may be administered orally 30-60 minutes before administration of obinutuzumab.

The therapeutic methods described (including but not limited to some embodiments of the invention) may further include administering an effective amount of a non-steroidal anti-inflammatory drug or NSAID (*e.g.,* in conjunction with the type II anti-CD20 antibody, i.e. obinutuzumab). NSAIDs known in the art include acetic acid derivatives, propionic acid derivatives, salicylates, enolic acid derivatives, anthranilic acid derivatives, selective COX-2 inhibitors, sulfonanilides, and the like. In some embodiments, the NSAID includes acetaminophen. Effective amounts of the NSAIDs of the present disclosure are known in the art and readily ascertainable by standard assays. For example, acetaminophen may be administered in 650-1000mg oral doses.

The NSAID may be administered before, during, or after administration of the type II anti-CD20 antibody, *e.g.*, as a prophylactic treatment. For example, an NSAID may be administered prior to administration of obinutuzumab, *e.g.*, 30-60 minutes before obinutuzumab. In some cases, 650-1000mg acetaminophen may be administered orally 30-60 minutes before administration of obinutuzumab.

The therapeutic methods described (including some embodiments of the invention) may further include administering an effective amount of an anti-malarial agent (*e.g.,* in conjunction with a type II anti-CD20 antibody as described herein). Examples of anti-malarial agents that may be used include without limitation hydroxychloroquine, chloroquine, and quinacrine. An anti-malarial agent may be administered before, during, or after administration of a type II anti-CD20 antibody of the present disclosure, *e.g.*, as a treatment for one or more symptoms of lupus.

The therapeutic methods described (including some embodiments of the invention) may further include administering an effective amount of an integrin antagonist (*e.g.*, in conjunction with a type II anti-CD20 antibody as described herein). Examples of integrin antagonists that may be used include without limitation an LFA-1 antibody, such as efalizumab (RAPTΓVA®) commercially available from Genentech, or an alpha 4 integrin antibody such as natalizumab (ANTEGREN®) available from Biogen, or diazacyclic phenylalanine derivatives, phenylalanine derivatives, phenylpropionic acid derivatives, enamine derivatives, propanoic acid derivatives, alkanoic acid derivatives, substituted phenyl derivatives, aromatic amine derivatives, ADAM disintegrin domain polypeptides, antibodies to alphavbeta3 integrin, aza-bridged bicyclic amino acid derivatives, etc. An integrin antagonist may be administered before, during, or after administration of a type II anti-CD20 antibody of the present disclosure, *e.g.*, as a treatment for one or more symptoms of lupus.

The therapeutic methods described (including some embodiments of the invention) may further include administering an effective amount of a cytokine antagonist (*e.g.*, in conjunction with a type II anti-CD20 antibody as described herein). Examples of cytokine antagonists that may be used include without limitation an antagonist (*e.g.*, an antagonist antibody) against IL-1, IL-1α, IL-2, IL-3, IL-4, IL-5, IL-6, IL-7, IL-8, IL-9, IL-11, IL-12, IL-15; a tumor necrosis factor such as TNF-α or TNF-β; and other polypeptide factors including LIF and kit ligand (KL). A cytokine antagonist may be administered before, during, or after administration of a type II anti-CD20 antibody of the present disclosure, *e.g.*, as a treatment for one or more symptoms of lupus.

The therapeutic methods described (including some embodiments of the invention) may further include administering an effective amount of a hormone (*e.g.*, in conjunction with a type II anti-CD20 antibody as described herein). A hormone (*e.g.,* for hormone replacement therapy) may be administered before, during, or after administration of a type II anti-CD20 antibody of the present disclosure, *e.g.*, for a medical treatment in a women with lupus.

The therapeutic methods described (including some embodiments of the invention) may further include administering a standard of care treatment (*e.g.*, in conjunction with type II anti-CD20 antibody). The standard of care treatment may be administered before, during, or after administration of the type II anti-CD20 antibody (in the case of the invention, obinutuzumab), *e.g.*, for treating or preventing one or more symptoms of lupus. In certain cases, a standard of care treatment may be administered after a second antibody exposure of the present disclosure. In certain cases, a standard of care treatment may be administered after a third antibody exposure of the present disclosure. For example, obinutuzumab may be administered as described herein to a patient as an induction therapy, then the patient may be treated according to standard of care as a maintenance therapy. Standard of care treatments for lupus are well known in the art and include without limitation an angiotensin-converting enzyme (ACE) inhibitor, an angiotensin-receptor blocker, cyclophosphamide, mycophenolate mofetil (*e.g.,* at a dose as described herein, such as 2.0-2.5 g/day), azathioprine, and a prednisone, such as a prednisone taper.

The therapeutic methods described (including some embodiments of the invention) may further include administering an anti-hypertensive agent in conjunction with the type II anti-CD20 antibody (obinutuzumab in the case of the invention). In some cases, an anti-hypertensive agent may be administered before, during, or after administration of the type II anti-CD20 antibody, *e.g.*, for treating or preventing hypertension. In some cases, anti-hypertensive agents includes without limitation ACE inhibitors and angiotensin-receptor blockers.

The therapeutic methods described (including that of the invention) may result in a complete renal response (CRR) in an individual, which may comprise all of the following: a normalization of serum creatinine, an inactive urinary sediment, and a urinary protein to creatinine ratio of < 0.5. A normalization of serum creatinine is typically characterized by serum creatinine less than or equal to the upper limit of normal (ULN) range of central laboratory values, and/or serum creatinine ≤ 15% above baseline and less than or equal to the ULN range of central laboratory values if baseline (*e.g.*, Day 1) serum creatinine is within the normal range of the central laboratory values. An inactive urinary sediment is typically characterized by < 10 RBCs/high-power field (HPF) and/or the absence of red cell casts. For more detailed discussion of CRR and partial renal response (PRR) in LN, see, *e.g.,* Chen, Y.E. et al. (2008) Clin. J. Am. Soc. Nephrol. 3:46-53.

The therapeutic methods described (including that of the invention) may result in a complete renal response (CRR) or a partial renal response (PRR) in an individual. A PRR could comprise one or more of the following: a normalization of serum creatinine, an inactive urinary sediment, and a urinary protein to creatinine ratio of < 0.5. A PRR could also be characterized by one or more of the following: mitigation of one or more symptoms including without limitation a reduction in serum creatinine, reduced urinary sediment, a reduction in proteinuria, and any other improvement in renal function. A CRR or PRR may comprise a reduction in one or more biomarkers of lupus activity, including without limitation anti-dsDNA antibodies, antinuclear antibodies/ENA, anti-complement antibodies, reduced levels of complement C3 and/or C4, and reduced complement activity (*e.g.*, as measured by CH50 assay).

The therapeutic methods described (including that of the invention) may typically result in the depletion of circulating peripheral B cells in an individual, such as circulating peripheral CD19+ B cells. Such cells can be naive B cells, memory B cells, plasmablasts or plasma cells. In some cases, after administration of a type II anti-CD20 antibody, circulating peripheral B cells are present in peripheral blood at about about 7 cells/μL or fewer, about 6 cells/μL or fewer, about 5 cells/μL or fewer, about 4 cells/μL or fewer, about 3 cells/μL or fewer, about 2 cells/μL or fewer, about 1 cell/μL or fewer, or about 0.5 cells/ μL or fewer. In some cases, the level of circulating peripheral B cells are measured using highly sensitive flow cytometry (HSFC) described herein. In some cases, B cells are depleted to a level that is below the detectable limit using HSFC. In some cases, the HSFC has a lower limit of quantitation (LLOQ) for B cells of about 1.0 cells/μL or fewer, about 0.8 cells/μL or fewer, about 0.6 cells/μL or fewer, about 0.5 cells/μL or fewer, or 0.441 cells/μL or fewer. In some cases, circulating peripheral B cells in the individual are depleted by at least about 50%, at least about 60%, at least about 70%, at least about 80%, at least about 90%, or about 100%. Ideally, depletion of circulating peripheral B cells is sustained for at least 52 weeks after the first dose of the first antibody exposure. In some cases, depletion of circulating peripheral B cells is sustained for at least 51 weeks, at least 50 weeks, at least 49 weeks, at least 48 weeks, at least 47 weeks, at least 46 weeks, at least 45 weeks, at least 44 weeks, at least 43 weeks, at least 42 weeks, at least 41 weeks, at least 40 weeks, at least 39 weeks, at least 38 weeks, at least 37 weeks, at least 36 weeks, at least 35 weeks, at least 34 weeks, at least 33 weeks, at least 32 weeks, at least 31 weeks, at least 30 weeks, at least 29 weeks, at least 28 weeks, at least 27 weeks, at least 26 weeks, at least 25 weeks, or at least 24 weeks after the first dose of the first antibody exposure. Depletion of circulating peripheral B cells refers to a measurement of circulating peripheral B cells taken after a first antibody exposure (*e.g.*, including 1 or 2 doses of an anti-CD20 antibody as described herein), after a second antibody exposure (*e.g.*, including 1 or 2 doses of an anti-CD20 antibody as described herein), after a third antibody exposure (*e.g.*, including 1 or 2 doses of an anti-CD20 antibody as described herein), 3 months after treatment (*e.g.*, after receiving a first, and/or a second, and/or a third antibody exposure as described herein), 6 months after treatment (*e.g.*, after receiving a first, and/or a second, and/or a third antibody exposure as described herein), 9 months after treatment (*e.g.*, after receiving a first, and/or a second, and/or a third antibody exposure as described herein), or 12 months after treatment (*e.g.*, after receiving a first, and/or a second, and/or a third antibody exposure as described herein), *e.g.,* as compared to a corresponding measurement in the same individual before treatment, or as compared to a corresponding measurement in a control individual (*e.g.*, an individual that has not received treatment).

Methods for assaying depletion of circulating peripheral B cells in an individual are known in the art, *e.g.,* flow cytometry using one or more antibodies that recognize a B cell marker. In some cases, highly sensitive flow cytometry (HSFC) may be used to assay depletion of circulating peripheral B cells (see, *e.g.,* Vital, E.M. et al. (2011) Arthritis Rheum.

63:3038-3047 and Example 1). In some cases, the B cells are CD19+ B cells. In some cases, the B cells are naive B cells (*e.g.,* CD19+ CD27- B cells), memory B cells (*e.g.,* CD19+ CD27+ B cells), or plasmablasts (*e.g.,* CD19+ CD27+ CD38++ B cells). In some cases, the B cells are CD19+CD3-CD14- cells and/or CD19+CD33-CD56- cells. In some cases, the B cells are CD19+CD3-CD14-CD33-CD56- cells. In some cases, the B cells comprise CD19+CD20+ B cells, CD19+CD20- B cells, and CD19+CD22+ B cells. Preferably, the B cells are circulating peripheral B cells, *e.g.,* from a peripheral blood sample.

The level of circulating peripheral B cells present in a peripheral blood sample can be measured (*e.g.,* by HSFC) as follows. Lymphocytes are identified in a sample by flow cytometry (*e.g.,* by plotting CD45 vs. side scatter and gating CD45+ cells). Doublets can be excluded from analysis prior to this step (*e.g.,* by gating single cells and excluding forward scatter and/or side scatter doublets). CD19+ B cells are then identified by excluding T cells, NK cells, and monocytes. For example, CD19+CD3-CD14- cells can be identified from a parent CD45+ lymphocyte gate (*e.g.,* by plotting CD19 vs. CD3/CD14 and gating CD19+CD3-CD14- cells), and CD19+CD33-CD56- B cells can be identified from a parent CD19+CD3-CD14- cells (*e.g.,* by plotting CD19 vs. CD33/CD56 and gating CD19+CD33-CD56- cells). B cell counts can then be determined, *e.g.,* by dividing the number of CD19+ B cells detected (*e.g.,* CD19+CD3-CD14-CD33-CD56- cells) by the sample volume. A number of beads or other QC control can be also quantified, and B cell counts can then be determined, *e.g.,* by calculating (CD19+ events x bead count)/(bead count x sample volume).

After administration of a type II anti-CD20 antibody according to any of the methods described herein, circulating peripheral B cells should typically be present in peripheral blood at about 7 cells/$\mu$L or fewer, about 6 cells/$\mu$L or fewer, about 5 cells/$\mu$L or fewer, about 4 cells/$\mu$L or fewer, about 3 cells/$\mu$L or fewer, about 2 cells/$\mu$L or fewer, about 1 cell/$\mu$L or fewer, or about 0.5 cells/$\mu$L or fewer, *e.g.,* 5 cells/$\mu$L or fewer. In some cases, B cells will be depleted to a level that is below the detectable limit using HSFC. In some cases, the HSFC has a lower limit of quantitation (LLOQ) for B cells of about 1.0 cells/$\mu$L or fewer, about 0.8 cells/$\mu$L or fewer, about 0.6 cells/$\mu$L or fewer, about 0.5 cells/$\mu$L or fewer, or 0.441 cells/$\mu$L or fewer.

The specification is considered to be sufficient to enable one skilled in the art to practice the invention. Various modifications of the invention in addition to those shown and described herein will become apparent to those skilled in the art from the foregoing description and fall within the scope of the appended claims.

EXAMPLES

The invention will be more fully understood by reference to the following examples, which however do not limit the scope of the appended claims.

### *Example 1: Obinutuzumab plus mycophenolate and corticosteroids for the treatment of proliferative lupus nephritis*

B-cells are central to the pathogenesis of lupus nephritis yet randomized controlled trials of type I anti-CD20 antibodies failed to demonstrate superiority over standard of care alone. Obinutuzumab is a glycoengineered type II anti-CD20 monoclonal antibody that induces greater B-cell depletion than type I anti-CD20 antibodies. A comparison was made between Obinutuzumab versus placebo treatment in patients with proliferative lupus nephritis treated with mycophenolate and corticosteroids.

The results of a phase 2, multicenter, randomized, double-blind trial (NOBILITY) comparing obinutuzumab with placebo among patients with proliferative lupus nephritis treated with mycophenolate and corticosteroids are presented below.

Materials and Methods

126 patients were enrolled at 43 sites in North America, South America, Europe, and Israel. After a 4-week screening period, patients were randomly assigned to one of two groups in a 1:1 ratio by an interactive Web-response system to receive intravenous obinutuzumab 1000 mg or placebo infusions on study days 1, 15, 168, and 182. In order to reduce the risk of infusion-related reactions, patients randomized to obinutuzumab or placebo received blinded methylprednisolone 80 mg IV or placebo, respectively, prior to study drug administration. All patients received mycophenolate (mycophenolate mofetil, target dose 2-2.5 grams per day or equivalent dose of mycophenolic acid) and a standardized corticosteroid taper (starting prednisone 0.5 mg/kg per day, maximum 60 mg per day, with taper to 7.5 mg per day by week 12). Patients were followed in a blinded fashion until week 104 and patients with persistent B-cell depletion were followed for safety and B-cell assessments. *See* also the protocol published in WO2016/183104.

Trial visits were scheduled on weeks 4, 12, 24, 36, 52, 76, and 104 to assess safety, urinary protein excretion (as measured by a UPCR from a 24-hour urine collection and/or a random UPCR, preferably from a first morning void), serum creatinine, levels of autoantibodies and serum complement components, and clinical disease activity. Peripheral blood B-cells were quantified at baseline (week 0) and weeks 2, 4, 12, 24, 52, and 104 (with data taken from some patients at week 76 for exploratory findings) using a high-sensitivity flow cytometry (HSFC) technique. An optional repeat renal biopsy was offered to all patients at week 52 and performed in accordance with local clinical practice.

**Patients**

Patients were eligible if they were between the ages of 18 and 75 years, had systemic lupus erythematosus (SLE) as established by the American College of Rheumatology criteria, renal biopsy evidence of International Society of Nephrology/Renal Pathology Society 2003 Class III or IV within six months of randomization (concomitant Class V was permitted), a urine protein to creatinine ratio (UPCR) >1 on a 24-hour urine collection, and estimated glomerular filtration rate (eGFR) $\geq$30 mL/min/1.73 m$^2$ without a rapidly progressive decline in kidney function. All of the patients provided written informed consent.

**End Points**

The primary end point was the proportion of patients who achieved complete renal response, defined as UPCR <0.5, serum creatinine less than or equal to the upper limit of normal and no greater than 15% above the baseline value, and fewer than 10 red blood cells per high powered field (HPF) without red blood cell casts on urinary sediment examination, at week 52. Key secondary endpoints were the achievement of partial renal response, defined as $\geq$50% reduction in UPCR from baseline to <1 (<3 if baseline UPCR $\geq$3), serum creatinine not increased from baseline >15%, and urinary red blood cells <10 or not increased from baseline >50%; overall renal response, defined as achievement of complete or partial response; modified complete renal response, defined as complete renal response excluding the urinary sediment criterion; second modified complete renal response, which permitted the serum creatinine to be less than or equal to the upper limit of normal or not increased from baseline >15%; change from baseline in biomarkers of lupus nephritis disease activity including dsDNA antibody levels, complement component 3 (C3), complement component 4 (C4); and safety. Patients who received rescue with pulse-dose methylprednisolone ($\geq$ 500 mg), cyclophosphamide, rituximab, or other new immunosuppressive therapies after baseline or who withdrew from the study prematurely were imputed as non-responders for all response end points.

**High Sensitivity Flow Cytometry (HSFC)**

A Minimal Residual B cell (MRB 1.1) panel including CD19, CD20, and CD22 markers was assayed by HSFC in order to provide an absolute count of peripheral B cells. CD19 appears early in B cell ontogeny and remains expressed on all B lineage cells but is down-modulated on plasma cells. CD20 is expressed on all normal B cells with the exception of very early progenitors and terminally differentiated plasma cells. CD22 is found primarily on mature B cells.

The HSFC assay used two tubes each for quality control (QC) and test sample: a control, fluorescence-minus-one (FMO) tube for gating, and an experimental tube, both of which were analyzed by a FACSCanto™ II flow cytometer (Becton Dickinson) equipped with 405 nm, 488 nm, and 633 nm lasers and BD FACSDiva™ flow cytometry analysis software (Becton Dickinson). Briefly, whole blood was collected from patients. 300μL of QC or whole blood was pipetted into the bottom of each staining tube using reverse pipetting. A 50μL mAb cocktail was mixed and added to each tube. The FMO tube used the following: 5μL anti-CD3:FITC, 5μL anti-CD14:FITC, 5μL anti-CD33:PerCP-Cy5.5, 5μL anti-CD56: PerCP-Cy5.5, 15μL anti-CD45:APC-H7, and 15μL PBS. The experimental tube used the following: 5μL anti-CD19:BV421, 5μL anti-CD3:FITC, 5μL anti-CD14:FITC, 5μL anti-CD22:PE, 5μL anti-CD33:PerCP-Cy5.5, 5μL anti-CD56: PerCP-Cy5.5, 5μL anti-CD20:APC, and 15μL anti-CD45:APC-H7. Tubes were mixed well (vortexed), and incubated at room temperature (18-26°C) in the dark for 15 minutes. Next, 1.5mL of BD FACSLysing solution was added to each tube. Tubes were mixed well (vortexed) and allowed to sit for 30 minutes in the dark at room temperature. Tubes were vortexed again, then analyzed.

Specimens were acquired on the FACSCanto™ II flow cytometer (Becton Dickinson) using a saved FACSDiva™ MRB Panel-1.1 acquisition template. The threshold was set at 1,000 on parameter 780/60 (633) (CD45 APC-H7). Before acquisition, it was verified with the first sample that the SSC and FSC voltages and threshold were appropriately set. A minimum of 20,000 events in the lymph gate were set as the stopping gate.

The following gating strategy was used. First, a dot plot of time (x-axis) vs. CD3/CD14 FITC-A (y-axis) was used for acquisition quality monitoring (plot 1). Events collected during system disruptions were negatively selected from analysis with a "time" gate. A bivariate dot plot of SSC-A (x-axis) vs. SSC-H (Y-axis) was used to gate single cells and exclude side scatter doublets (plot 2). A bivariate dot plot of FSC-A (x-axis) vs. FSC-H (Y-axis) of the previous gate was used to gate single cells and exclude forward scatter doublets (plot 3). A bi-exponential, bivariate dot plot of CD45 APC-H7-A (x-axis) vs. SSC-A (y-axis) of the previous gate was used to gate CD45+ lymphocytes (plot 4). A bivariate dot plot of FSC-A (x-axis) vs. SSC-A (Y-axis) was used to verify placement of the lymphocytes gate from plot 4 (plot 5). A bivariate dot plot of CD19 BV421-A (x-axis) vs. CD3/CD14 FITC-A (Y-axis) of the lymphocytes gate from plot 4 was used to exclude CD3+ T cells and CD14+ monocytes and gate CD3$^-$CD14$^-$CD19$^+$ cells (plot 6). A bivariate dot plot of CD19 BV421-A (x-axis) vs. CD33/CD56 PerCP-C5.5-A (Y-axis) of the CD3$^-$CD14$^-$ CD19$^+$ gate was used to exclude CD33+ monocytes and T or NK cells expressing CD56 and report on CD19+ B cells (CD33$^-$CD56$^-$CD19$^+$) (plot 7). A bi-exponential, bivariate dot plot of CD33/CD56 PerCP-Cy5.5-A (x-axis) vs. CD22 PE-A (Y-axis) of the time gate from plot 1was used to gate bead events for calculating absolute counts (plot 8). Absolute counts were determined as follows. CD19 B cells: cells/$\mu$L = (CD19+ events x bead count)/(bead count x 300$\mu$L blood volume used for staining). The assay was validated to a lower limit of quantitation (LLOQ) of 0.441 cells/$\mu$L.

## Statistical Analysis

Estimates indicated that the enrollment of 60 patients in each treatment group would provide a power of 83% to detect a 20% difference in achievement of complete renal response (CRR) in the obinutuzumab group (50% response rate) and the control group (30% response rate), using the Cochrane-Mantel-Haenzel test, at a two-sided alpha of 0.2. Assumptions were based on the response rates observed in recent randomized clinical trials involving patients with proliferative lupus nephritis. To control for type I error in the primary analysis, hypothesis testing on study end points was conducted sequentially starting with the primary end point.

The safety analysis population consisted of all patients who had received at least one dose of obinutuzumab or placebo. Descriptive statistics were used to evaluate safety.

Results

126 patients were randomized. One patient was randomized but discontinued the study due to pregnancy prior to the first blinded infusion; the remaining 125 patients received at least one dose of the assigned intervention and are included in the modified intention to treat population. 115 (92%) completed 52 weeks of treatment. Four patients (6%) in the obinutuzumab group and seven patients (11%) in the control group required rescue immunosuppression prior to week 52.

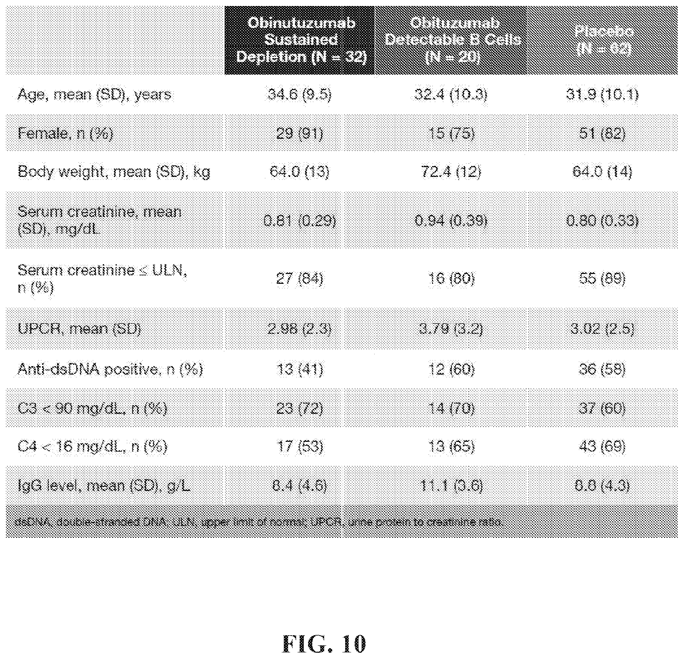

Most (85%) patients were female and the mean age was 33 years (Table 4). 73% identified as Hispanic or Latino and 43% were white. A total of 78% had class IV lupus nephritis; the remainder had class III lupus nephritis. Concomitant class V lupus nephritis was present in 29%. The mean (+/- SD) UPCR at baseline was 3.12$\pm$2.56, the mean serum creatinine at baseline was 0.84$\pm$0.77, and the mean eGFR at baseline was 102.0$\pm$31.7. The patients' disease characteristics at baseline were similar in both treatment groups. A further breakdown of the obinutuzumab group divided into patients later observed to have sustained depletion of B cells after obinutuzumab vs. patients later observed with detectable B cells after obinutuzumab treatment is shown in **FIG. 10.**

**Table 4.** Baseline characteristics and demographics of patients

| | Obinutuzumab (n=63) | Placebo (n=62) |
|---|---|---|
| Age - years | 33.1$\pm$9.8 | 31.9$\pm$10.1 |
| Male sex - no. (%) | 8 (13) | 11 (18) |
| Prior history of lupus nephritis - no. (%) | 32 (51) | 32 (52) |
| Class IV lupus nephritis - no. (%) | 49 (78) | 44 (71) |
| Concomitant class V lupus nephritis - no. (%) | 20 (32) | 17 (27) |
| Serum creatinine - mg/dL | 0.87$\pm$0.34 | 0.80$\pm$0.33 |
| eGFR - mL/min/1.73 m$^2$ | 102.0$\pm$30.1 | 102.1$\pm$32.9 |
| UPCR | 3.3$\pm$2.7 | 2.9$\pm$2.5 |
| Urine RBC - RBC/hpf | 10.8$\pm$16.3 | 9.1$\pm$20.0 |
| anti-dsDNA Ab $\geq$75 IU/mL - no. (%) | 31 (49) | 36 (58) |
| C3 <90 mg/dL - no. (%) | 43 (68) | 37 (60) |
| C4 <16 mg/dL - no. (%) | 41 (65) | 46 (62) |
| eGFR = estimated glomerular filtration rate; RBC = red blood cells; UPCR = urine protein to creatinine ratio | | |

**Clinical Outcomes**

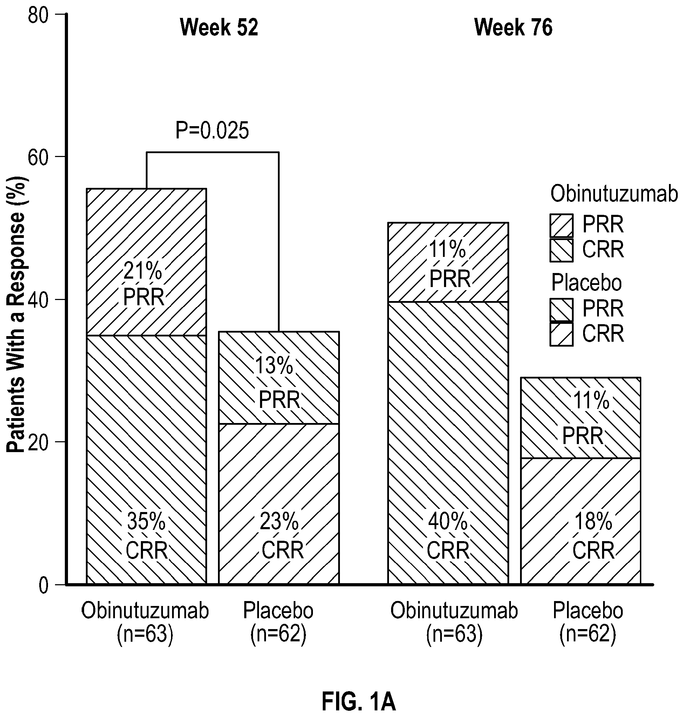

Complete renal response at week 52 (the primary end point) was achieved by 22 patients (35%) in the obinutuzumab group and 14 patients (23%) in the control group (risk difference, 12 percentage points; 80% CI, 2 to 22; P = 0.115) (**FIG. 1A**). Overall response at week 52 was achieved by 35 patients (56%) in the obinutuzumab group and 22 patients (36%) in the control group (risk difference, 20 percentage points; 80% CI, 9 to 31; P = 0.025). Modified complete renal response at week 52 was achieved by 25 patients (40%) in the obinutuzumab group and 16 patients (26%) in the control group (risk difference, 14 percentage points; 80% CI, 3 to 25; P = 0.09). Primary and secondary efficacy end points are presented in Table 5A.

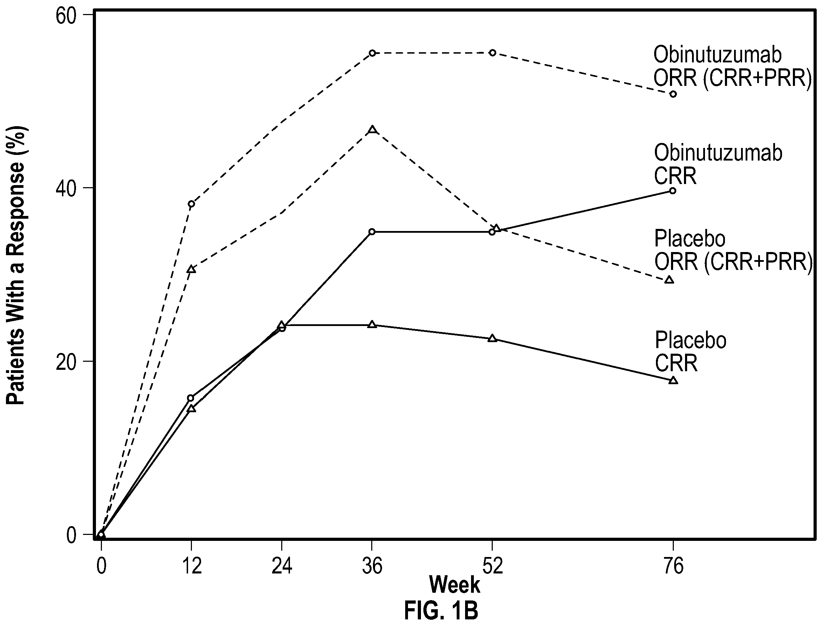

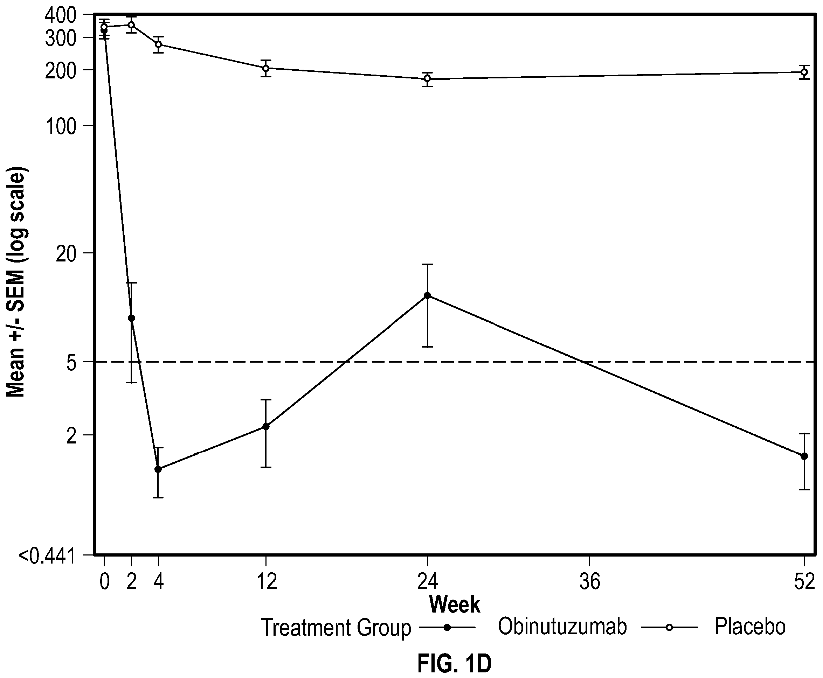

In an exploratory analysis at week 76, complete renal response was achieved by 25 patients (40%) in the obinutuzumab group and 11 patients (18%) in the control group (risk difference, 22 percentage points; 80% CI, 12 to 32). Pre-specified alternative complete renal response definitions increased rates of response in both groups while maintaining the treatment benefit of obinutuzumab at week 76. Renal responses over time are presented in **FIG. 1B.** Results of the week 76 exploratory analysis are presented in Tables 5A & 5B. Additional efficacy data are presented in **FIGS. 1C & 1D.**

Six patients (10%) in the obinutuzumab group and twelve patients (19%) in the placebo group received rescue therapy through week 76. Of these, two patients in the obinutuzumab group and six patients in the placebo group received cyclophosphamide rescue.

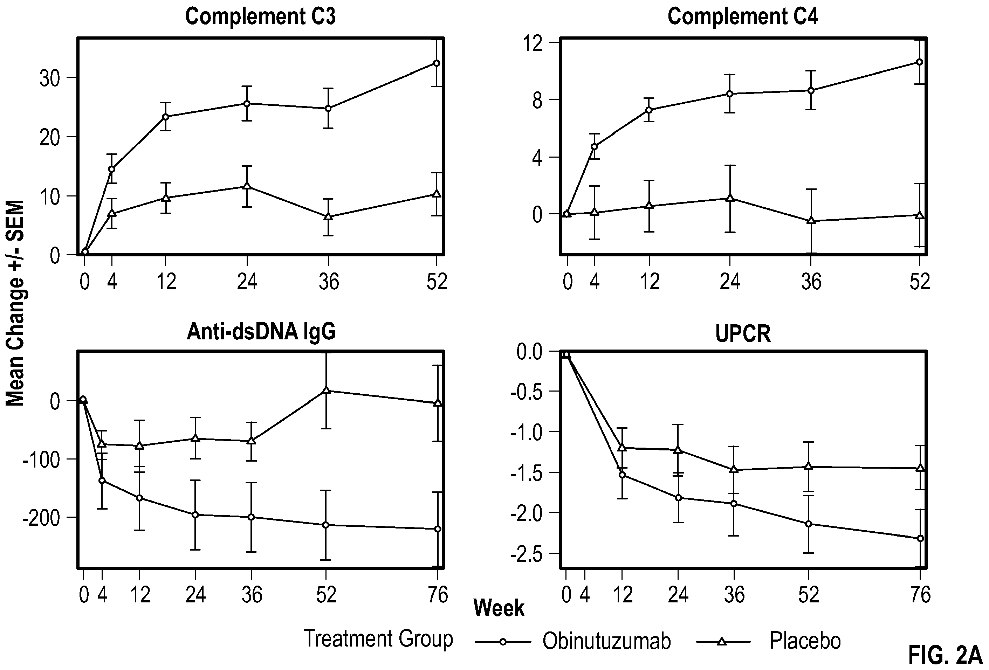

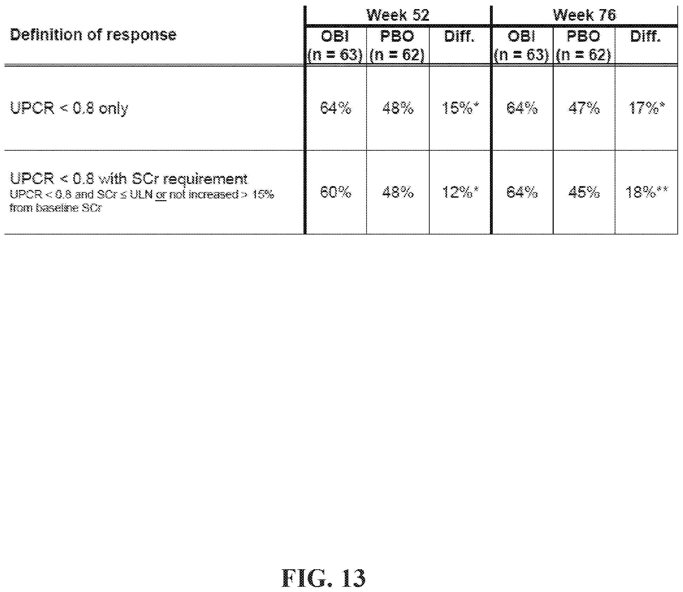

Obinutuzumab was associated with significant improvements in C3, C4, anti-dsDNA antibodies, and UPCR compared with placebo (Table 5A). At weeks 52 and 76, the adjusted mean differences in UPCR reduction from baseline between treatment groups were 0.57 (80% CI, 0.2 to 1.0) and 0.72 (80% CI, 0.4 to 1.1), respectively. Change from baseline in each of these measures is presented in **FIG. 2A.** Obinutuzumab was associated with increased achievement of CRR (40% vs. 18%, P = 0.007) and ORR (51% vs. 29%, P = 0.015) at week 76. Alternative response definitions demonstrated increased rates of response in both treatment groups (Table 5A and **FIG. 13**).

**Table 5A:** Primary and secondary end points at weeks 52 and 76.

| | Obinutuzumab (n=63) | Placebo (n=62) | Difference (80% CI) | P value* |
|---|---|---|---|---|
| **Week 52** | | | | |
| CRR | 22 (35) | 14 (23) | 12 (2, 22) | 0.115 |
| CRR excluding urinary sediment | 25 (40) | 16 (26) | 14 (3, 25) | 0.090 |
| Modified CRR | 29 (46) | 24 (39) | 7 (-4, 19) | 0.373 |
| PRR** | 35 (56) | 21 (34) | 22 (11, 33) | 0.015 |
| ORR (CRR or PRR) | 35 (56) | 22 (36) | 20 (9, 31) | 0.025 |
| ≥50% reduction in UPCR from baseline | 45 (71) | 35 (57) | 15 (4, 26) | - |
| anti-dsDNA < 30 IU/mL | 31 (49) | 16 (26) | 24 (13, 36) | 0.007 |
| C3 > 90 mg/dL | 50 (79) | 30 (48) | 31 (21, 41) | 0.0004 |
| C4 > 10 mg/dL | 61 (97) | 46 (74) | 23 (15, 30) | 0.0004 |
| **Week 76** | | | | |
| CRR | 25 (40) | 11 (18) | 22 (12, 32) | - |
| CRR excluding urinary sediment | 30 (48) | 14 (23) | 25 (15, 36) | - |
| Modified CRR | 36 (57) | 23 (37) | 20 (9, 31) | - |
| PRR** | 32 (51) | 18 (29) | 22 (11, 33) | - |

(continued)

| Week 76 | | | | |
|---|---|---|---|---|
| ORR (CRR or PRR) | 32 (51) | 18 (29) | 22 (11, 33) | - |

| |
|---|
| * *P* values are presented for prespecified analyses controlled for type I error<br>** Includes all patients who met PRR criteria, regardless of CRR achievement<br>CRR = complete renal response, which required urine protein to creatinine ratio (UPCR) <0.5, serum creatinine less than the upper limit of normal and not increased from baseline by >15%, and urine red blood cell (RBC) <10/hpf without RBC casts.<br>Modified CRR = modified complete renal response, which required UPCR < 0.5 with serum creatinine less than the upper limit of normal.<br>ORR = overall renal response.<br>PRR = partial renal response, which required UPCR ≥50% reduction from baseline to <1 (to <3 if baseline ≥3), serum creatinine not increased from baseline by >15%, and urine RBC <10/hpf or not increased from baseline by >50%.<br>SCr = serum creatinine; ULN = upper limit of normal at the central laboratory |

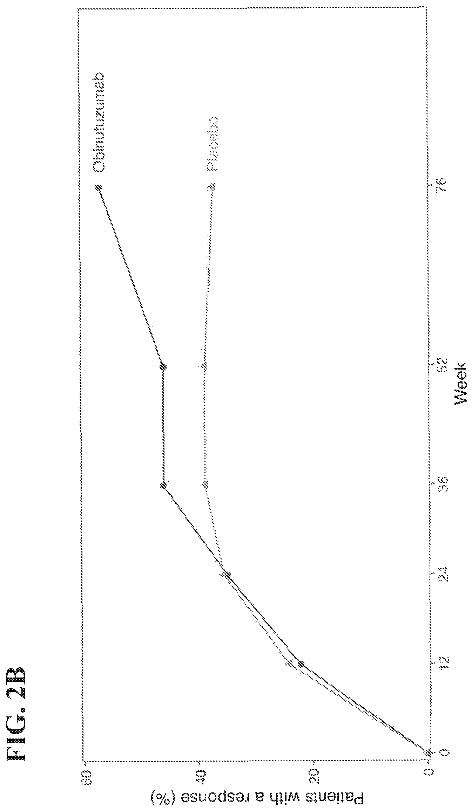

Recent studies have suggested that a 25% threshold for increases in serum creatinine may be appropriate for patients with normal serum creatinine. Therefore, new modified complete renal response (mCRR) and modified partial renal response (mPRR) criteria were applied to the data. Under the new criteria, mCRR required all of: UPCR <0.5; serum creatinine less than or equal to upper limit of normal; and serum creatinine not increased more than 25% from baseline; and mPRR required all of: UPCR ≥50% reduction to <1 (to <3 if baseline ≥3); and serum creatinine not increased more than 25% from baseline. Using these criteria, obinutuzumab was associated with increased mCRR at weeks 52 (43% vs. 29%, a difference of 14%, p<0.2) and 76 (54% vs. 31%, a difference of 23%, p<0.01), and obinutuzumab was also associated with increased mPRR at weeks 52 (68% vs. 45%, a difference of 23%, p<0.05) and 76 (68% vs. 50%, a difference of 18%, p<0.05), as compared to placebo. CRR over time (CRR definition: UPCR <0.5 and serum creatinine ≤ upper limit of normal) for both cohorts are shown in **FIG. 2B**.

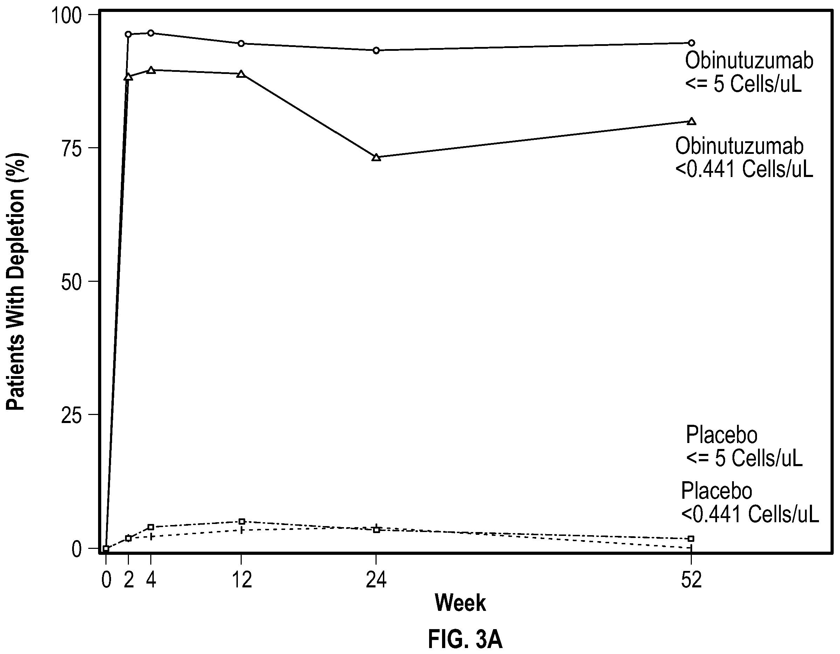

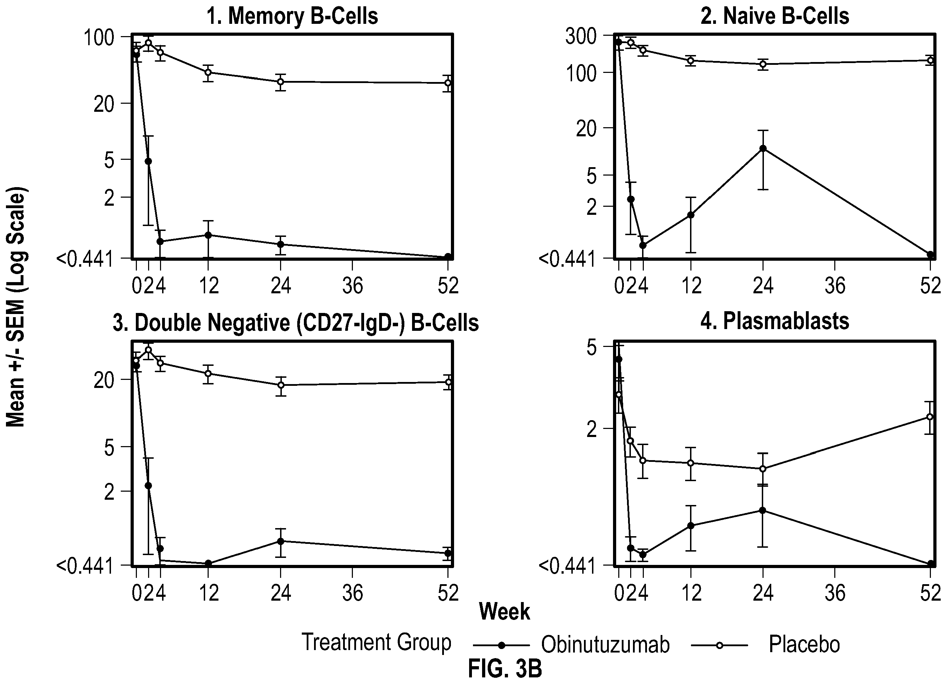

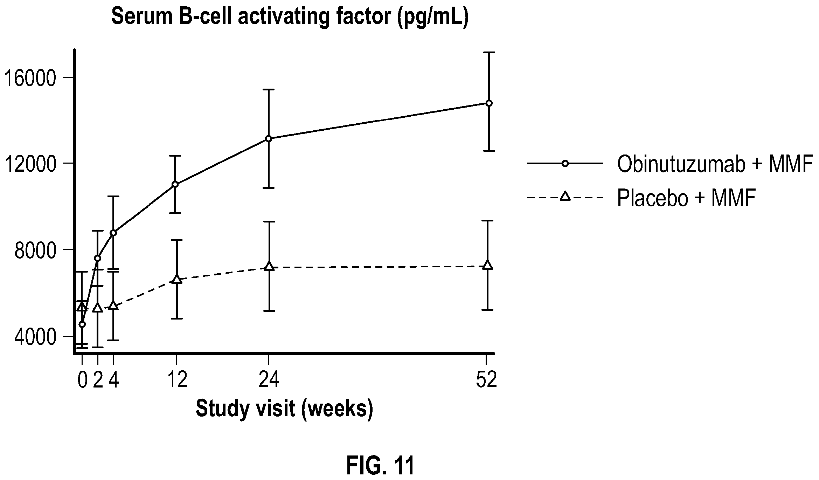

At week 4, 98% and 89% of patients in the obinutuzumab group had depletion of peripheral CD19+ B-cells below the lower limits of quantitation using conventional flow cytometry (<5 cells/μL) and high-sensitivity flow cytometry (<0.441 cells/μL), respectively. The proportions of patients with depletion by high-sensitivity flow cytometry at weeks 24 and 52 were 73% and 80%, respectively. At week 4, 98% and 89% of patients in the obinutuzumab group had depletion of peripheral CD19+ B-cells below the lower limits of quantitation using conventional flow cytometry (<5 cells/μL) and high-sensitivity flow cytometry (<0.441 cells/μL), respectively. The proportions of patients with depletion by high-sensitivity flow cytometry at weeks 24 and 52 were 73% and 80%, respectively (**FIG. 3A**). Memory and naive B-cells and plasmablasts were also rapidly depleted, with evidence of naive B-cell and plasmablast repopulation at week 24 prior to the third infusion (**FIG. 3B**). Mean serum B-cell activating factor (BAFF) levels increased from 4,585pg/mL at baseline to 14,601pg/mL at week 52 in the obinutuzumab group (**FIG. 11**), compared with an increase of 36% from a baseline value of 5,341 to 7,278 pg/mL at week 52 in the placebo group. In the obinutuzumab group, BAFF levels started to increase within 2 weeks.

Thirty-two patients (51%) in the obinutuzumab group remained depleted below the limit of detection using high-sensitivity flow cytometry at both weeks 24 and 52. These patients had numerically greater rates of CRR (50%) and ORR (66%) at week 76 as compared with those who had detectable B-cells at either time point, whose rates of CRR and ORR were 35% and 45%, respectively (**FIG. 4** and Table 5B). Among patients treated with obinutuzumab, achievement of sustained B-cell depletion was associated with greater renal response benefits at week 76 (Table 5B), although patients who achieved sustained B-cell depletion also had lower baseline proteinuria and serum creatinine. Sustained B-cell depletion was achieved in 32/52 (62%) of patients with complete data.

**Table 5B:** Renal responses at week 76 by depletion status.

| | Obinutuzumab sustained depletion (N=32) | Obinutuzumab detectable B-cells (N=20) | Placebo group (N=62) |
|---|---|---|---|
| CRR | 50%** | 35%* | 18% |
| Modified CRR | 72%** | 50% | 37% |

(continued)

| | Obinutuzumab sustained depletion (N=32) | Obinutuzumab detectable B-cells (N=20) | Placebo group (N=62) |
|---|---|---|---|
| ORR | 66%*** | 45%* | 29% |

Eleven patients in the obinutuzumab group with insufficient data to determine depletion status are excluded.
* P < 0.2 vs. placebo group
** P < 0.05 vs. placebo group
*** P < 0.001 vs. placebo group
CRR = complete renal response, which required UPCR <0.5 with serum creatinine (SCr) $\leq$ the upper limit of normal and not increased >15% from baseline with <10 RBCs/HPF and no RBC casts.
Modified CRR = UPCR < 0.5 with serum creatinine $\leq$ the upper limit of normal.
ORR = overall renal response, which required either CRR or partial renal response: $\geq$50% reduction in UPCR from baseline to <1 (<3 if baseline UPCR $\geq$3) with serum creatinine not increased >15% from baseline and $\leq$50% increase in urinary RBCs (or <10 RBCs/HPF).

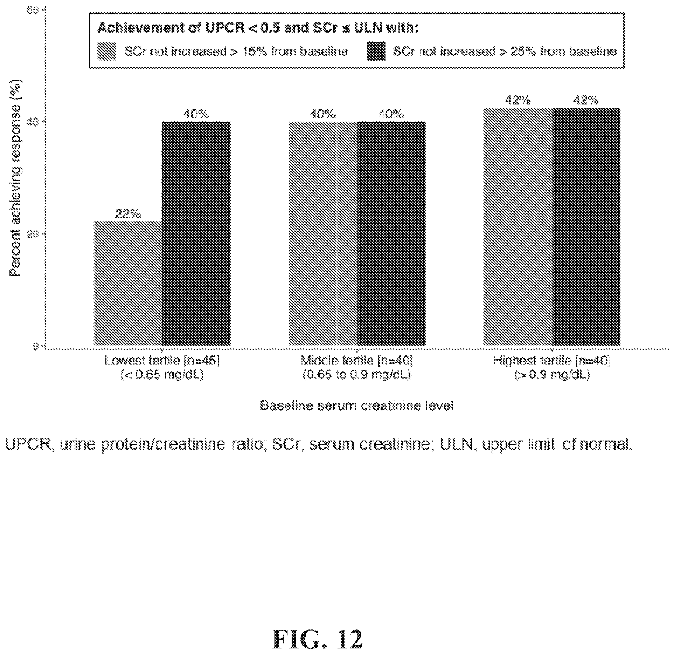

Response rates were low among patients with baseline SCr < 0.65mg/dL (n=45) due to the requirement that SCr not increase >15% from baseline (**FIG. 12**). Increasing this threshold to 25% increased the response rate to a level similar to other groups.

B-cell depletion achieved in this phase II clinical study (NOBILITY) examining obinutuzumab was compared against that achieved in a prior clinical study (LUNAR) on rituximab, a type I anti-CD20 antibody. Both antibodies were administered at 1000mg at weeks 0, 2, 24, and 26. As shown in Tables 5C and 5D, obinutuzumab treatment achieved superior B-cell depletion, whether measured to $\leq$5 cells/$\mu$L using conventional methodology (Table 5C) or depletion to 0 cells/$\mu$L as measured by HSFC as described herein (Table 5D).

**Table 5C:** B-cell depletion to $\leq$5 cells/$\mu$L

| | Obinutuzumab | Rituximab |
|---|---|---|
| Week 2 | 96% | 52% |
| Week 4 | 96% | 74% |
| Week 12 | 94% | 87% |
| Week 24 | 93% | 52% |
| Week 52 | 94% | 48% |

**Table 5D:** B-cell depletion to $\leq$0 cells/$\mu$L

| | Obinutuzumab | Rituximab |
|---|---|---|
| Week 2 | 71% | 12% |
| Week 4 | 71% | 17% |
| Week 12 | 74% | 25% |
| Week 24 | 66% | 5% |
| Week 52 | 81% | 13% |

**Adverse Events**

A summary of safety data is presented in Table 6. Median follow up duration at the data cutoff date was 78 weeks (range, 5 to 104 weeks). One patient randomized to placebo inadvertently received two obinutuzumab infusions during the first cycle and was included in the obinutuzumab group for safety analyses. The incidence of serious adverse events was 23% in the obinutuzumab group and 30% in the placebo group, and the incidence of serious infections was 6% in the obinutuzumab group and 18% in the placebo group, respectively. One patient in the obinutuzumab group and three in the placebo group discontinued blinded obinutuzumab infusions because of adverse events. Infusion-related reactions occurred in ten patients in the obinutuzumab group (16%) and in six patients in the placebo group (10%); all were non-serious and resolved with supportive care. The most frequent adverse events with obinutuzumab were bronchitis and infusion-related reactions.

As of the cutoff date, there were five deaths, one in the obinutuzumab group and four in the placebo group. One fatal case of progressive multifocal leukoencephalopathy occurred in the placebo group in a patient who had received cyclophosphamide rescue approximately six months prior to the diagnosis.

**Table 6:** Adverse events at week 76.

|  | Obinutuzumab (n=64) | Placebo (n=61) |
|---|---|---|
| Any adverse event - no. (%) | 56 (88) | 55 (90) |
| Total number of events | 355 | 314 |
| Deaths - no. (%) | 1 (2) | 4 (7) |
| Serious adverse events - no. (%) | 15 (23) | 18 (30) |
| Serious infection adverse events - no. (%) | 4 (6) | 11 (18) |
| Infection adverse event - no. (%) | 45 (70) | 39 (64) |
| Adverse event leading to discontinuation from blinded obinutuzumab - no. (%) | 1 (2) | 3 (5) |
| Infusion related reaction - no. (%) | 10 (16) | 6 (10) |
| Serious infusion related reaction - no. (%) | 0 | 0 |
| Progressive multifocal leukoencephalopathy - no. (%) | 0 | 1 (2) |
| One patient randomized to placebo inadvertently received two infusions of obinutuzumab during the first cycle. This patient is included in the obinutuzumab group for safety analyses. | | |

As noted above, CRR was greater with obinutuzumab than placebo at weeks 52 and 76. Results from week 104 are shown in Table 7 below.

**Table 7:** Week 104 results.

| Week 104 endpoint | OBI (n=63) | PBO (n=62) | Difference (80% CI) | *P* value |
|---|---|---|---|---|
| CRR | 41% | 23% | 19% (8, 29) | 0.026 |
| ORR | 54% | 29% | 25% (14, 36) | 0.005 |
| mCRR | 56% | 33% | 22% (11, 33) | 0.015 |
| C3 >90 mg/dL | 76% | 47% | 29% (19, 40) | 0.008 |
| C4 >10 mg/dL | 95% | 74% | 21% (13, 29) | 0.001 |
| Mean change in eGFR from baseline (mL/min/1.73 m$^2$) | +6.5 | -3.2 | 9.7 (1.7, 18) | 0.018 |
| Mean change in UPCR from baseline | -2.3 | -1.4 | -0.96 (-1.4, -0.57) | 0.002 |
| CRR=UPCR <0.5, serum creatinine (SCr) $\leq$upper limit of normal (ULN) and $\leq$115% of baseline, <10 red blood cells per high power field (RBCs/HPF), and no RBC casts. ORR = CRR or partial renal response=UPCR <1 (<3 if baseline UPCR $\geq$3) and $\leq$50% of baseline, SCr $\leq$115% of baseline, and $\leq$50% increase in urinary RBCs (or <10 RBCs/HPF). mCRR = UPCR <0.5, SCr $\leq$ ULN. | | | | |

Like the results at weeks 52 and 76, CRR was greater with obinutuzumab than placebo at week 104 (41% vs 23%, $P$ = 0.026). At Week 104, patients treated with obinutuzumab had greater improvement in eGFR (+6.5 vs. -3.2 mL/min/1.73 m$^2$, $P$ = 0.018), UPCR, anti-dsDNA, C3, and C4. Serious adverse events (OBI 25% vs. PBO 30%), serious infections (8% vs. 18%) and deaths (1 vs. 4) were not increased with OBI.

In summary, this study demonstrated a sustained benefit of obinutuzumab through week 104, approximately 18 months after the final obinutuzumab infusion.

**Conclusions and Discussion**

Non-clinical data indicated the possibility of treating Lupus with the anti-CD20 antibodies rituximab, ocrelizumab, and obinutuzumab. However, clinical trials for rituximab and ocrelizumab did not achieve their primary or key secondary endpoints for treatment. Unlike rituximab and ocrelizumab, the data presented herein demonstrate that the clinical trial for obinutuzumab met primary and key secondary efficacy endpoints. At one year, patients treated with obinutuzumab had increased complete and partial renal response as compared to patients receiving a placebo treatment when added to mycophenolate mofetil and corticosteroids for the treatment of proliferative lupus nephritis. Further, obinutuzumab was not associated with increased rates of serious adverse events or serious infections.

Obinutuzumab treatment resulted in rapid and complete depletion of peripheral CD19+ B-cells, memory and naive B-cell subsets, and plasmablasts in the majority of patients with a lower incidence of safety events, as compared to ocrelizumab treatment. At week 4, 89% of patients in the obinutuzumab group had depletion of peripheral CD19+ B-cells below the lower limit of quantitation (<0.441 cells/$\mu$L) using high-sensitivity flow cytometry (HSFC). Additionally, improved clinical efficacy was observed as compared to the placebo group.

Of the 63 patients in the obinutuzumab/mycophenolate mofetil group, 36 had confirmed sustained depletion $\leq 5$ cells/uL from day 28 through week 52, 6 had at least one measurement >5 cells/uL (no sustained depletion) and 21 were not evaluable for this analysis (one or more missing data points). Therefore, the majority of the patients in this group had sustained B-cell depletion to 52 weeks.

In the trial results, obinutuzumab was superior to placebo for achievement of complete and overall responses at week 52 for the treatment of proliferative lupus nephritis when given in combination with mycophenolate and corticosteroids. An exploratory analysis at week 76 showed an increasing efficacy benefit over control. In addition, patients receiving obinutuzumab had greater improvements in anti-dsDNA antibody levels, C3, C4, and UPCR than control.

We hypothesized that residual B-cells in peripheral blood and renal tissue explained the lack of efficacy of type I anti-CD20 monoclonal antibodies in prior lupus nephritis trials. We hypothesized that enhanced B-cell depletion with obinutuzumab would meaningfully improve renal responses over control. Results from the present study suggest that B-cells play a key role in the pathogenesis of lupus nephritis and that achievement of complete depletion is associated with clinical benefit.

Obinutuzumab was associated with improvements in the achievement of complete and partial renal responses at one year, which are each associated with improved long-term outcomes in lupus nephritis (Chen, YE. et al. (2008) Clin J Am Soc Nephrol 3:46-53; Davidson, J. et al. (2018) The Journal of Rheumatology 45:5). Because complete responses are uncommon during the first year of treatment, the European League Against Rheumatism (EULAR) guidelines now recommend partial renal response as the initial goal of therapy during the first year of treatment (Fanouriakis, A. et al. (2019) Ann Rheum Dis. Jun;78(6):736-745). Exploratory results from week 76 suggest the benefit of obinutuzumab on overall renal responses compared with control at one year precedes a similar magnitude of benefit on complete renal responses at 18 months.

Infusion-related reactions were more common in patients treated with obinutuzumab than control. In CLL, the incidence and severity of infusion-related reactions with obinutuzumab appears to be greater than observed with rituximab and is associated with marked release of proinflammatory cytokines, in particular IL-6 and IL-8 (Freeman, CL. et al. (2015) Blood 126:2646-2649). Enhanced cross-linking between CD20-expressing leukemic cells and Fc$\gamma$RIIIA-bearing effector cells has been proposed as a mechanism (Freeman, CL et al. (2016) Leukemia 30:1763-1766). We hypothesize that the relatively low rate of infusion-related reactions observed in the present study and the absence of serious infusion-related reactions may be attributed to the corticosteroid regimen given for the treatment of lupus nephritis and differences in CD20 expression between oncology and lupus nephritis patients. As in oncology, the incidence of infusion-related reactions was greatest with the first obinutuzumab infusion and decreased with successive infusions.

The advent of immunosuppressive therapies for the treatment of lupus nephritis has been associated with improvements in short- and long-term outcomes. However, use of current, unapproved standard of care has not been associated with improvements in the incidence of ESRD in recent decades and there are still no therapies approved for the treatment of lupus nephritis in the United States.

***Example 2: A modified obinutuzumab dosing regimen for the treatment of proliferative lupus nephritis in combination with mycophenolate and corticosteroids.***

The study described in Example 1 showed that the dosing regimen of 1000 mg obinutuzumab infusions at weeks 0, 2, 24, and 26, combined with standard of care immunosuppression, demonstrated efficacy and acceptable safety at 52 and 76 weeks in lupus nephritis (LN) patients. This Example describes how a modeling approach was used to predict the expected obinutuzumab PK following a dosing regimen of 1000 mg on weeks 0, 2, 24, 26 and 52 in combination with mycophenolate mofetil and corticosteroids.

*Population Pharmacokinetics Model*

A population pharmacokinetics (PK) model was developed on the basis of the data presented in Example 1. The analysis dataset used to develop the PK model included 658 post-dose serum concentration values of obinutuzumab from 63 patients treated with 1000 mg obinutuzumab infusions at weeks 0, 2, 24, and 26, combined with standard of care immunosuppression, as described in Example 1.

The PK of obinutuzumab was well described by a two-compartment linear population model, in which clearance was the sum of two elimination pathways: a) time-varying clearance ($CL_{T0}$) that decreases with time with a decay coefficient ($k_{des}$), likely related to CD20 target reduction and proteinuria improvement over time; and b) time-independent clearance ($CL_{INF}$) related to endogenous catabolic processes of IgG. The covariates found to influence obinutuzumab PK parameters were body weight (BW), serum albumin at baseline (ALB), and serum IgG levels at baseline.

The final model included allometric dependence of $CL_{INF}$, $CL_{T0}$, and Q (inter-compartmental clearance) on body weight with power coefficient of 0.66, dependence of central and peripheral volumes of distribution on body weight with power coefficient of 0.600, dependence of peripheral volume of distribution on body weight with fixed power coefficient of 1. $CL_{10}$ and $CL_{INF}$ decreased with increase of albumin with the powers of 2.8 and 0.685, respectively. $CL_{INF}$ decreased with increase of IgG concentration with the power of 0.4. No apparent effect of anti-drug antibodies was observed. The model parameter estimates of the final model are presented in **Table 8**.

**Table 8**: Parameter estimates of the final PK model (SE = standard error; RSE = relative standard error; %RSE = 100xSE/PE; PE = parameter estimate; 95% CI = 95% confidence interval; SD = standard deviation; CV = coefficient of variation, 100xSD%; kdes = decay coefficient of time-dependent clearance (day-1); V₁ = central volume of distribution; V₂ = peripheral volume of distribution).

| Fixed Effect Parameter | | Estimate | RSE (%) | 95%CI |
|---|---|---|---|---|
| $k_{des}$ (1/day) | $\theta_1$ | 0.0144 | 20.9 | 0.00848 - 0.0203 |
| $CL_{T0}$ (L/day) | $\theta_2$ | 0.0642 | 15.3 | 0.0449 - 0.0835 |
| $CL_{INF}$ (L/day) | $\theta_3$ | 0.133 | 7.43 | 0.113 - 0.152 |
| $V_1$ (L) | $\theta_4$ | 2.33 | 3.98 | 2.15 - 2.52 |
| $V_2$ (L) | $\theta_5$ | 1.50 | 13.7 | 1.10 - 1.90 |
| Q (L/day) | $\theta_6$ | 0.313 | 47.2 | 0.0233 - 0.602 |
| $CL_{T0}$, $CL_{INF}$, Q ~ WT | $\theta_7$ | 0.660 | 31.8 | 0.249 - 1.07 |
| $V_1$, $V_2$ ~ WT | $\theta_8$ | 0.600 | 13.8 | 0.438 - 0.762 |
| SDL | $\theta_9$ | 1.36 | 6.75 | 1.18 - 1.54 |
| SDH | $\theta_{10}$ | 0.111 | 8.21 | 0.0931 - 0.129 |
| $SD_{50}$ (µg/mL) | $\theta_{11}$ | 5.58 | 20.6 | 3.33 - 7.84 |
| $CL_{T0}$ ~ ALB | $\theta_{12}$ | 2.80 | 18.9 | 1.76 - 3.84 |
| $CL_{INF}$ ~ ALB | $\theta_{13}$ | 0.685 | 39.8 | 0.15 - 1.22 |
| $CL_{INF}$ ~ IgG | $\theta_{14}$ | 0.400 | 27.1 | 0.188 - 0.613 |

| Variance Parameter | | Estimate | RSE (%) | 95%CI | Variability | Shrinkage |
|---|---|---|---|---|---|---|
| $\omega^2_{CLT0}$ | $\Omega(1,1)$ | 0.274 | 46.1 | 0.0267 - 0.522 | CV=52.4% | 19.5% |
| $\omega^2_{CLINF}$ | $\Omega(2,2)$ | 0.0699 | 34.9 | 0.0221 - 0.118 | CV=26.4% | 6.3% |
| $\omega^2_{V1}$ | $\Omega(3,3)$ | 0.0299 | 24.9 | 0.0153 - 0.0444 | CV=17.3% | 7.4% |
| $\sigma^2$ | $\Sigma(1,1)$ | 1 | FIXED | | | 12.1% |

The effects of covariates on model parameters are provided in **Table 9**.

**Table 9:** Covariate effects in the final PK model. [a]The values of the continuous covariates represent 2.5th and 97.5th percentiles of the values in the analysis data set. CI = confidence interval.

| Parameter | Covariate | Reference Value | Covariate Value[a] | Covariate Effect Value [95%CI](%) |
|---|---|---|---|---|
| $CL_T$, $CL_{INF}$, Q | Body weight | 75 kg | 45.1 kg | -28.5 [-42.1; -11.8] |
| | | | 98.1 kg | 19.4 [6.8; 33.4] |
| $CL_T$ | Albumin | 35 g/L | 21.0 g/L | 317.5 [147.9; 603.1] |
| | | | 39.5 g/L | -28.7 [-37; -19.3] |
| $CL_{INF}$ | Albumin | 35 g/L | 21 g/L | 41.9 [8.3; 85.9] |
| | | | 39.5 g/L | -8 [-13.7; -1.9] |
| $CL_{INF}$ | IgG level | 10 g/L | 2.45 g/L | -43.1 [-57.7; -23.3] |
| | | | 21.5 g/L | 35.9 [15.6; 59.7] |
| $V_1$, $V_2$ | Body weight | 75 kg | 45.1 kg | -26.3 [-32.1; -20] |
| | | | 98.1 kg | 17.5 [12.5; 22.7] |

The final model was validated using goodness-of-fit plots, plots of random effects and inter-individual parameters, and predictive check procedures such as visual predictive checks (VPC).

*PK Model Validation*

Validation of the model showed that the final PK model could be used to predict obinutuzumab exposure. For example, visual predictive check (VPC) plots indicated good agreement between observed obinutuzumab concentrations and data simulated using the final PK model (**FIG. 5**).

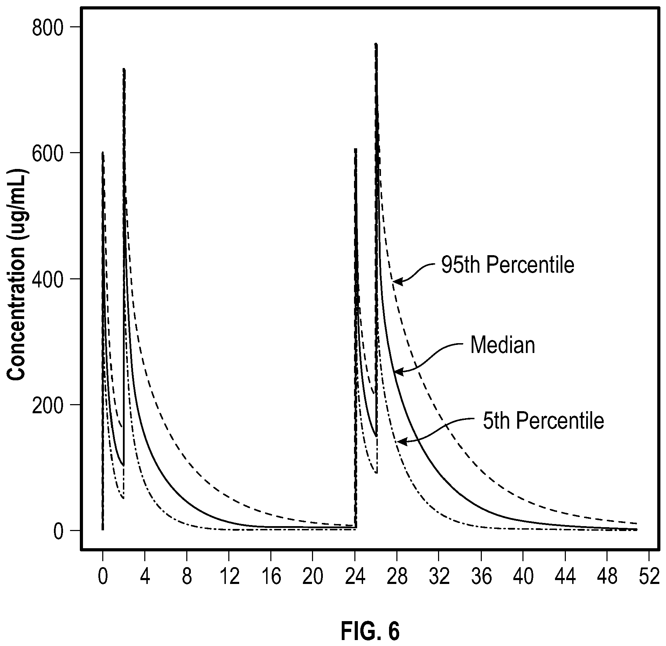

Obinutuzumab concentration profiles over time were simulated using the validated final PK model for all patients following the dosing regimen described in Example 1 (1000 mg at weeks 0, 2, 24, 26, and 52). The predicted concentration profiles of obinutuzumab over time are illustrated in **FIG. 6.**

*Safety- and Efficacy-Exposure Analyses*

Exploratory graphical and logistic regression analyses of exposure-safety relationships did not reveal any relationship with the adverse events (AEs) analyzed. This indicated that a favorable therapeutic window may exist, and that it is possible to improve efficacy by increasing the administered dose without adversely affecting the safety profile. Logistic regression analysis for the probability of adverse events versus obinutuzumab exposure were evaluated for 3 types of events (Late SAEs, infections and infestations, and infusion-related reactions after the first dose) and showed no correlation between exposure to obinutuzumab and the probability of events. For example, as shown in **FIG. 7,** there was no statistically significant relationship between the probability of occurrences of late SAEs and cumulative exposure from treatment start to week 52 ($AUC_{52}$; p = 0.383).

Exploratory graphical analyses of B-cell-efficacy relationships from the study described in Example 1 suggested that a greater proportion of obinutuzumab-dosed patients who achieved sustained peripheral B-cell depletion achieved a complete renal response (CRR) as compared to those who did not. For example, as shown in **Table 10,** 73.7% of patients who achieved CRR had B-cell levels below the B-cell quantification limit (BQL = 0.441 cell/$\mu$l ), whereas 65.8% of patients who did not achieve CRR had B-cell levels below the BQL. Additionally, pharmacokinetic and pharmacodynamic analyses showed that patients with higher obinutuzumab exposures were more likely to have sustained peripheral B-cell depletion as compared to those with lower exposures at Weeks 24 and 52. For example, as shown in **Table C,** 82.8% of patients with high obinutuzumab exposure (cumulative AUC at week 52 above the median) had B-cells below the limit of quantification compared to 53.6% of patients in the low exposure group (cumulative AUC at week 52 below the median).

**Table 10:** Proportion of patients with sustained B-cell depletion by exposure level. Low exposure = cumulative AUC at week 52 below median; high exposure = cumulative AUC at week 52 above median; BQL = Below B-cell quantification limit (0.441 cell/$\mu$l); CRR = complete renal response; PRR = partial renal response (0 = response not achieved; 1 = response achieved).

| Group | N | Percentage of patients with B-cells below threshold for both Week 24 and Week 52 | | |
|---|---|---|---|---|
| | | BQL (0.441 cells/$\mu$L) | 5 cells/$\mu$L | 10 cells/$\mu$L |
| Low Exposure | 28 | 53.6 | 85.7 | 89.3 |
| High Exposure | 29 | 82.8 | 100 | 100 |
| CRR=0 | 38 | 65.8 | 94.7 | 94.7 |
| CRR=1 | 19 | 73.7 | 89.5 | 94.7 |
| CRR / PRR = 0 | 26 | 61.5 | 96.2 | 96.2 |
| CRR / PRR = 1 | 31 | 74.2 | 90.3 | 93.5 |

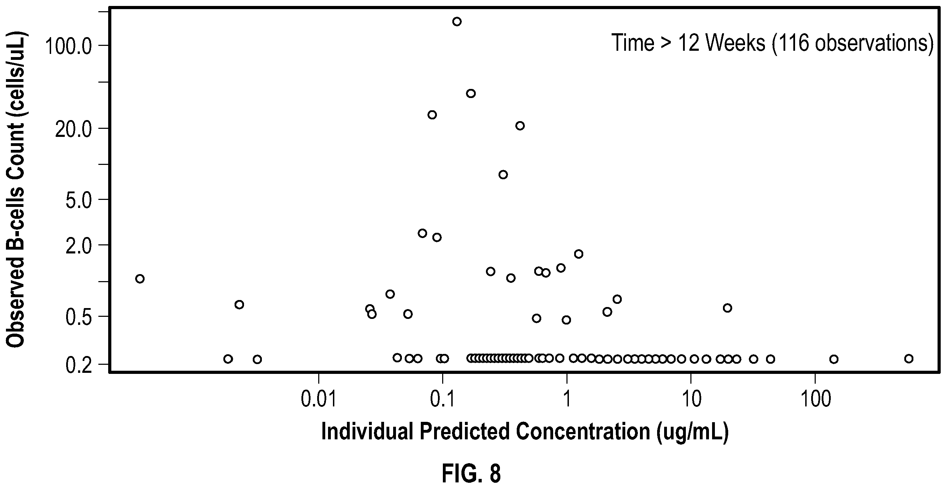

In addition, B-cell depletion appeared to last longer in patients with higher exposure. For example, as shown in **FIG. 8,** at 12 weeks and later, B cell counts dropped below BQL and stayed low as long as obinutuzumab concentrations remained above 1 $\mu$g/mL. In addition, as shown in **FIG. 9,** the probability of B-cell counts rebounding above the BQL (B-cells > BQL) at week 52 decreased with increasing exposure ($AUC_{52}$) (p = 0.045).

*Model-Based Simulations* of *the Proposed Additional Dose of 1000 mg Obinutuzumab at Week 52*

In order to maintain B-cell depletion and potentially increase efficacy at Week 76, an additional infusion of 1000 mg obinutuzumab administered at Week 52 was proposed (1000 mg obinutuzumab on weeks 0, 2, 24, 26, and 52). The fraction of patients with trough obinutuzumab concentrations above 1$\mu$g/mL at Week 76 following administration of an additional dose at Week 52 dose was assessed by simulations using the population PK model described above.

As shown in **Table 11,** simulations of exposure parameters for 5 different obinutuzumab dosing regimens showed that dosing on weeks 0, 2, 24, 26, and 52 results in a predicted 45.9% of subjects having trough obinutuzumab concentrations above 1$\mu$g/mL at week 76. In contrast, only 1.6% of subjects dosed on weeks 0, 2, 24, and 26 or on weeks 0, 2, 12, 24, and 26 were predicted to have trough obinutuzumab concentrations above 1$\mu$g/mL at week 76. Likewise, only 3.3% of subjects dosed on weeks 0, 2, 16, 18, 32, and 34 were predicted to have trough obinutuzumab concentrations above 1$\mu$g/mL at week 76.

**Table 11:** Simulations of exposure parameters for 5 different obinutuzumab dosing regimens. The percent of patients with obinutuzumab concentration > 1$\mu$g/mL at weeks 16 ($C_{16}$), 24 ($C_{24}$), 32 ($C_{32}$), ($C_{52}$) 52, and 76 ($C_{76}$) is provided.

| Case | Dosing Weeks | Percent of subjects with | | |
|---|---|---|---|---|
| | | $C_{24}$ >1 $\mu$g/mL | $C_{52}$ >1 $\mu$g/mL | $C_{76}$ >1 $\mu$g/mL |
| 1 | 0, 2, 12, 24, 26 | 95.1 | 45.9 | 1.6 |
| 2 | 0, 2, 24, 26, 52 | 44.3 | 45.9 | 45.9 |
| 7 | 0, 2, 24, 26 | 44.3 | 45.9 | 1.6 |

As was shown in **FIG. 8,** at 12 weeks and later, B cell counts dropped below BQL and stayed low as long as obinutuzumab concentrations remained above 1 $\mu$g/mL. Thus, an additional dose at Week 52 is expected to maintain B-cell depletion and translate into better efficacy at Week 76.

Furthermore, based on the overall knowledge on obinutuzumab safety, the lack of a statistically significant exposure-safety relationship, and the week 76 safety data collected in the study described in Example 1 (*See* **Table 6** in Example 1), an additional dose at week 52 would still result in an acceptable safety profile.

Given the risk of infusion-related reactions, each obinutuzumab administration is preceded by 80 mg IV methylprednisolone.

In summary, based on the estimated exposures from dosing regimen simulations and an assessment of available safety data, a dosing regimen similar to that used in the study described in Example 1 (*i.e.,* obinutuzumab 1000 mg IV given at weeks 0, 2, 24, and 26) with an additional dose of 1000 mg IV every 6 months thereafter, beginning at Week 52 is proposed.

The data from Example 1 and the modeling and simulation results presented in this Example suggest that the proposed dosing regimen (*i.e.,* dosing at weeks 0, 2, 24, 26, and 52) induces rapid and prolonged B-cell depletion and is effective for treating LN. This dosing regimen (including an additional dose at w52) is expected to provide a safety profile similar to that observed in the study described in Example 1.

*Example 3: Testing a modified obinutuzumab dosing regimen for the treatment of proliferative lupus nephritis in combination with mycophenolate and corticosteroids.*

The modeling approach described in Example 2 was used to predict the PK exposure of an obinutuzumab dosing regimen of 1000 mg on weeks 0, 2, 24, 26 and 52 in combination with mycophenolate mofetil and corticosteroids. This Example describes the administration of an obinutuzumab dosing regimen of 1000 mg on weeks 0, 2, 24, 26 and 52.

**Dosing**

Patients are randomly assigned to one of two groups: obinutuzumab arm and placebo arm. Patients in the obinutuzumab arm receive obinutuzumab 1000 mg by intravenous (IV) infusion at baseline and study weeks 2, 24, 26, and 52 along with the premedications. Patients in the obinutuzumab arm are split into two sub-groups. Both sub-groups receive obinutuzumab 1000 mg by intravenous (IV) infusion. One sub-group receives infusions at baseline and study weeks 2, 24, 26, and 52 along with the premedications; the other sub-group receives infusions at baseline and study weeks 2, 24, 26, 50, and 52 along with the premedications. Mycophenolate mofetil (MMF) is administered on day 1, and titrations are made by week 4 to target dose and remain at target dose until week 80. MMF is administered at a starting dose of 1500mg/day (or equivalent) in divided doses with titration by 500mg/week to 2.0-2.5 g/day by week 4, then kept at target dose through week 80. Oral prednisone is initiated at 0.5mg/kg/day on day 2 until week 2 visit. Tapering of prednisone begins on day 16 to target dose of 5mg/day by week 24. Prednisone remains at 5mg/day from week 24-80.

Patients in the placebo arm receive placebo matching to obinutuzumab IV infusion at baseline and weeks 2, 24, 26, and 52 alongside the premedications. MMF is administered on day 1, and titrations are made by week 4 to target dose and remain at target dose until week 80. MMF is administered at a starting dose of 1500mg/day (or equivalent) in divided doses with titration by 500mg/week to 2.0-2.5 g/day by week 4, then kept at target dose through week 80. Oral prednisone is initiated at 0.5mg/kg/day on day 2 until week 2 visit. Tapering of prednisone begins on day 16 to target dose of 5mg/day by week 24. Prednisone remains at 5mg/day from week 24-80.

For premedications, methylprednisolone 80mg is administered by IV infusion at weeks 0, 2, 24, 26, and 52. Acetaminophen is administered at 650-1000mg orally between 30-60 minutes prior to obinutuzumab or placebo infusion. Diphenhydramine is administered at 50mg orally between 30-60 minutes prior to obinutuzumab or placebo infusion.

Patients are followed in a blinded fashion and patients with persistent B-cell depletion are followed for safety and B-cell assessments. *See* also the protocol published in WO2016/183104.

**Patients**

Patients are eligible if they are between the ages of 18 and 75 years, had systemic lupus erythematosus (SLE) as established by current American College of Rheumatology criteria, diagnosis of International Society of Nephrology/Renal Pathology Society 2003 Class III or IV as evidenced by renal biopsy performed 6 months prior to or during screening (can exhibit concomitant Class V disease in addition to Class III or Class IV disease), a urine protein to creatinine ratio (UPCR) >1 on a 24-hour urine collection, had received at least one dose of pulse methylprednisolone IV (500-1000mg) or equivalent for treatment of the current episode of active LN during the 6 months prior to screening or during screening, and had received an ACE inhibitor or angiotensin-receptor blocker (ARB) at a stable dose of ≥10 days prior to randomization (unless these therapies are contraindicated).

Exclusion criteria include: pregnancy, breastfeeding, or intending to become pregnant during the study or within 18 months after final dose of study drug; severe renal impairment or need for dialysis or renal transplantation; sclerosis in >50% of glomeruli on renal biopsy; presence of rapidly progressive glomerulonephritis; receipt of excluded therapy (any anti-CD20 therapy during 12 months prior to randomization, cyclophosphamide, tacrolimus, ciclosporin, or voclosporin during 2 months prior to randomization, any biologic therapy other than anti-CD20 during 3 months prior to randomization, oral inhibitors of janus associated kinase (JAK), Bruton's tyrosine kinase (BTK), or tyrosine kinase 2 (TYK2) during 3 months prior to randomization, any live vaccine during 2 months prior to randomization); severe, active central nervous system SLE; high risk for clinically significant bleeding or organ dysfunction due to thrombocytopenia, anemia, and/or coagulopathy, or if plasmapheresis, intravenous immunoglobulin, or acute blood product transfusions are required; known HIV infection; known active infection of any kind excluding fungal infection of nail beds; any major episode of infection that requires hospitalization or treatment with IV antibiotics or anti-infectives during 3 months prior to randomization or requires treatment with oral antibiotics or anti-infectives during 6 weeks prior to randomization; history of progressive multifocal leukoencephalopathy (PML); history of cancer except for non-melanomatous carcinomas of the skin that have been treated or excised and have resolved; and intolerance or contraindication to study therapies, including positive hepatitis C serology, hemoglobin < 7g/dL (unless caused by autoimmune hemolytic anemia resulting from SLE), platelet count <25,000/uL, positive serum human chorionic gonadotropin measured prior to first obinutuzumab infusion, AST or ALT > 2.5x upper limit of normal (ULN), amylase or lipase > 2x ULN, neutrophils < $1.5 \times 10^3$/uL, and positive hepatitis B surface antigen (HBsAg).

**End Points**

Peripheral blood B-cells, safety, urinary protein excretion, serum creatinine, levels of autoantibodies and serum complement components, and clinical disease activity are assessed as described in Example 1. Endpoints such as proportion of patients who achieved CRR, proportion of patients who achieved modified CRR, and proportion of patients who achieved PRR are measured as described in Example 1.

Primary outcome measure is the percentage of participants with a complete renal response (CRR) at week 76.

Secondary outcome measures include: percentage of participants with overall renal response (ORR), defined as achievement of either CRR or partial renal response (PRR); change in anti-dsDNA titer; change in complement C3; time to first CRR; percentage of participants who achieve CRR including urinary sediment (CRR-sediment); percentage of participants who achieve ORR including urinary sediment (ORR-sediment); change in systematic lupus erythematosus disease activity index 2000 (SLEDAI-2k); change in fatigue scale (FACIT-F); change in HRQoL (SF-36) scale; change in estimated glomerular filtration rate (eGFR); percentage of participants who achieved CRR with eGFR criterion; percentage of participants with adverse events; maximum serum concentration of obinutuzumab; percentage of participants with anti-drug antibodies (ADAs) at baseline and ADAs posttreatment; and change from baseline in total peripheral B-cell count.

***Example 4: A Phase III, randomized, open-label active comparator-controlled multicenter study to evaluate efficacy and safety of obinutuzumab in patients with primary membranous nephropathy***

This study evaluates the efficacy, safety, pharmacodynamics, and pharmacokinetics of obinutuzumab compared with tacrolimus in patients with primary membranous nephropathy (pMN). This is a Phase III, randomized, parallel-group, active-controlled, open-label study evaluating the efficacy and safety of obinutuzumab as compared to tacrolimus in patients with pMN.

Membranous nephropathy (MN) is classified as either primary or secondary MN depending on its etiology. Idiopathic or primary MN (pMN) is autoimmune in nature, caused by autoantibodies targeting the podocyte membrane. Secondary MN can be caused by an underlying condition such as cancer, infection, autoimmune disease such as systemic lupus erythematosus or treatment with certain drugs, e.g. gold/penicillamine. pMN is a kidney-specific, autoimmune glomerular disease that presents with increased protein in the urine associated with a pathognomonic pattern of injury in glomeruli. Most pMN is mediated by antibodies to the M-type phospholipase A2 receptor (anti-PLA2R) (70-85%), thrombospondin type 1 domain containing 7A (THSD7A) (3-5%), or by others as unidentified anti-podocyte autoantibodies (10%) (Couser WG. Clin J Am Soc Nephrol 2017;12(6):983-97). These autoantibodies target the podocyte membrane resulting in subepithelial deposits of immune complexes and extensive podocyte foot process effacement, leading to ineffective filtration and proteinuria. These autoantibodies likely arise from dysregulated B cells, and patients with persistent proteinuria, when treated with immunosuppressive agents, show improvement (KDIGO Clinical Practice Guideline for Glomerulonephritis - Chapter 7: Idiopathic membranous nephropathy).

pMN is the most common cause of idiopathic nephrotic syndrome in nondiabetic adults worldwide, accounting for 20-37% of cases and rising to as high as 40% in adults over 60 years of age (Couser WG. Clin J Am Soc Nephrol 2017;12(6):983-97). Signs and symptoms of nephrotic syndrome are hypoalbuminemia, edema, weight gain, hyperlipidemia, fatigue, and loss of appetite. Thromboembolism, infections, hypothyroidism, hypertension, anemia, and coronary artery disease are common complications (de Seigneux S, Martin PY. Swiss Med Wkly 2009;139(29-30):416-22). The natural course of pMN is variable with one third of patients achieving spontaneous remission, another third developing chronic subnephrotic range proteinuria and the remaining third progressing to end-stage renal disease (ESRD) over 5 to 10 years. Clinically, 80% of pMN patients present with nephrotic syndrome and 20% with non-nephrotic proteinuria (Couser WG. Clin J Am Soc Nephrol 2017;12(6):983-97). Complete remission of nephrotic range proteinuria predicts excellent long-term kidney and patient survival with achievement of partial remission to subnephrotic range proteinuria also significantly reducing the risk of progression to ESRD requiring dialysis or kidney transplantation (Cattran DC, Brenchley PE. Kidney Int 91 2017;566-574; Troyanov S, et al., and the Toronto Glomerulonephritis Registry Group. Kidney Int 2004;66(3):1199-205; Fervenza FC, Sethi S, Specks U. Clin J Am Soc Nephrol 2008;3(3):905-19; Hladunewich MA, et al., and the Metropolitan Toronto Glomerulonephritis Registry. Clin J Am Soc Nephrol 2009;4(9):1417-22; Polanco N, et al., and the Grupo de Estudio de las Enfermedades Glomerulares de la Sociedad Española de Nefrologia. J Am Soc Nephrol 2010;21(4):697-704).

There is currently no United States Food and Drug Administration (FDA) approved treatment for pMN and the current treatment options remain controversial. Due to the variable natural course of the disease, the KDIGO guidelines suggest that initial therapy for patients with MN is optimized supportive care (including Renin Angiotensin System [RAS] blockade and blood pressure control) and immunosuppressive therapy is recommended for patients with persistent nephrotic syndrome. A regimen of alternating glucocorticoids and alkylating agents such as chlorambucil (Italian Ponticelli protocol) or cyclophosphamide (modified Ponticelli protocol) is effective in 60-70% of patients for reaching some form of remission but has been associated with clinically significant toxicities and adverse effects, including hyperglycemia, myelosuppression, infections, infertility, and cancer (Waldman M, Austin HA 3rd. J Am Soc Nephrol 2012;23(10):1617-30). Calcineurin inhibitors (CNIs), including cyclosporin, are effective and are the preferred treatment for MN in the United States and Canada. However, these agents are associated with a high incidence of relapse after discontinuation and with frequent side effects, including hypertension, hyperlipidemia, and nephrotoxic effects (Rojas-Rivera JE, Carriazo S, Ortiz A. Clin Kidney J 2019;12(5):629-38; Fervenza FC, Appel GB, Barbour SJ, et al., and the MENTOR Investigators. N Engl J Med 2019, 381(1):36-46). Given the high relapse rates and serious adverse effects, there is a high unmet need for effective treatment options in pMN.

Since B cells play a key role in autoantibody production and as such pMN pathogenesis, the type I anti-CD20 antibody, rituximab, was used in multiple studies with the resultant B cell depletion leading to remission of nephrotic syndrome (Fervenza FC, et al., and the MENTOR Investigators. N Engl J Med 2019; 381(1):36-46; Dahan K, et al. Kidney Int Rep 2018;3(2):498-501; Ruggenenti P, et al. J Am Soc Nephrol 2015;26(10):2545-58). In a recent study, MENTOR, a randomized controlled clinical study comparing rituximab and cyclosporin A (CsA) in 130 pMN patients, rituximab showed superior overall renal response over active comparator CsA at 24 months, with reduced occurrence of relapses and of serious adverse events (Fervenza FC, et al., and the MENTOR Investigators. N Engl J Med 2019; 381(1):36-46). However, with rituximab use, approximately 40% of patients fail treatment or do not show any type of response (neither complete nor partial) at 24 months highlighting the remaining unmet medical need (Fervenza FC, et al., and the MENTOR Investigators. N Engl J Med 2019; 381(1):36-46).

### Objectives

The primary efficacy objective is to evaluate the efficacy of obinutuzumab compared with tacrolimus on the basis of the proportion of patients who achieve a complete response (CR) at week 104. CR is defined as a urinary protein-to-creatinine ratio (UPCR) $\leq 0.3$ (24-hour collection) with a stable estimated GFR (eGFR), defined as an eGFR that is $\leq 15\%$ below baseline and with no occurrence of intercurrent events (escape therapy, treatment failure, or early study withdrawal). eGFR is calculated using the chronic kidney disease epidemiology collaboration (CKD-EPI) equation (Levey, A.S. et al. (2009) Ann. Intern. Med. 150:604-12).

The secondary efficacy objective is to evaluate the efficacy of obinutuzumab compared with tacrolimus on the basis of the following endopoints:

- Proportion of patients who achieve an overall remission (OR) (CR and/or partial remission [PR]) at week 104. PR is defined as a 50% reduction in UPCR from baseline and UPCR $\leq 3.5$ but >0.3 with stable eGFR, defined as an eGFR that is $\leq 15\%$ below baseline. eGFR is calculated using the CKD-EPI equation.
- Proportion of patients who achieve CR at week 76.
- Proportion of patients who relapse by week 104, defined as an increase in the UPCR to >3.5 following achievement of a CR or PR. Relapse of a patient who had achieved PR additionally requires either a >50% increase in UPCR to >3.5 (with 50% increase assessed from nadir in UPCR during PR), or in the investigator's opinion requires additional or change in immunosuppressive therapy.
- Proportion of patients who receive escape therapy by week 104.
- Proportion of patients who achieve immunologic remission (change in status from anti-phospholipase A2 receptor [PLA2R] autoantibody positive at baseline to anti-PLA2R negative) at week 52.
- Mean change in ankle circumference (edema) from baseline to week 104.
- Proportion of patients with $\geq 30\%$ reduction in eGFR from baseline to week 104.
- Mean change from baseline in the patient-reported outcomes measurement information system (PROMIS) global assessment of physical health scale at week 104.
- Mean change from baseline in the PROMIS fatigue scale at week 104.
- Duration of CR.

The exploratory efficacy objective is to evaluate the efficacy of obinutuzumab compared with tacrolimus on the basis of the following endpoints:

- Proportion of patients who achieve an OR (CR and/or PR) at Week 52 or Week 76. PR is defined as a 50% reduction in UPCR from baseline and UPCR $\leq$ 3.5 but > 0.3 with stable eGFR, defined as an eGFR is that $\leq$ 15% below baseline. eGFR is calculated using the CKD-EPI equation.
- Proportion of patients who have a UPCR $\leq$ 0.3 at Week 52, Week 76, and Week 104.
- Time to first CR over the course of 104 weeks.
- Time to relapse after CR or PR over the course of 104 weeks.
- Change from baseline in anti-PLA2R autoantibody titer (in patients positive for anti-PLA2R autoantibody at baseline).
- Change from baseline in anti-THSD7A autoantibody titer (in patients positive for anti-THSD7A autoantibody at baseline).
- Proportion of patients who achieve immunological remission by Week 4, Week 12, Week 76, and Week 104.
- Time to immunologic remission.
- Proportion of patients who achieve OR at Week 104 based on anti PLA2R autoantibody status (positive/negative) at baseline.
- Proportion of patients who achieve CR at Week 104 based on anti-PLA2R autoantibody status (positive/negative) at baseline.
- Proportion of patients who achieve OR at Week 104 based on anti-PLA2R autoantibody titer (low/medium/high) at baseline.
- Proportion of patients who achieve CR at Week 104 based on anti-PLA2R autoantibody titer (low/medium/high) at baseline.
- Proportion of patients who achieve OR at Week 104 based on immunological remission status at Week 24.
- Proportion of patients who achieve CR at Week 104 based on immunological remission status at Week 24.
- Mean change in ankle circumference (edema) from baseline to week 76.
- Proportion of patients with a $\geq$ 50% decrease in creatinine clearance from baseline at week 52.
- Mean change from baseline in the PROMIS Global Assessment of Physical Health scale at Week 52 and Week 76
- Mean change from baseline in the PROMIS Fatigue scale at Week 52 and Week 76.
- Mean change from baseline in the Cure Glomerulonephropathy Network (CureGN) Patient-Reported Edema scale at Week 52, Week 76, and Week 104.
- Mean change from baseline in the PROMIS Global Assessment of Mental Health scale at Week 52, Week 76, and Week 104.
- Mean change from baseline in the PROMIS Anxiety scale at Week 52, Week 76, and Week 104.
- Mean change from baseline in the PROMIS Sleep scale at Week 52, Week 76, and Week 104.
- Mean change from baseline in the Subject Global Assessment (SGA) at Week 52, Week 76, and Week 104.
- Change from baseline in EuroQol 5-Dimension Questionnaire (5-level version; EQ 5D-5L) index-based and Visual Analog Scale (VAS) scores at Week 24, Week 52, Week 76, and Week 104.
- The proportion of escape patients who achieve renal remission at Week 52, Week 76, and Week 104 of escape therapy.

The safety objective for this study is to evaluate the safety of obinutuzumab compared with tacrolimus on the basis of the following endpoints:

- Incidence and severity of adverse events, with severity determined according to National Cancer Institute Common Terminology Criteria for Adverse Events (NCI CTCAE) v5.0.
- Characterization of adverse events of special interest.
- Change from baseline in targeted vital signs.
- Change from baseline in targeted clinical laboratory test results.

The pharmacodynamic (PD) objective for this study is to characterize the PD effects of obinutuzumab in pMN patients on the basis of peripheral B-cell counts at specified timepoints.

The pharmacokinetic (PK) objective for this study is to characterize the PK of obinutuzumab in the pMN population on the basis of serum concentrations of obinutuzumab at specified timepoints.

The immunogenicity objective for this study is to evaluate the immune response to obinutuzumab on the basis of prevalence of anti-drug antibodies (ADAs) at baseline and incidence of ADAs during the study.

**Target Population**

Approximately 140 patients are enrolled. The proportion of patients who have received prior immunosuppressant treatments is no more than 30% of the total population. Patients are 18-75 years old with renal biopsy-confirmed pMN. At time of enrollment, patients must have been and remain on best supportive care (e.g., maximally tolerated renin-angiotensin system blockade with angiotensin-converting enzyme [ACE] inhibitor and/or angiotensin-receptor blocker [ARB]) for at least 3 months with UPCR ≥5 or for at least 6 months with UPCR ≥4 without experiencing a 50% decrease in proteinuria during that time. The study includes the following study periods: screening, open-label treatment, long-term follow-up (LTFU), escape treatment (for a subset of patients fulfilling escape criteria), and safety follow-up (SFU).

Patients must meet the following inclusion criteria for study entry:

- Age 18-75 years at time of signing Informed Consent Form
- Diagnosis of pMN according to renal biopsy prior to or during screening

  ○ Diagnosis should be based on light and immunofluorescence microscopy and, if possible, electron microscopy

- UPCR ≥ 5g from 24-hour urine collection despite best supportive care for ≥ 3 months prior to screening or UPCR ≥ 4g despite best supportive care ≥ 6 months prior to screening. Best supportive care comprises renin-angiotensin system blockade with ACE inhibitor and/or ARB at maximally tolerated approved dose.
- Systolic blood pressure ≤ 140 mmHg and diastolic blood pressure ≤ 90 mmHg at screening
- eGFR ≥ 40mL/min/1.73m$^2$ or qualified endogenous creatinine clearance ≥ 40mL/min based on 24-hour urine collection during screening. eGFR is calculated using the CKD-EPI equation.
- Patients who previously responded to CNIs (CsA or tacrolimus), rituximab, or alkylating agents with either a CR or PR and subsequently relapsed are eligible but require discontinuation of CNIs or alkylating agents for ≥ 6 months and rituximab for ≥ 9 months prior to screening.

Patients who meet any of the following exclusion criteria are excluded from study entry:

- Patients with a secondary cause of MN (e.g., hepatitis B, SLE, medications, malignancies)
- Uncontrolled blood pressure during 3 months prior to screening
- Evidence of ≥ 50% reduction in proteinuria during the previous 6 months prior to screening
- Receipt of renal replacement therapy (e.g., renal transplantation, chronic dialysis)
- Type 1 or 2 diabetes mellitus (to exclude proteinuria secondary to diabetic nephropathy). Patients who have recent history of steroid-induced diabetes but no evidence on renal biopsy for diabetic nephropathy performed within 6 months of entry into the study are eligible.
- History of resistance (no response) to CNIs or B-cell depleting antibodies. Patients with non-response to rituximab due to development of human anti-chimeric antibodies are eligible
- Receipt of previous therapies as follows:

  ○ Treatment with MMF or oral, intramuscular, or intravenous corticosteroids within 1 month prior to screening
  ○ Any B-cell depleting therapy such as rituximab, ocrelizumab, or ofatumumab within 9 months prior to screening
  ○ Treatment with cyclophosphamide or CNI within 6 months prior to screening
  ○ Treatment with any biologic therapy (other than B-cell depleting agents) such as belimumab, ustekinumab, or anifrolumab within 6 months prior to screening
  ○ Treatment with an inhibit of Janus-associated kinase, Bruton's tyrosine kinase, or tyrosine kinase 2, including but not limited to tofacitinib, baricitinib, upadacitinib, filgotinib, ibrutinib, or fenebrutinib within 3 months prior to screening
  ○ Receipt of a live vaccine within 28 days prior to screening or during screening

- Thrombocytopenia, anemia, and/or coagulopathy with high risk for clinical significant bleeding or organ dysfunction or requiring plasmapheresis, IVIg, or acute blood product transfusions
- Known HIV infection
- Tuberculosis (TB) infection
- Known active infection of any kind, excluding fungal infection of nail beds
- Any major episode of infection requiring hospitalization or treatment either with IV anti-infective treatments during the 2 months prior to or during screening or with oral anti-infective treatments during the 2 weeks prior to or during screening
- History of serious recurrent or chronic infection
- History of progressive multifocal leukoencephalopathy (PML)
- History of cancer, including solid tumors, hematological malignancies, and carcinoma in situ, except non-melanomatous carcinomas of the skin that have been treated or excised and have resolved
- Laboratory parameters

○ AST or ALT >2.5 x the upper limit of normal (ULN)

○ Amylase or lipase > 2 x ULN

○ Neutrophils <1.5x10$^3$/μL

○ CD19+ B cells <5/μL

○ Positive for hepatitis B surface antigen (HBsAg) at screening. Patients who are HBsAg negative and hepatitis B core antibody positive with no detectable hepatitis B virus (HBV) DNA are allowed into the study but require regular HBV DNA monitoring.

○ Positive hepatitis C virus (HCV) antibody at screening. Patients with positive hepatitis C antibody test result with no detectable HCV RNA for at least 12 months after completion of antiviral therapy are eligible but require regular HCV RNA monitoring.

○ Hemoglobin < 9g/dL

○ Platelet count < 75,000/μL

○ Positive serum human chorionic gonadotropin measured at screening

**Treatment**

At enrollment, patients are randomized at a 1:1 ratio to receive open-label treatment with either obinutuzumab or tacrolimus. The randomization is stratified by region and by anti-PLA2R autoantibody titer (high titer [≥175 RU/mL] vs. non-high titer [<175 RU/mL]).

Patients assigned to the obinutuzumab arm receive infusions of obinutuzumab 1000mg at Week 0 (Day 1), Week 2, Week 24, and Week 26. Obinutuzumab infusions are preceded by methylprednisolone 80mg IV, oral antihistamine, and analgesic to reduce the probability of infusion-related reactions (IRRs).

Patients assigned to tacrolimus receive tacrolimus starting at an oral dose of 0.05mg per kilogram (patient dry weight) per day, divided into 2 equal doses given at 12-hour intervals. Blood tacrolimus concentrations are assessed every 2 weeks, and the tacrolimus dose is titrated to maintain a target trough concentration of 5-7ng/mL. Optimized tacrolimus dose is maintained for 52 weeks and then tapered over 8 weeks. All patients assigned to tacrolimus are tapered off by the start of Week 60.

The open-label treatment period ends after Week 104 or upon start of escape therapy.

Patients who meet the escape criteria or who relapse during the open-label treatment period after Week 52 follow the escape treatment plan. The patient can only be eligible for escape treatment once. Patients who were treated with obinutuzumab in the escape treatment period and who failed to respond to that treatment receive therapy according to the investigator's best medical judgment. The escape treatment period ends when the study achieves the common-close timepoint. For patients originally in the obinutuzumab arm, escape therapy includes obinutuzumab 1000mg IV at Week 0 (Day 1) and Week 2, and tacrolimus for 26 weeks then tapered over 8 weeks. For patients originally in the tacrolimus arm, escape therapy includes obinutuzumab 1000mg IV at Week 0 (Day 1) and Week 2, with another course (2 infusions administered 14 days apart) repeated 26 weeks later, with dose of tacrolimus tapered from week 24 over 8 weeks.

Patients who complete the Week 104 assessments in the open-label treatment period enter and remain in the LTFU period until the study achieves the common-close timepoint. In principle, consistent with established clinical guidance, patients are observed and treated with obinutuzumab per clinical evaluation.

All patients in the study, excepting those patients who discontinue early have a SFU visit. The SFU period is a monitoring period for patients who received obinutuzumab, including patients assigned to the tacrolimus arm who received any amount of obinutuzumab at any time during the study, and whose peripheral CD19+ B cells are below the lower limit of normal (LLN) or the baseline value (whichever is lower). Patients in the SFU period are assessed Q26W until their peripheral CD19+ B cells return to the LLN or the baseline value, whichever is lower. Patients may be eligible to enter the SFU period after completion in the LTFU or escape-treatment periods, or after early treatment discontinuation. If a patient receives any B-cell depleting therapies (including, but not limited to, rituximab, cyclophosphamide, obinutuzumab, ofatumumab, or belimumab) during the SFU period, a final SFU visit is required 28 days after the final dose of the B-cell depleting therapy, and the patient discontinues from the SFU period. If patient received tacrolimus only, then a single SFU visit is required 28 days after the final dose of tacrolimus, and the patient does not enter the SFU period. No study drug is provided during SFU or the SFU period.

**Claims**

1. Obinutuzumab for use in a method of treating lupus nephritis in a human individual having lupus, wherein the method comprises:

   (a) the treatment of the individual with (i) an effective amount of mycophenolate mofetil, or mycophenolic acid or a salt thereof, and (ii) an effective amount of a glucocorticoid or a corticosteroid; and
(b) the administration to the individual of four doses of 1000 mg each of obinutuzumab intravenously (IV) at weeks 0, 2, 24 and 26 of treatment; followed by the administration of an additional dose of 1000 mg obinutuzumab IV every six months thereafter, beginning at week 52 of treatment.

2.  Obinutuzumab for the use according to claim 1, wherein the obinutuzumab is administered on days 1, 15, 168 and 182 of treatment, and the additional doses are administered every six months starting on day 364 of treatment.

3.  Obinutuzumab for the use according to claims 1 or 2, wherein the method further comprises an administration of 1000 mg IV obinutuzumab at week 50, preferably on day 350.

4.  Obinutuzumab for the use according to any of the preceding claims, wherein the individual

    (i) has, or is at risk of developing, class III or class IV lupus nephritis; or
    (ii) has class III(C) or class IV(C) lupus nephritis; or
    (iii) has concomitant class V lupus nephritis.

5.  Obinutuzumab for the use according to any of the preceding claims, wherein the individual is administered at least 1500 mg/day mycophenolate mofetil starting with 1500 mg on day 1 of treatment, and then increasing the dose by 500 mg/week to a dose of between 2.0 g/day and 2.5 g/day by week 4 of treatment.

6.  Obinutuzumab for the use according to any of the preceding claims, wherein the glucocorticoid or corticosteroid is prednisone or methylprednisolone.

7.  Obinutuzumab for the use according to claim 6, wherein the individual is administered oral prednisone at a dose of 0.5 mg/kg/day on day 2 of the treatment and until week 2, then tapered to a dose of 5 mg/day by week 24 of treatment.

8.  Obinutuzumab for the use according to claim 6, wherein the individual is administered an intravenous infusion of methylprednisolone at weeks 0, 2, 24 and 26 of treatment.

9.  Obinutuzumab for the use according to any of the preceding claims, wherein the method further comprises administering to the individual an effective amount of an antihistamine, preferably diphenhydramine.

10. Obinutuzumab for the use according to claim 9, wherein 50 mg diphenhydramine is administered orally between 30 and 60 minutes prior to a dose of obinutuzumab.

11. Obinutuzumab for the use according to any of the preceding claims, wherein the method further comprises administering to the individual an effective amount of a non-steroidal anti-inflammatory drug (NSAID), preferably acetaminophen.

12. Obinutuzumab for the use according to claim 11, wherein between 650 mg and 1000 mg acetaminophen are administered orally between 30 and 60 minutes prior to a dose of obinutuzumab.

13. Obinutuzumab for the use according to any of the preceding claims, wherein the method further comprises administering to the individual an effective amount of an antihypertensive agent, such as an angiotensin-converting enzyme (ACE) inhibitor or an angiotensin-receptor blocker.

**Patentansprüche**

1.  Obinutuzumab zur Verwendung in einem Verfahren zur Behandlung von Lupus-Nephritis bei einem menschlichen Individuum mit Lupus, wobei das Verfahren Folgendes umfasst:

    (a) die Behandlung des Individuums mit (i) einer wirksamen Menge von Mycophenolatmofetil oder Mycophenolsäure oder einem Salz davon und (ii) einer wirksamen Menge eines Glucocorticoids oder eines Corticosteroids; und
    (b) die Verabreichung von vier Dosen von jeweils 1000 mg Obinutuzumab intravenös (i.v.) an das Individuum in den Wochen 0, 2, 24 und 26 der Behandlung; gefolgt von der Verabreichung einer zusätzlichen Dosis von 1000 mg Obinutuzumab i.v. alle sechs Monate danach, beginnend in Woche 52 der Behandlung.

2. Obinutuzumab zur Verwendung nach Anspruch 1, wobei das Obinutuzumab an den Tagen 1, 15, 168 und 182 der Behandlung verabreicht wird und die zusätzlichen Dosen alle sechs Monate, beginnend am Tag 364 der Behandlung, verabreicht werden.

3. Obinutuzumab zur Verwendung nach Anspruch 1 oder 2, wobei das Verfahren ferner eine Verabreichung von 1000 mg i.v. Obinutuzumab in Woche 50, vorzugsweise am Tag 350, umfasst.

4. Obinutuzumab zur Verwendung nach einem der vorhergehenden Ansprüche, wobei das Individuum

(i) Lupus-Nephritis der Klasse III oder der Klasse IV hat oder gefährdet ist, eine solche zu entwickeln; oder
(ii) Lupus-Nephritis der Klasse III(C) oder der Klasse IV(C) hat; oder
(iii) begleitende Lupus-Nephritis der Klasse V hat.

5. Obinutuzumab zur Verwendung nach einem der vorhergehenden Ansprüche, wobei dem Individuum mindestens 1500 mg/Tag Mycophenolatmofetil, beginnend mit 1500 mg am Tag 1 der Behandlung, verabreicht werden und dann die Dosis bis Woche 4 der Behandlung um 500 mg/Woche auf eine Dosis zwischen 2,0 g/Tag und 2,5 g/Tag erhöht wird.

6. Obinutuzumab zur Verwendung nach einem der vorhergehenden Ansprüche, wobei das Glucocorticoid oder Corticosteroid Prednison oder Methylprednisolon ist.

7. Obinutuzumab zur Verwendung nach Anspruch 6, wobei dem Individuum am Tag 2 der Behandlung und bis Woche 2 orales Prednison in einer Dosis von 0,5 mg/kg/Tag verabreicht wird, das dann bis Woche 24 der Behandlung auf eine Dosis von 5 mg/Tag reduziert wird.

8. Obinutuzumab zur Verwendung nach Anspruch 6, wobei dem Individuum eine intravenöse Infusion von Methylprednisolon in den Wochen 0, 2, 24 und 26 der Behandlung verabreicht wird.

9. Obinutuzumab zur Verwendung nach einem der vorhergehenden Ansprüche, wobei das Verfahren ferner das Verabreichen einer wirksamen Menge eines Antihistaminikums, vorzugsweise Diphenhydramin, an das Individuum umfasst.

10. Obinutuzumab zur Verwendung nach Anspruch 9, wobei zwischen 30 und 60 Minuten vor einer Dosis von Obinutuzumab 50 mg Diphenhydramin oral verabreicht werden.

11. Obinutuzumab zur Verwendung nach einem der vorhergehenden Ansprüche, wobei das Verfahren ferner das Verabreichen einer wirksamen Menge eines nichtsteroidalen Antirheumatikums (NSAID), vorzugsweise Paracetamol, an das Individuum umfasst.

12. Obinutuzumab zur Verwendung nach Anspruch 11, wobei zwischen 30 und 60 Minuten vor einer Dosis von Obinutuzumab zwischen 650 mg und 1000 mg Paracetamol oral verabreicht werden.

13. Obinutuzumab zur Verwendung nach einem der vorhergehenden Ansprüche, wobei das Verfahren ferner das Verabreichen einer wirksamen Menge eines blutdrucksenkenden Mittels, wie etwa eines Angiotensin-Converting-Enzyme (ACE)-Inhibitors oder eines Angiotensin-Rezeptor-Blockers, an das Individuum umfasst.

**Revendications**

1. Obinutuzumab pour une utilisation dans une méthode de traitement de la néphropathie lupique chez un individu humain ayant un lupus, dans lequel la méthode comprend :

(a) le traitement de l'individu avec (i) une quantité efficace de mycophénolate mofétil, ou d'acide mycophénolique ou d'un sel de celui-ci, et (ii) une quantité efficace d'un glucocorticoïde ou d'un corticoïde ; et
(b) l'administration à l'individu de quatre doses de 1000 mg chacune d'obinutuzumab par voie intraveineuse (IV) aux semaines de traitement 0, 2, 24 et 26 ; suivie de l'administration d'une dose supplémentaire de 1000 mg d'obinutuzumab IV tous les six mois par la suite, en commençant à la semaine de traitement 52.

2. Obinutuzumab pour l'utilisation selon la revendication 1, dans lequel l'obinutuzumab est administré aux jours de traitement 1, 15, 168 et 182, et les doses supplémentaires sont administrées tous les six mois en commençant au jour de traitement 364.

3. Obinutuzumab pour l'utilisation selon les revendications 1 ou 2, dans lequel la méthode comprend en outre une administration de 1000 mg IV d'obinutuzumab à la semaine 50, de préférence au jour 350.

4. Obinutuzumab pour l'utilisation selon l'une quelconque des revendications précédentes, dans lequel l'individu

(i) a, ou présente un risque de développer, une néphropathie lupique de classe III ou de classe IV ; ou
(ii) a une néphropathie lupique de classe III(C) ou de classe IV(C) ; ou
(iii) a une néphropathie lupique concomitante de classe V.

5. Obinutuzumab pour l'utilisation selon l'une quelconque des revendications précédentes, dans lequel l'individu se voit administrer au moins 1500 mg/jour de mycophénolate mofétil en commençant par 1500 mg au jour de traitement 1, puis en augmentant la dose de 500 mg/semaine jusqu'à une dose entre 2,0 g/jour et 2,5 g/jour à partir de la semaine de traitement 4.

6. Obinutuzumab pour l'utilisation selon l'une quelconque des revendications précédentes, dans lequel le glucocorticoïde ou le corticoïde est la prednisone ou la méthylprednisolone.

7. Obinutuzumab pour l'utilisation selon la revendication 6, dans lequel l'individu se voit administrer de la prednisone par voie orale à une dose de 0,5 mg/kg/jour au jour 2 du traitement et jusqu'à la semaine 2, puis la dose est progressivement réduite jusqu'à une dose de 5 mg/jour à partir de la semaine de traitement 24.

8. Obinutuzumab pour l'utilisation selon la revendication 6, dans lequel l'individu se voit administrer une perfusion intraveineuse de méthylprednisolone aux semaines de traitement 0, 2, 24 et 26.

9. Obinutuzumab pour l'utilisation selon l'une quelconque des revendications précédentes, dans lequel la méthode comprend en outre l'administration à l'individu d'une quantité efficace d'un antihistaminique, de préférence de diphénhydramine.

10. Obinutuzumab pour l'utilisation selon la revendication 9, dans lequel 50 mg de diphénhydramine sont administrés par voie orale entre 30 et 60 minutes avant une dose d'obinutuzumab.

11. Obinutuzumab pour l'utilisation selon l'une quelconque des revendications précédentes, dans lequel la méthode comprend en outre l'administration à l'individu d'une quantité efficace d'un anti-inflammatoire non stéroïdien (AINS), de préférence d'acétaminophène.

12. Obinutuzumab pour l'utilisation selon la revendication 11, dans lequel entre 650 mg et 1000 mg d'acétaminophène sont administrés par voie orale entre 30 et 60 minutes avant une dose d'obinutuzumab.

13. Obinutuzumab pour l'utilisation selon l'une quelconque des revendications précédentes, dans lequel la méthode comprend en outre l'administration à l'individu d'une quantité efficace d'un agent antihypertenseur, tel qu'un inhibiteur de l'enzyme de conversion de l'angiotensine (ACE) ou un bloqueur des récepteurs de l'angiotensine.

FIG. 1A

FIG. 1B

FIG. 1C

EP 4 028 054 B1

**FIG. 1D**

**Complement C3**

**Complement C4**

**Anti-dsDNA IgG**

**UPCR**

Mean Change +/- SEM

Week

Treatment Group ──○── Obinutuzumab ──△── Placebo

**FIG. 2A**

**FIG. 2B**

58

FIG. 3A

FIG. 3B

## FIG. 4

Complete renal response

Modified Complete Renal Response

Overall renal response (CRR+PRR)

Percent with a response (%)

Week

Obinutuzumab
detectable B–cells (n=20)

Obinutuzumab
sustained depletion (n=32)

Obinutuzumab
insufficient data (n=11)

Placebo
detectable B–cells (n=62)

FIG. 5

FIG. 6

**Late SAE of Any Grade, Active Arm Patients**
**(Without Event: 54, With Event: 7)**

p = 0.383

Logistic regression line

Probability

AUC at week 52 (ug*day/mL)

**FIG. 7**

EP 4 028 054 B1

FIG. 8

**Probability of B-Cells > BQL at Week 52 (No:41, Yes:7)**
**(Patients administed all 4 doses)**

FIG. 9

| | Obinutuzumab Sustained Depletion (N = 32) | Obinutuzumab Detectable B Cells (N = 20) | Placebo (N = 62) |
|---|---|---|---|
| Age, mean (SD), years | 34.6 (9.5) | 32.4 (10.3) | 31.9 (10.1) |
| Female, n (%) | 29 (91) | 15 (75) | 51 (82) |
| Body weight, mean (SD), kg | 64.0 (13) | 72.4 (12) | 64.0 (14) |
| Serum creatinine, mean (SD), mg/dL | 0.81 (0.29) | 0.94 (0.39) | 0.80 (0.33) |
| Serum creatinine ≤ ULN, n (%) | 27 (84) | 16 (80) | 55 (89) |
| UPCR, mean (SD) | 2.98 (2.3) | 3.79 (3.2) | 3.02 (2.5) |
| Anti-dsDNA positive, n (%) | 13 (41) | 12 (60) | 36 (58) |
| C3 < 90 mg/dL, n (%) | 23 (72) | 14 (70) | 37 (60) |
| C4 < 16 mg/dL, n (%) | 17 (53) | 13 (65) | 43 (69) |
| IgG level, mean (SD), g/L | 8.4 (4.6) | 11.1 (3.6) | 8.8 (4.3) |

dsDNA, double-stranded DNA; ULN, upper limit of normal; UPCR, urine protein to creatinine ratio

**FIG. 10**

FIG. 11

UPCR, urine protein/creatinine ratio; SCr, serum creatinine; ULN, upper limit of normal.

**FIG. 12**

| Definition of response | Week 52 | | | Week 76 | | |
|---|---|---|---|---|---|---|
| | OBI (n = 63) | PBO (n = 62) | Diff. | OBI (n = 63) | PBO (n = 62) | Diff. |
| UPCR < 0.8 only | 64% | 48% | 15%* | 64% | 47% | 17%* |
| UPCR < 0.8 with SCr requirement<br>UPCR < 0.8 and SCr ≤ ULN or not increased > 15% from baseline SCr | 60% | 48% | 12%* | 64% | 45% | 18%** |

**FIG. 13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4816567 A
- WO 199824893 A
- WO 199634096 A
- WO 199633735 A
- WO 199110741 A
- US 5545807 A
- US 5545806 A
- US 5569825 A
- US 5625126 A
- US 5633425 A
- US 5661016 A
- US 5641870 A
- EP 404097 A
- WO 9311161 A
- US 6982321 B
- US 7087409 B
- US 6075181 A
- US 6150584 A
- WO 200492219 A, Hinton
- WO 200442072 A, Presta
- WO 2005044859 A
- WO 2004035607 A
- WO 2005103081 A
- WO 2004056312 A
- WO 2007031875 A
- US 5736137 A, Andersen
- WO 2004065540 A
- WO 99154342 A
- WO 2003011878 A, Jean-Mairet
- US 6602684 B, Umana
- US 20050123546 A, Umana
- WO 2003085107 A
- US 8883980 B, Umana
- US 5821337 A
- US 7527791 B
- WO 2008077546 A
- US 20030157108 A, Presta, L
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1, Presta, L
- WO 2004056312 A1, Adams
- WO 199730087 A, Patel
- WO 199858964 A, Raju, S
- WO 199922764 A, Raju, S.
- US 5500362 A
- WO 5821337 A
- WO 2006029879 A
- WO 2005100402 A
- US 6737056 B
- US 7332581 B
- US 20120251531 A
- US 6194551 B
- WO 9951642 A
- US 20050014934 A1, Hinton
- US 7371826 B
- US 5648260 A
- US 5624821 A
- WO 9429351 A
- US 5648237 A
- US 5789199 A
- US 5840523 A
- US 5959177 A
- US 6040498 A
- US 6420548 B
- US 7125978 B
- US 6417429 B
- WO 2016183104 A

**Non-patent literature cited in the description**

- **HAHN et al.** *Arthritis Care and Research*, 2012, vol. 64, 797-808
- **FANOURIAKIS et al.** *Ann. Rheum. Dis.*, 2019, vol. 78, 736-45
- **HANLY et al.** *Rheumatology*, 2016, vol. 55 (2), 252-62
- **TEKTONIDOU et al.** *Arthritis Rheumatol*, 2016, vol. 68 (6), 1432-1441

- **MYSLER, E.F. et al.** *Arthritis Rheum.*, 2013, vol. 65, 2368-2379
- **SAMBROOK et al.** Molecular Cloning: A Laboratory Manual. Cold Spring Harbor Laboratory Press, 2001
- Current Protocols in Molecular Biology. 2003
- PCR 2: A Practical Approach. Methods in Enzymology. Academic Press, Inc, 1995
- Antibodies, A Laboratory Manual, and Animal Cell Culture. 1987
- Oligonucleotide Synthesis. 1984
- Methods in Molecular Biology. Humana Press
- Cell Biology: A Laboratory Notebook. Academic Press
- Animal Cell Culture. 1987
- **J.P. MATHER ; P.E. ROBERTS**. Introduction to Cell and Tissue Culture. Plenum Press, 1998
- Cell and Tissue Culture: Laboratory Procedures. J. Wiley and Sons, 1993
- Handbook of Experimental Immunology
- Gene Transfer Vectors for Mammalian Cells. 1987
- PCR: The Polymerase Chain Reaction. 1994
- Current Protocols in Immunology. 1991
- Short Protocols in Molecular Biology. Wiley and Sons, 1999
- **C.A. JANEWAY ; P. TRAVERS**. Immunobiology. 1997
- **P. FINCH**. Antibodies. 1997
- Antibodies: A Practical Approach. IRL Press, 1988
- Monoclonal Antibodies: A Practical Approach. Oxford University Press, 2000
- **E. HARLOW ; D. LANE**. Using Antibodies: A Laboratory Manual. Cold Spring Harbor Laboratory Press, 1999
- The Antibodies. Harwood Academic Publishers, 1995
- Cancer: Principles and Practice of Oncology. J.B. Lippincott Company, 1993
- Basic and Clinical Immunology. Appleton & Lange, 1994, 71
- **KABAT et al.** Sequences of Immunological Interest,. 1991
- **KOHLER ; MILSTEIN**. *Nature*, 1975, vol. 256, 495-97
- **HONGO et al.** *Hybridoma*, 1995, vol. 14 (3), 253-260
- **HARLOW et al.** Antibodies: A Laboratory Manual,. Cold Spring Harbor Laboratory Press, 1988
- **HAMMERLING et al.** Monoclonal Antibodies and T-Cell Hybridomas. Elsevier, 1981, 563-681
- **CLACKSON et al.** *Nature*, 1991, vol. 352, 624-628
- **MARKS et al.** *J. Mol. Biol.*, 1992, vol. 222, 581-597
- **SIDHU et al.** *J. Mol. Biol.*, 2004, vol. 338 (2), 299-310
- **LEE et al.** *J. Mol. Biol.*, 2004, vol. 340 (5), 1073-1093
- **FELLOUSE**. *Proc. Natl. Acad. Sci. USA*, 2004, vol. 101 (34), 12467-12472
- **LEE et al.** *J. Immunol. Methods*, 2004, vol. 284 (1-2), 119-132
- **JAKOBOVITS et al.** *Proc. Natl. Acad. Sci. USA*, 1993, vol. 90, 2551
- **JAKOBOVITS et al.** *Nature*, 1993, vol. 362, 255-258
- **BRUGGEMANN et al.** *Year in Immunol.*, 1993, vol. 7, 33
- **MARKS et al.** *Bio/Technology*, 1992, vol. 10, 779-783
- **LONBERG et al.** *Nature*, 1994, vol. 368, 856-859
- **MORRISON**. *Nature*, 1994, vol. 368, 812-813
- **FISHWILD et al.** *Nature Biotechnol.*, 1996, vol. 14, 845-851
- **NEUBERGER**. *Nature Biotechnol.*, 1996, vol. 14, 826
- **LONBERG ; HUSZAR**. *Intern. Rev. Immunol.*, 1995, vol. 13, 65-93
- **ZAPATA et al.** *Protein Eng.*, 1995, vol. 8 (10), 1057-1062
- **PLUCKTHUN**. The Pharmacology of Monoclonal Antibodies. Springer-Verlag, 1994, vol. 113, 269-315
- **HOLLINGER et al.** *Proc. Natl. Acad. Sci. USA*, 1993, vol. 90, 6444-6448
- **MORRISON et al.** *Proc. Natl. Acad. Sci. USA*, 1984, vol. 81, 6851-6855
- **JONES et al.** *Nature*, 1986, vol. 321, 522-525
- **RIECHMANN et al.** *Nature*, 1988, vol. 332, 323-329
- **PRESTA**. *Curr. Op. Struct. Biol.*, 1992, vol. 2, 593-596
- **VASWANI ; HAMILTON**. *Ann. Allergy, Asthma & Immunol.*, 1998, vol. 1, 105-115
- **HARRIS**. *Biochem. Soc. Transactions*, 1995, vol. 23, 1035-1038
- **HURLE ; GROSS**. *Curr. Op. Biotech.*, 1994, vol. 5, 428-433
- **HOOGENBOOM ; WINTER**. *J. Mol. Biol.*, 1991, vol. 227, 381
- **MARKS et al.** *J. Mol. Biol.*, 1991, vol. 222, 581
- **COLE et al.** Monoclonal Antibodies and Cancer Therapy,. Alan R. Liss, 1985, 77
- **BOERNER et al.** *J. Immunol.*, 1991, vol. 147 (1), 86-95
- **VAN DIJK ; VAN DE WINKEL**. *Curr. Opin. Pharmacol.*, 2001, vol. 5, 368-74
- **LI et al.** *Proc. Natl. Acad. Sci. USA*, 2006, vol. 103, 3557-3562
- **XU et al.** Immunity. 2000, vol. 13, 37-45
- **JOHNSON ; WU**. Methods in Molecular Biology. Human Press, 2003, vol. 248, 1-25

- **HAMERS-CASTERMAN et al.** *Nature,* 1993, vol. 363, 446-448
- **SHERIFF et al.** *Nature Struct. Biol.*, 1996, vol. 3, 733-736
- **KABAT et al.** Sequences of Proteins of Immunological Interest. Public Health Service, National Institutes of Health, 1991
- **CHOTHIA** ; **LESK**. *J. Mol. Biol.*, 1987, vol. 196, 901-917
- **BARBAS et al.** *Proc Nat. Acad. Sci. USA*, 1994, vol. 91, 3809-3813
- **SCHIER et al.** *Gene*, 1995, vol. 169, 147-155
- **YELTON et al.** *J. Immunol.*, 1995, vol. 155, 1994-2004
- **JACKSON et al.** *J. Immunol.*, 1995, vol. 154 (7), 3310-9
- **HAWKINS et al.** *J. Mol. Biol.*, 1992, vol. 226, 889-896
- **M. DAËRON**. *Annu. Rev. Immunol.*, 1997, vol. 15, 203-234
- **RAVETCH** ; **KINET**. *Annu. Rev. Immunol.*, 1991, vol. 9, 457-92
- **CAPEL et al.** *Immunomethods*, 1994, vol. 4, 25-34
- **DE HAAS et al.** *J. Lab. Clin. Med.*, 1995, vol. 126, 330-41
- **GUYER et al.** *J. Immunol.*, 1976, vol. 117, 587
- **KIM et al.** *J. Immunol.*, 1994, vol. 24, 249
- **GHETIE** ; **WARD**. *Immunol. Today*, 1997, vol. 18 (12), 592-8
- **GHETIE et al.** *Nature Biotechnology*, 1997, vol. 15 (7), 637-40
- **HINTON et al.** *J. Biol. Chem.*, 2004, vol. 279 (8), 6213-6
- **SHIELDS et al.** *J. Biol. Chem.*, 2001, vol. 9 (2), 6591-6604
- **VALENTINE, M.A. et al.** *J. Biol. Chem.*, 1989, vol. 264 (19), 11282-11287
- **TEDDER, T.F. et al.** *Proc. Natl. Acad. Sci. U.S.A.*, 1988, vol. 85, 208-12
- **STAMENKOVIC, I. et al.** *J. Exp. Med.*, 1988, vol. 167, 1975-80
- **EINFELD, D.A. et al.** *EMBO J.*, 1988, vol. 7, 711-7
- **TEDDER, T.F. et al.** *J. Immunol.*, 1989, vol. 142, 2560-8
- **CRAGG, M.S. et al.** *Blood*, 2004, vol. 103, 2738-2743
- **CRAGG, M.S. et al.** *Blood*, 2003, vol. 101, 1045-1052
- **REFF, M.E.** *Blood*, 1994, vol. 83 (2), 435-445
- **POPPEMA, S.** ; **VISSER, L**. *Biotest Bulletin*, 1987, vol. 3, 131-139
- **UMANA, P. et al.** *Nature Biotechnol.*, 1999, vol. 17, 176-180
- *WHO Drug Information*, 2012, vol. 26 (4), 453
- *WHO Drug Information*, 2009, vol. 23 (2), 176
- *WHO Drug Information*, 2008, vol. 22 (2), 124
- **YAMANE-OHNUKI et al.** *Biotech. Bioeng.*, 2004, vol. 87, 614
- **KANDA, Y. et al.** *Biotechnol. Bioeng.*, 2006, vol. 94 (4), 680-688
- **JENKINS, N. et al.** *Nature Biotechnol.*, 1996, vol. 14, 975-81
- **CUMMING, D.A. et al.** *Glycobiology*, 1991, vol. 1, 115-30
- **JENKINS, N et al.** *Nature Biotechnol.*, 1996, vol. 14, 975-981
- **WRIGHT, A** ; **MORRISON, S.L**. *Trends Biotech.*, 1997, vol. 15, 26-32
- **WRIGHT, A.** ; **MORRISON, S.L**. *Trends Biotech.*, 1997, vol. 15, 26-32
- **LIFELY, M.R. et al.** *Glycobiology*, 1995, vol. 5 (8), 813-22
- **LIFELY, M.R et al.** *Glycobiology*, 1995, vol. 5, 813-822
- **JEFFERIS, R. et al.** *Immunol. Rev*, 1998, vol. 163, 59-76
- **WRIGHT, A** ; **MORRISON, S.L**. *Trends Biotechnol.*, 1997, vol. 15, 26-32
- **ALMAGRO** ; **FRANSSON**. *Front. Biosci.*, 2008, vol. 13, 1619-1633
- **QUEEN et al.** *Proc. Nat'l Acad. Sci. USA*, 1989, vol. 86, 10029-10033
- **KASHMIRI et al.** *Methods*, 2005, vol. 36, 25-34
- **PADLAN**. *Mol. Immunol.*, 1991, vol. 28, 489-498
- **DALL' ACQUA et al.** *Methods*, 2005, vol. 36, 43-60
- **OSBOURN et al.** *Methods*, 2005, vol. 36, 61-68
- **KLIMKA et al.** *Br. J. Cancer*, 2000, vol. 83, 252-260
- **SIMS et al.** *J. Immunol.*, 1993, vol. 151, 2296
- **CARTER et al.** *Proc. Natl. Acad. Sci. USA*, 1992, vol. 89, 4285
- **PRESTA et al.** *J. Immunol.*, 1993, vol. 151, 2623
- **BACA et al.** *J. Biol. Chem.*, 1997, vol. 272, 10678-10684
- **ROSOK et al.** *J. Biol. Chem.*, 1996, vol. 271, 22611-22618
- **WRIGHT et al.** *TIBTECH*, 1997, vol. 15, 26-32
- **OKAZAKI et al.** *J. Mol. Biol.*, 2004, vol. 336, 1239-1249
- **RIPKA et al.** *Arch. Biochem. Biophys.*, 1986, vol. 249, 533-545
- **RAVETCH** ; **KINET**. *Annu. Rev. Immunol.*, 1991, vol. 9, 457-492
- **HELLSTROM, I. et al.** *Proc. Nat'l Acad. Sci. USA*, 1986, vol. 83, 7059-7063
- **HELLSTROM, I et al.** *Proc. Nat'l Acad. Sci. USA*, 1985, vol. 82, 1499-1502

- **BRUGGEMANN, M. et al.** *J. Exp. Med.*, 1987, vol. 166, 1351-1361
- **CLYNES et al.** *Proc. Nat'l Acad. Sci. USA*, 1998, vol. 95, 652-656
- **GAZZANO-SANTORO et al.** *J. Immunol. Methods*, 1996, vol. 202, 163
- **CRAGG, M.S** ; **M.J. GLENNIE**. *Blood*, 2004, vol. 103, 2738-2743
- **PETKOVA, S.B. et al.** *Int'l. Immunol.*, 2006, vol. 18 (12), 1759-1769
- **IDUSOGIE et al.** *J. Immunol.*, 2000, vol. 164, 4178-4184
- **DUNCAN** ; **WINTER**. *Nature*, 1988, vol. 322, 738-40
- **CHARLTON**. Methods in Molecular Biology. Humana Press, 2003, vol. 248, 245-254
- **GERNGROSS**. *Nat. Biotech.*, 2004, vol. 22, 1409-1414
- **LI et al.** *Nat. Biotech.*, 2006, vol. 24, 210-215
- **GRAHAM et al.** *J. Gen Virol.*, 1977, vol. 36, 59
- **MATHER**. *Biol. Reprod.*, 1980, vol. 23, 243-251
- **MATHER et al.** *Annals N.Y. Acad. Sci.*, 1982, vol. 383, 44-68
- **URLAUB et al.** *Proc. Natl. Acad. Sci. USA*, 1980, vol. 77, 4216
- **YAZAKI** ; **WU**. Methods in Molecular Biology. Humana Press, 2003, vol. 248, 255-268
- Epitope Mapping Protocols. **MORRIS**. Methods in Molecular Biology. Humana Press, 1996, vol. 66
- **HARLOW** ; **LANE**. Antibodies: A Laboratory Manual. Cold Spring Harbor Laboratory, 1988
- **CAMERON, J.S.** *J. Am. Soc. Nephrol.*, 1999, vol. 10, 413-424
- **ROUJEAU, J.C. et al.** *Acta Derm. Venereol.*, 1984, vol. 64, 160-163
- **SMITH, P.R. et al.** *Rheumatology (Oxford)*, 1999, vol. 38, 1017-1018
- **FURIE et al.** *Arthritis Rheum.*, 2009, vol. 61 (9), 1143-51
- **TAN et al.** The Revised Criteria for the Classification of SLE. *Arth Rheum*, 1982, vol. 25
- **MCNEILAGE et al.** *J., Clin. Lab. Immunol.*, 1984, vol. 15, 1-17
- **WHITTINGHAM**. *Ann. Acad. Med.*, 1988, vol. 17 (2), 195-200
- **WALLACE** ; **HAHN**. DUBOIS' LUPUS ERYTHEMATOSUS. LIPPINCOTT, 2007
- **TANG et al.** *Medicine*, 2010, vol. 89 (1), 62-67
- **ASHERSON et al.** *Medicine*, 1989, vol. 68 (6), 366-374
- **MARKOWITZ GS** ; **D'AGATI VD**. *Kidney Int*, 2007, vol. 71, 491-495
- **WEENING, JJ**. *Kidney Int*, 2004, vol. 65, 521-530
- **HAHN, B et al.** American College of Rheumatology Guidelines for Screening, Case Definition, Treatment and Management of Lupus Nephritis. *Arthritis Care Res.*, 2012, vol. 64, 797-808
- **CHEN, Y.E. et al.** *Clin. J. Am. Soc. Nephrol.*, 2008, vol. 3, 46-53
- **VITAL, E.M. et al.** *Arthritis Rheum.*, 2011, vol. 63, 3038-3047
- **CHEN, YE. et al.** *Clin J Am Soc Nephrol*, 2008, vol. 3, 46-53
- **DAVIDSON, J et al.** *The Journal of Rheumatology*, 2018, vol. 45, 5
- **FANOURIAKIS, A. et al.** *Ann Rheum Dis.*, June 2019, vol. 78 (6), 736-745
- **FREEMAN, CL et al.** *Blood*, 2015, vol. 126, 2646-2649
- **FREEMAN, CL et al.** *Leukemia*, 2016, vol. 30, 1763-1766
- **COUSER WG.** *Clin J Am Soc Nephrol*, 2017, vol. 12 (6), 983-97
- **DE SEIGNEUX S** ; **MARTIN PY.** *Swiss Med Wkly*, 2009, vol. 139 (29-30), 416-22
- **CATTRAN DC** ; **BRENCHLEY PE**. *Kidney Int*, 2017, vol. 91, 566-574
- **TROYANOV S et al.** Toronto Glomerulonephritis Registry Group.. *Kidney Int*, 2004, vol. 66 (3), 1199-205
- **FERVENZA FC** ; **SETHI S** ; **SPECKS U.** *Clin J Am Soc Nephrol*, 2008, vol. 3 (3), 905-19
- **HLADUNEWICH MA et al.** Metropolitan Toronto Glomerulonephritis Registry.. *Clin J Am Soc Nephrol*, 2009, vol. 4 (9), 1417-22
- **POLANCO N et al.** *Nefrologia. J Am Soc Nephrol*, 2010, vol. 21 (4), 697-704
- **WALDMAN M** ; **AUSTIN HA**. *J Am Soc Nephrol*, 2012, vol. 23 (10), 1617-30
- **ROJAS-RIVERA JE** ; **CARRIAZO S** ; **ORTIZ A.** *Clin Kidney J*, 2019, vol. 12 (5), 629-38
- **FERVENZA FC** ; **APPEL GB** ; **BARBOUR SJ et al.** MENTOR Investigators.. *N Engl J Med*, 2019, vol. 381 (1), 36-46
- **FERVENZA FC et al.** MENTOR Investigators.. *N Engl J Med*, 2019, vol. 381 (1), 36-46
- **DAHAN K et al.** *Kidney Int Rep*, 2018, vol. 3 (2), 498-501
- **RUGGENENTI P et al.** *J Am Soc Nephrol*, 2015, vol. 26 (10), 2545-58
- **LEVEY, A.S. et al.** *Ann. Intern. Med.*, 2009, vol. 150, 604-12